# EUROPEAN PATENT SPECIFICATION

(11) **EP 0 777 498 B1**
(45) Date of publication and mention of the grant of the patent: **28.04.2004**
(21) Application number: 95925792.4
(22) Date of filing: 30.06.1995
(51) Int. Cl.: A61K 41/00, A61K 51/04

(54) **CONJUGATES MADE OF METAL COMPLEXES AND OLIGONUCLEOTIDES**
KONJUGATE AUS METALLKOMPLEXEN UND OLIGONUKLEOTIDEN
CONJUGUES CONSTITUES DE COMPLEXES METALLIQUES ET D'OLIGONUCLEOTIDES

(30) Priority: 14.07.1994 DE 4424922; 05.12.1994 DE 4445078
(43) Date of publication of application: 11.06.1997
(73) Proprietor: SCHERING AKTIENGESELLSCHAFT, 13353 Berlin (DE); NeXstar Pharmaceuticals, Inc., Boulder, CO 80301 (US)
(72) Inventor: DINKELBORG, Ludger, D-13585 Berlin (DE); HILGER, Christoph-Stephan, D-13353 Berlin (DE); NIEDBALLA, Ulrich, D-14195 Berlin (DE); PLATZEK, Johannes, D-14195 Berlin (DE); RADÜCHEL, Bernd, D-13465 Berlin (DE); SPECK, Ulrich, D-13465 Berlin (DE); GOLD, Larry, Boulder, CO 80302 (US); PIEKEN, Wolfgang, Longmont, CO 80503 (US)
(86) International application number: PCT/EP1995/002539
(87) International publication number: WO 1996/002274

(56) References cited:
- EP-A- 0 279 307
- EP-A- 0 490 434
- EP-A- 0 588 229
- WO-A-88/09152
- WO-A-89/00062
- WO-A-91/19813
- WO-A-95/10307
- US-A- 5 013 831

## Description

This invention relates to the object characterized in the claims, i.e., oligonucleotide conjugates, which contain a complexing agent or a complex. These conjugates are used in the fields of diagnosis and treatment.

The imaging diagnosis has achieved great progress in the past decades and is continuously further developing. It is now possible to make visible the vascular system, most organs and many tissues in the living body without major intervention. Diseases are diagnosed in many cases, because they lead to clear changes of shape, size and position of anatomical structures in the body. Such anatomical data from the inside of the body can be obtained by x-ray technology, ultrasonic diagnosis and magnetic resonance tomography. The efficiency of each of the mentioned technologies can be improved by the use of pharmaceutical agents for enhancement of the natural contrasts of the tissues and body fluids in the resulting picture. The pharmaceutical agents in question are introduced in body cavities or injected in blood vessels, with the purpose of changing the contrast of the cavities or vessels. In addition, they are spread by the blood-stream in the organism and can change the visibility of organs and tissues. In exceptional cases, such substances are bound to certain structures in the body and/or actively transported and/or excreted by the latter. In this way, functions can also be made visible in individual cases and used to diagnose diseases.

In contrast to that, the nuclear diagnosis is based on substances that can themselves be made visible. In this case, radioactive isotopes, which emit long-range radiation, are introduced in the body. The spreading of these substances in the organism can be tracked by suitable detectors. The advantage of the nuclear medical process is the high effectiveness at low dosage of the signal-transmitting radioactive substances designated as radiopharmaceutical agents.

If isotopes are used, which release α- or β-radiation or other toxic decomposition products effective in the tissue, radiopharmaceutical agents can also be used for therapeutic purposes, e.g., for destruction of tumors. The same end can also be achieved in that nonharmful isotopes or substances are introduced in the body and converted only there by, e.g., neutron or x-ray radiation, ultrasound or radio waves, to a therapeutically effective form.

A general problem is the diagnosis and localization of pathological changes at a time at which no clear changes of shape, structure and circulation of the organs and tissues in question are available. Such a diagnosis and follow-up is of decisive importance, e.g., in the case of tumor diseases, including the search for metastases, assessment of a deficient supply of tissues with oxygen and in the case of certain infections as well as metabolic diseases.

The now available therapeutic and imaging diagnostic methods are considerably dependent on the availability of pharmaceutical preparations, which accumulate at sites of otherwise undetectable pathological changes.

The contrast media available commercially at this time are quite predominantly so-called nonspecific preparations. They spread passively in the spaces in which they are introduced, e.g., by injection.

In the past, many substances and substance classes have been identified that can detect or can be expected to have a specificity with respect to their spreading in the living organism. Examples in this respect are, in addition to the antibodies, lectins, all types of receptor-bound substances, cells, membranes and membrane components, nucleic acids, natural metabolites and their derivatives, as well as countless pharmaceutical substances. Peptides have been and are also being studied with special care.

US Patent No. 4,707,352 deals with a special process to label complexing molecules with radioactive isotopes, but no well-suited complexing agents for the bonding of metal ions are described.

EP-A-0 285 057 describes nucleotide-complexing agent conjugates, which are not suitable, i.a., because of the in vivo instability of the nucleotides used, for use as in vivo diagnostic agents or therapeutic agents and also hardly meet the other requirements of compatibility and pharmacokinetics.

Many US patents, such as, for example, US Patent No. 4,707,440, deal with modified polymers, which contain a detectable chemical group. The polymers can be polynucleotides and oligonucleotides, but they are neither stabilized against a degradation by naturally occurring nucleases nor selected by a special process, so that they bond specifically with high bonding affinity to target structures. Special embodiments of these detectable molecules are mentioned in US Patents No. 4,843,122 and 4,943,523. An individual nucleotide, modified in this way, is claimed in US Patent No. 4,952,685. The use of these agents in imaging processes is disclosed in US Patent No. 4,849,208.

The object of this invention is the preparation of specifically bonding diagnostic agents for the detection of target structures, by which, for example, the visualization of organs, tissues and their pathological changes in vitro and in vivo is made possible.

It has now been found that this object is achieved by oligonucleotide conjugates, which in addition to an oligonucleotide radical exhibit a complexing agent, bound by a direct bond or a connecting component, and whose oligonucleotide radical is modified so that the degradation by naturally occurring nucleases is prevented or at least significantly inhibited.

Object of this invention is:
Oligonucleotide conjugates consisting of an oligonucleotide radical N and n substituents (B-K), in which the compound exhibits general formula (I)

   N-(B-K)ₙ (I)

   in which N is an oligonucleotide with 15 to 100 nucleotides, which bonds specifically with high bonding affinity to other target structures and exhibits modifications that prevents or at least significantly reduce the degradation by naturally occurring nucleases,
B is a chemical bond or a connecting component to the oligonucleotide radical, which produces the connection between N and K, and K means a complexing agent or complex of radioactive metal isotopes, or stable isotopes, which
   - are converted to radioactive isotopes by radiation from outside,
   - convert radiation from outside to radiation of different quality, different energy content and/or different wavelength,
   of elements of atomic numbers 5, 21-29, 31, 39, 42-44, 49, 57-83 or 85,
n is a number between 1 and 10,
wherein
a) the 2'-position of the sugar unit, independently of one another, is occupied by the following groups:
   a group OR, in which
      R is an alkyl radical with 1 to 20 carbon atoms, which optionally contains up to 2 hydroxyl groups and which optionally is interrupted by 1-5 oxygen atoms,
   a hydrogen atom,
   a hydroxyl group,
   a fluorine atom,
   an amine radical,
   an amino group,
   and hydroxyl groups present in terminal positions 3' and 5', independently of one another, optionally are etherified with radical R and/or
b) the phosphodiesters, optionally being used as the internucleotide bond, independently of one another, are replaced by phosphorothioates, phosphorodithioates or alkylphosphonates, preferably methyl phosphonate, and/or
c) the terminal radicals in 3'- and 5'-positions are linked in an intramolecular manner with one another by an internucleotide bond as described in b) and/or
d) it contains an intemucleotide bond as described in b), which links 3'-3'- or 5'-5'-position, and/or
e) it contains a phosphodiester bond as described in b), which connects, esterlike, two thymidines by a C₂-C₁₀ hydroxyalkyl radical respectively in 3-position or connects an analogously substituted thymidine radical, esterlike, with a hydroxyl group of another sugar in 2'- or 3'- or 5'-position and/or
f) the terminal radicals in 3'- and 5'-positions contain intemucleotide bonds optionally modified as described in b).

Preferred are the inventive compounds wherein N is an oligonucleotide, which bonds specifically with high bonding affinity to other target structures and which can be obtained in that a mixture of oligonucleotides containing random sequences is brought together with the target structure, and certain oligonucleotides exhibit an increased affinity to the target structure relative to the mixture of the oligonucleotides, the latter are separated from the remainder of the oligonucleotide mixture, then the oligonucleotides with increased affinity to the target structure are amplified to obtain a mixture of oligonucleotides that exhibits an increased portion of oligonucleotides that bond on the target structures.

Especially preferred are the inventive compounds wherein N is an oligonucleotide, which specifically bonds with high bonding affinity to other target structures, and which can be obtained in that
a) first, a DNA strand is produced by chemical synthesis, so that on the 3'-end, this DNA strand exhibits a defined sequence, which is complementary to a promoter for an RNA-polymerase and at the same time complementary to a primer of the polymerase chain reaction (PCR), and so that this DNA strand exhibits a defined sequence on the 5'-end, which is complementary to a primer sequence for the polymerase chain reaction, and the sequence between the defined sequences contains a random sequence, and in that
b) this DNA strand is transcribed in a complementary RNA strand with the help of an RNA-polymerase, and nucleotides are offered to the polymerase, which are modified in the 2'-position of the ribose unit, and in that
c) the RNA oligonucleotides, produced in this way, are brought together with the target structure on which the oligonucleotide specifically is to bond, and in that
d) those oligonucleotides that have bound on the target structure are separated first together with the target structure from the nonbinding oligonucleotides and then the bound oligonucleotides are separated again from the target structure, and in that
e) these target-structure-specific RNA oligonucleotides are transcribed with the help of reverse transcriptase in a complementary DNA strand, and in that
f) these DNA strands are amplified with the polymerase chain reaction with use of the defined primer sequences, and in that
g) the DNA oligonucleotides amplified in this manner are then transcribed again with the help of the RNA-polymerase and with modified nucleotides in RNA-oligonucleotides, and in that
h) above-mentioned selection steps c) to g) optionally are repeated often until the oligonucleotides, which are characterized by a high bonding affinity to the target structure, are sufficiently selected, and then the sequences of the thus obtained oligonucleotide optionally are able to be determined.

Especially preferred are the inventive compounds wherein the target structure is selected among macromolecules, tissue structures of higher organisms, such as animals or humans, organs or parts of organs of an animal or human, cells, tumor cells or tumors.

Substances, wherein connecting component(s) B is (are) bound
* a) to the 4'-end of oligonucleotide radical N reduced in 4'-position by the CH₂-OH group and/or
* b) to the 3'-end of oligonucleotide radical N reduced in 3'-position by a hydrogen atom and/or
* c) to the phosphodiester bridge(s), reduced by the OH group(s), between two nucleotides each and/or
* d) to 1 to 10 nucleobase(s), which is (are) reduced by a hydrogen atom respectively in 5-, 8-position(s) and/or the amino group(s) in 2-, 4- and 6-position(s) are especially considered in this application.

Especially preferred are the inventive compounds, above described under *a) or *b) wherein B has general formula X-Y-Z¹, which is connected on the X side with the complexing agent or complex and on the Z side with the oligonucleotide, in which
X stands for a direct bond, an -NH or -S group,
Y stands for a straight-chain, branched-chain, saturated or unsaturated C₁-C₂₀ alkylene chain, which optionally contains
1-2 cyclohexylene, 1-5 imino, 1-3 phenylene, 1-3 phenylenimino, 1-3 phenylenoxy, 1-3 hydroxyphenylene, 1-5 amido, 1-2 hydrazido, 1-5 carbonyl, 1-5 ethylenoxy, a ureido, a thioureido, 1-2 carboxyalkylimino, 1-2 ester groups, 1-3 groups of Ar, in which Ar stands for a saturated or unsaturated 5- or 6-ring, which optionally contains 1-2 heteroatoms selected from nitrogen, oxygen and sulfur and/or 1-2 carbonyl groups; 1-10 oxygen, 1-5 nitrogen and/or 1-5 sulfur atoms, and/or optionally is substituted by 1-5 hydroxy, 1-2 mercapto, 1-5 oxo, 1-5 thioxo, 1-3 carboxy, 1-5 carboxy-C₁-C₄ alkyl, 1-5 ester, 1-3 amino, 1-3 hydroxy-C₁-C₄ alkyl, 1-3 C₁-C₇ alkoxy groups, and
Z¹ stands for -CONH-CH₂-4', -NH-CO-4',
-O-P(O)R¹-NH-CH₂-4', -O-P(O)R¹-O-CH₂-4', -O-P(S)R¹-O-3' or -O-P(O)R¹-O-3', in which 4' or 3' indicates the linkage to the terminal sugar unit(s) and R¹ stands for
O⁻, S⁻, a C₁-C₄ alkyl or NR²R³ group, with R² and R³ meaning hydrogen and C₁-C₄ alkyl radicals.

As cyclic structures (Ar), especially cyclic saturated or unsaturated alkylenes with 3 to 6, especially 5 or 6 C atoms, which optionally contain heteroatoms, such as N, S or O, are suitable. As examples, there can be mentioned: cyclopentylene, pyrrolylene, furanylene, thiophenylene, imidazolylene, oxazolylidene, thiazolylene, pyrazolylene, pyrrolidylene, pyridylene, pyrimidylene, maleinimidylene and phthalimidylene groups.

Furthermore compounds above described under *c) wherein B has general formula X-Y-Z², which is connected on the X side with the complexing agent or complex and on the Z side with the oligonucleotide are meant. Herein
Z², in the bridge linking two adjacent sugar units, 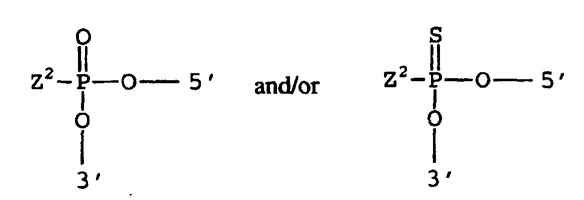 stands for the group -NR²-, -O- or -S-, and X stands for a direct bond, an -NH or -S group,
Y stands for a straight-chain, branched-chain, saturated or unsaturated C₁-C₂₀ alkylene chain, which optionally contains
1-2 cyclohexylene, 1-5 imino, 1-3 phenylene, 1-3 phenylenimino, 1-3 phenylenoxy, 1-3 hydroxyphenylene, 1-5 amido, 1-2 hydrazido, 1-5 carbonyl, 1-5 ethylenoxy, a ureido, a thioureido, 1-2 carboxyalkylimino, 1-2 ester groups, 1-3 groups of Ar, in which Ar stands for a saturated or unsaturated 5- or 6-ring, which optionally contains 1-2 heteroatoms selected from nitrogen, oxygen and sulfur and/or 1-2 carbonyl groups; 1-10 oxygen, 1-5 nitrogen and/or 1-5 sulfur atoms, and/or optionally is substituted by 1-5 hydroxy, 1-2 mercapto, 1-5 oxo, 1-5 thioxo, 1-3 carboxy, 1-5 carboxy-C₁-C₄ alkyl, 1-5 ester, 1-3 amino, 1-3 hydroxy-C₁-C₄ alkyl, 1-3 C₁-C₇ alkoxy groups, and
R² means hydrogen or C₁-C₄ alkyl radicals.

As radicals Y of connecting component Z¹-Y-X (according to point 6) or Z²-Y-X (according to point 7), there can be mentioned as examples the radicals
-(CH₂)₆-NH-CS-NH-C₆H₄-CH(CH₂CO₂H)-CH₂-CO-NH-CH₂-CH(OH)-CH₂-, -(CH₂)₆-NH-CS-NH-C₆H₄-CH₂-, -(CH₂)₆-NH-CO-CH₂-, -(CH₂)₆-NH-CO-CH₂-CH₂-, -(CH₂)₂-, -(CH₂)₆-, -(CH₂)₆-S-(CH₂)₂-, -(CH₂)₆-S-(CH₂)₆-, -(CH₂)₂-NH-CO-, -(CH₂)₆-NH-CO-, -(CH₂)₆-S-(CH₂)-NH-CO, -(CH₂)₆-S-(CH₂)₆-NH-CO-, 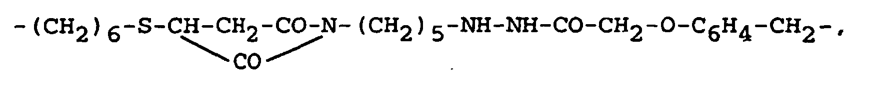 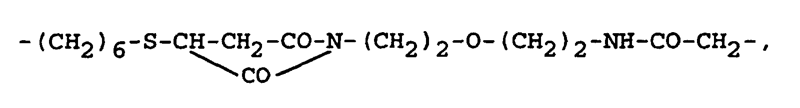 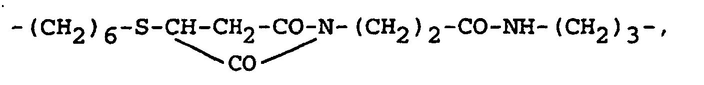 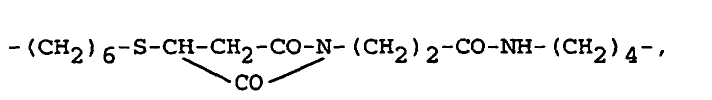 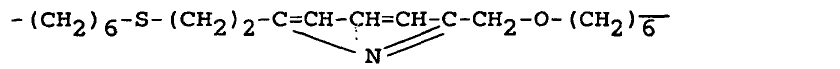 or

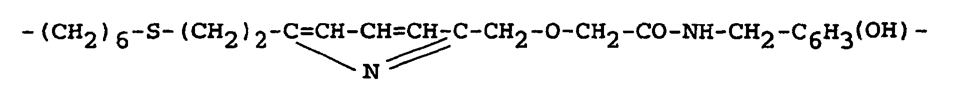

Especially preferred are the inventive compounds above described under *d) wherein B has general formula X-Y-Z³, in which Z³ stands for an -NH group or a direct bond to the nucleobase and X stands for a direct bond, an -NH or -S group,
Y stands for a straight-chain, branched-chain, saturated or unsaturated C₁-C₂₀ alkylene chain, which optionally contains
1-2 cyclohexylene, 1-5 imino, 1-3 phenylene, 1-3 phenylenimino, 1-3 phenylenoxy, 1-3 hydroxyphenylene, 1-5 amido, 1-2 hydrazido, 1-5 carbonyl, 1-5 ethylenoxy, a ureido, a thioureido, 1-2 carboxyalkylimino, 1-2 ester groups, 1-3 groups of Ar, in which Ar stands for a saturated or unsaturated 5- or 6-ring, which optionally contains 1-2 heteroatoms selected from nitrogen, oxygen and sulfur and/or 1-2 carbonyl groups; 1-10 oxygen, 1-5 nitrogen and/or 1-5 sulfur atoms, and/or optionally is substituted by 1-5 hydroxy, 1-2 mercapto, 1-5 oxo, 1-5 thioxo, 1-3 carboxy, 1-5 carboxy-C₁-C₄ alkyl, 1-5 ester, 1-3 amino, 1-3 hydroxy-C₁-C₄ alkyl, 1-3 C₁-C₇ alkoxy groups.
There can be mentioned as examples the radicals -CH₂-CO-NH-CH₂-CH(OH)-CH₂-,-NH-CO-CH₂-CO-NH-CH₂-CH(OH)-CH₂-, -CO-NH-CH₂-CH₂-NH-, -CH₂-S-CH₂-CH₂-NH-, -CH₂-S-CH₂-CH₂-, -(CH₂)₄-S-CH₂-CH₂-NH-, -CO-CH₂-S-CH₂-CH₂-NH-, -CO-CH₂-S-(CH₂)₆-NH-,-CH=CH-CO-NH-CH₂-CH₂-NH-, -CH=CH-CH₂-NH-, -C≡C-CH₂-NH- or -CO-CH₂-CH₂-NH-CH₂-CH₂-NH-.

As bonding sites in the case of the purine bases, especially 8-position is suitable, and in the case of the pyrimidine bases, 5-position is suitable. Purely formally, in this case, a hydrogen atom of the respective base is substituted by radical B-K. But a linkage can also take place by amino groups optionally contained in 2-, 4- or 6-position, thus, e.g., by the 2-amino group in guanine, by the 6-amino group in adenine or by the 4-amino group in cytosine. In this case, a hydrogen atom of the respective amino group is respectively substituted by radical B-K.

Compounds according to one of the preceding points, wherein the metal complex, as imaging element, contains a radioactive isotope, selected from the elements copper, bismuth, technetium, rhenium or indium are considered in this application.

The invention also comprises a process for detecting a target structure, wherein one or more of the compounds according to one of the preceding points are brought together in vivo or in vitro with the sample to be studied and based on the signal, it is detected whether the target structure, on which oligonucleotide N bonds specifically and with high bonding affinity, is present in the sample.

A process for noninvasive diagnosis of diseases is part of the invention, wherein one or more of the inventive compounds mentioned are brought together with the target structure to be studied in vivo and based on the signal, it is detected whether the target structure, on which oligonucleotide N specifically bonds, is present in the organism to be studied.

The object of the invention is also the use of a compounds described above for the production of a medicament useful in radiodiagnosis and/or in radiotherapy.

Furthermore the use of a diagnosis kit for in vivo and/or in vitro detection of target structures, wherein the diagnosis kit contains at least one compound according to the description is object of the invention.

Especially preferred are compounds, wherein N is a non-naturally occuring oligonucleotide ligand having a specific binding affinity for a target molecule, such target molecule being a three dimensional chemical structure other than a polynucleotide that binds to said oligonucleotide ligand through a mechanism which predominantly depends on Watson/Crick base pairing or triple helix binding, wherein said oligonucleotide ligand is not a nucleic acid having the known physiological function of being bound by the target molecule.

If the conjugates according to the invention are to be used as a diagnostic agent, the complexing agent(s) contains (contain) an imaging radioactive isotope of the elements of atomic numbers 21, 26-27, 29, 31, 43 or 49, preferably 43 or 49. If the conjugates according to the invention are to be used as a therapeutic agent, besides the above-mentioned, in addition isotopes of the elements of atomic numbers 5, 22-25, 28, 42, 44, 57-83 and 85 are also suitable. Beyond the radioactive isotopes of the above-mentioned elements, especially also stable isotopes, which
a) are converted by radiation from outside to radioactive isotopes,
b) convert radiation from outside to radiation of different quality, different energy content and/or different wavelength,
are suitable in the range of the treatment.

The number of imaging or therapeutically effective substituents B-K linked with the oligonucleotide radical is, on the one hand, limited by the value of the oligonucleotide, but is never greater than 10. According to the invention, one or two substituents B-K are preferred.

The value of oligonucleotide radical N in this invention is limited to 15 to 100 nucleotides.

Oligonucleotides usable according to the invention are stabilized against degradation by nucleases occurring in vivo.

Unmodified oligonucleotides or polynucleotides are cleaved in vivo by endonucleases and exonucleases. The degradation reaction in the RNA series begins with an activation of the 2'-hydroxy group. Other catabolic enzymes are, e.g., ribozymes, which cleave the phosphodiester bond of RNS (see Science 261, 709 (1993)). The in vivo stability of RNS derivatives can be increased by partial or complete substitution of the 2'-hydroxyl group by other substituents. Such substituents are, e.g., alkoxy groups, especially the methoxy group (see, e.g., Chem. Pharm. Bull. 13, 1273 (1965), Biochemistry 10, 2581, (1971)), a hydrogen atom, a fluorine atom (see e.g., Can. J. Chem. 46, 1131 (1968)) or an amino group (see, e.g., J. Org. Chem. 42, 714 (1977)). Several of these substituents, as well as others, can also be introduced at the 2'-position using the methods disclosed in U.S. application Ser. No. 08/264,029, filed June 22, 1994. Other possibilities for stabilizing the internucleotide bond are the replacement of one or two oxygen atoms in the phosphodiester bridge while forming phosphorothioates (Trends Biochem. Sci. 14, 97 (1989)) or phosphorodithioates (J. Chem. Soc., Chem. Commun. 591 (1983) and Nucleic Acids Res. 12, 9095 (1984)) and the use of alkylphosphonates instead of phosphodiesters (Ann. Rep. N. Y. Acad. Sci. 507, 220 (1988)).

The stabilization can be achieved in that the hydroxyl groups in 2'-position of the ribose units, independently of one another, are modified. Such a modification can be achieved by a replacement of this hydroxyl group by an OR group, a halogen atom, especially a fluorine atom, a hydrogen atom or an amine radical, especially by an amino group. Radical R of the alkoxy group stands, in this case, for a straight-chain or branched alkyl radical with 1 to 20 C atoms, such as methyl, ethyl, propyl, isopropyl, butyl, tert-butyl, pentyl or hexyl or a cyclic unsubstituted or substituted alkyl radical with 4 to 20 C atoms, such as cyclopentyl or cyclohexyl, which optionally contain 1-2 hydroxy groups, and optionally is interrupted by 1-5 oxygen atoms. The stabilization is also increased because the present hydroxyl groups in 3'- and 5'-positions optionally are etherified.

Another stabilization of the polynucleotide takes place in that the phosphodiesters being used as internucleotide bond are replaced partially or completely, and independently of one another, by phosphorothioates, phosphorodithioates or alkylphosphonates, especially preferably by lower alkylphosphonates, such as, e.g., methyl phosphonate. These intemucleotide bonds can also be linked to the terminal radicals in 3'- and 5'-positions or else also connect 3'-3'- or 5'-5'-positions. The phosphodiester bond makes possible further linkages by hydroxyalkyl radicals, which are present on nitrogen or carbon atoms of the nucleobases, thus, for example, two thymidines can be linked by the hydroxyalkyl chains present in 3-position or two purine bases by the radicals present in 8-positions. The linkage can also take place to hydroxyl groups in 2'- or 3'- or 5'-position.

The modified intemucleotide bonds can optionally occur preferably at the ends of the polynucleotide, and they are especially preferably bound to the thymidine.

According to the invention, oligonucleotide radicals N used are not limited to specific oligonucleotide sequences. But preferred are those oligonucleotides that bond specifically with high bonding affinity to target structures with the exception of nucleic acid.

A process for identifying suitable oligonucleotides, which are required as initial substances for the conjugates according to the invention, is described in U.S. Patent 5,270,163. This process, termed SELEX, can be used to make a nucleic acid ligand to any desired target molecule.

The SELEX method involves selection from a mixture of candidate oligonucleotides and step-wise iterations of binding, partitioning and amplification, using the same general selection scheme, to achieve virtually any desired criterion of binding affinity and selectivity. Starting from a mixture of nucleic acids, preferably comprising a segment of randomized sequence, the SELEX method includes steps of contacting the mixture with the target under conditions favorable for binding, partitioning unbound nucleic acids from those nucleic acids which have bound specifically to target molecules, dissociating the nucleic acid-target complexes, amplifying the nucleic acids dissociated from the nucleic acid-target complexes to yield a ligand-enriched mixture of nucleic acids, then reiterating the steps of binding, partitioning, dissociating and amplifying through as many cycles as desired to yield highly specific, high affinity nucleic acid ligands to the target molecule.

The basic SELEX method has been modified to achieve a number of specific objectives. For example, U.S. patent application Ser. No. 07/960,093, filed October 14, 1992, describes the use of SELEX in conjunction with gel electrophoresis to select nucleic acid molecules with specific structural characteristics, such as bent DNA. U.S. patent application Ser. No. 08/123,935, filed September 17, 1993, describes a SELEX-based method for selecting nucleic acid ligands containing photoreactive groups capable of binding and/or photocrosslinking to and/or photoinactivating a target molecule. U.S. patent application Ser. No. 08/134,028, filed October 7, 1993, describes a method for identifying highly specific nucleic acid ligands able to discriminate between closely related molecules, termed Counter-SELEX. U.S. patent application Ser. No. 08/143,564, filed October 25, 1993, describes a SELEX-based method which achieves highly efficient partitioning between oligonucleotides having high and low affinity for a target molecules. U.S. patent application Ser. No. 07/964,624, filed October 21, 1992, describes methods for obtaining improved nucleic acid ligands after SELEX has been performed. U.S. patent application Ser. No. 08/400,440, filed March 8, 1995, describes methods for covalently linking a ligand to its target.

The SELEX method encompasses the identification of high-affinity nucleic acid ligands containing modified nucleotides conferring improved characteristics on the ligand, such as improved in vivo stability or improved delivery characteristics. Examples of such modifications include chemical substitutions at the ribose and/or phosphate and/or base substitutions. SELEX-identified nucleic acid ligands containing modified nucleotides are described in U.S. patent application Ser. No. 08/117,991, filed September 8, 1993, that describes oligonucleotides containing nucleotide derivatives chemically modified at the 5- and 2'-positions of pyrimidines. U.S. patent application Ser. No. 08/134,028, supra, describes highly specific nucleic acid ligands containing one or more nucleotides modified with 2'-amino (2'-NH₂), 2'-fluoro (2'-F), and/or 2'-O-methyl (2'-OMe). U.S. patent application Ser. No. 08/264,029, filed June 22, 1994, describes oligonucleotides containing various 2'-modified pyrimidines.

The SELEX method encompasses combining selected oligonucleotides with other selected oligonucleotides and non-oligonucleotide functional units as described in U.S. patent applications Ser. No. 08/284,063, filed August 2, 1994, and Ser. No. 08/234,997, filed April 28, 1994, respectively. These applications allow the combination of the broad array of shapes and other properties, and the efficient amplification and replication properties, of oligonucleotides with the desirable properties of other molecules.

In its most basic form, the SELEX process may be defined by the following series of steps:
1) A candidate mixture of nucleic acids of differing sequence is prepared. The candidate mixture generally includes regions of fixed sequences (i.e., each of the members of the candidate mixture contains the same sequences in the same location) and regions of randomized sequences. The fixed sequence regions are selected either: (a) to assist in the amplification steps described below, (b) to mimic a sequence known to bind to the target, or (c) to enhance the concentration of a given structural arrangement of the nucleic acids in the candidate mixture. The randomized sequences can be totally randomized (i.e., the probability of finding a base at any position being one in four) or only partially randomized (e.g., the probability of finding a base at any location can be selected at any level between 0 and 100 percent).
2) The candidate mixture is contacted with the selected target under conditions favorable for binding between the target and members of the candidate mixture. Under these circumstances, the interaction between the target and the nucleic acids of the candidate mixture can be considered as forming nucleic acid-target pairs between the target and those nucleic acids having the strongest affinity for the target.
3) The nucleic acids with the highest affinity for the target are partitioned from those nucleic acids with lesser affinity to the target. Because only an extremely small number of sequences (and possibly only one molecule of nucleic acid) corresponding to the highest affinity nucleic acids exist in the candidate mixture, it is generally desirable to set the partitioning criteria so that a significant amount of the nucleic acids in the candidate mixture (approximately 5-50%) are retained during partitioning.
4) Those nucleic acids selected during partitioning as having the relatively higher affinity to the target are then amplified to create a new candidate mixture that is enriched in nucleic acids having a relatively higher affinity for the target.
5) By repeating the partitioning and amplifying steps above, the newly formed candidate mixture contains fewer and fewer unique sequences, and the average degree of affinity of the nucleic acids to the target will generally increase. Taken to its extreme, the SELEX process will yield a candidate mixture containing one or a small number of unique nucleic acids representing those nucleic acids from the original candidate mixture having the highest affinity to the target molecule.

The SELEX patents and applications describe and elaborate on this process in great detail. Included are targets that can be used in the process; methods for partitioning nucleic acids within a candidate mixture; and methods for amplifying partitioned nucleic acids to generate enriched candidate mixture. The SELEX patents and applications also describe ligands obtained to a number of target species, including both protein targets where the protein is and is not a nucleic acid binding protein. Therefore, the SELEX process can be used to provide high affinity ligands of a target molecule.

Target molecules are preferably proteins, but can also include among others carbohydrates, peptidoglycans and a variety of small molecules. As with conventional proteinaceous antibodies, nucleic acid antibodies (oligonucleotide ligands) can be employed to target biological structures, such as cell surfaces or viruses, through specific interaction with a molecule that is an integral part of that biological structure. Oligonucleotide ligands are advantageous in that they are not limited by self tolerance, as are conventional antibodies. Also nucleic acid antibodies do not require animals or cell cultures for synthesis or production, since SELEX is a wholly in vitro process. As is well-known, nucleic acids can bind to complementary nucleic acid sequences. This property of nucleic acids has been extensively utilized for the detection, quantitation and isolation of nucleic acid molecules. Thus, the methods of the present invention are not intended to encompass these well-known binding capabilities between nucleic acids. Specifically, the methods of the present invention related to the use of nucleic acid antibodies are not intended to encompass known binding affinities between nucleic acid molecules. A number of proteins are known to function via binding to nucleic sequences, such as regulatory proteins which bind to nucleic acid operator sequences. The known ability of certain nucleic acid binding proteins to bind to their natural sites, for example, has been employed in the detection, quantitation, isolation and purification of such proteins. The methods of the present invention related to the use of oligonucleotide ligands are not intended to encompass the known binding affinity between nucleic acid binding proteins and nucleic acid sequences to which they are known to bind. However, novel, non-naturally-occurring sequences which bind to the same nucleic acid binding proteins can be developed using SELEX. In particular, the oligonucleotide ligands of the present invention bind to such target molecules which comprise a three dimensional chemical structure, other than a polynucleotide that binds to said oligonucleotide ligand through a mechanism which predominantly depends on Watson/Crick base pairing or triple helix binding, wherein said oligonucleotide ligand is not a nucleic acid having the known physiological function of being bound by the target molecule.

It should be noted that SELEX allows very rapid determination of nucleic acid sequences that will bind to a protein and, thus, can be readily employed to determine the structure of unknown operator and binding site sequences which sequences can then be employed for applications as described herein. SELEX is thus a general method for use of nucleic acid molecules for the detection, quantitation, isolation and purification of proteins which are not known to bind nucleic acids. In addition, certain nucleic acid antibodies isolatable by SELEX can also be employed to affect the function, for example inhibit, enhance or activate the function, of specific target molecules or structures. Specifically, nucleic acid antibodies can be employed to inhibit, enhance or activate the function of proteins.

The oligonucleotides used in the conjugates according to the invention are obtained in a preferred embodiment according to the process described below.

Thus, suitable oligonucleotides can be obtained in that a mixture of oligonucleotides containing random sequences is brought together with the target structure, and certain oligonucleotides exhibit an increased affinity to the target structure relative to the mixture of the oligonucleotides, the latter are separated from the remainder of the oligonucleotide mixture, then the oligonucleotides with increased affinity to the target structure are amplified to obtain a mixture of oligonucleotides that exhibits an increased portion of oligonucleotides that bond to the target structures.

In the process, first a DNA strand is produced in a preferred way by chemical synthesis. On the 3'-end, this DNA strand has a known sequence, which is used as promoter for an RNA polymerase and at the same time is complementary to a primer sequence for the polymerase chain reaction (PCR). In an especially preferred embodiment, in this case, the promoter for the T7 RNA-polymerase is involved. Then, a random sequence is synthesized on the promoter. The random sequence can be obtained in that the suitable four bases are fed in the same ratio to the synthesis machine. Thus, completely random DNA sequences result. In a preferred embodiment, the length of the random sequence is about 15 to 100 nucleotides. Another DNA sequence, which can be used for the polymerase chain reaction (PCR), is synthesized on this DNA piece with the random sequence.

After synthesis of this DNA strand, the latter is transcribed in a complementary RNA strand with the help of an RNA polymerase. In a preferred embodiment, the T7 RNA polymerase is used in this case. In the transcription, nucleotides that are modified are offered to the RNA polymerase. In an especially preferred embodiment, the ribose is modified in 2'-position. In this case, a substitution of the hydrogen atom or the hydroxyl group by an alkoxy group, preferably methoxy, amino or fluorine, can be involved. The RNA oligonucleotides produced in this manner are then introduced in the selection process.

In the selection process, the RNA oligonucleotides are brought together with the target structure. Target structure is understood to mean a structure on which the oligonucleotide is to bond specifically and with high affinity.

Such structures are, e.g., macromolecules, tissue structures of higher organisms, such as animals or humans, organs or parts of organs, cells, especially tumor cells or tumors.

In this connection, the target structure must not absolutely be in pure form, it can also be present on a naturally occurring organ or on a cell surface. Stringency may applied to the selection process by the addition of polyamino (tRNA, heparin), plasma or whole blood to the SELEX reaction.

If an isolated protein is involved here, the latter can be bound to a solid phase, for example, a filter. In the selection, an excess of the target structure relative to the RNA mixture is used. In the incubation, the specific oligonucleotide molecules bond on the target structures, while the unbound oligonucleotides are separated from the mixture, for example by washing.

Then, the oligonucleotide molecules are separated from the target molecules or removed by washing with suitable buffers or solvents.

With the help of the reverse transcriptase, the RNA oligonucleotide found is transcribed in the complementary DNA strand.

Since the DNA strand obtained exhibits primer sequences (or promoter sequences) on both ends, an amplification of the DNA sequences found can be performed simply with the help of the polymerase chain reaction.

The DNA oligonucleotides amplified in this way are then again transcribed with the help of the RNA polymerase in RNA oligonucleotides and the thus obtained RNA oligonucleotides can be used in a further selection step (as described above).

After separating the bonding RNA oligonucleotides, obtained in the second selection step, from the target molecules, the latter are again transcribed in DNA with the help of the reverse transcriptase, the thus obtained complementary DNA oligonucleotides are amplified with the help of the polymerase chain reaction and then transcribed again with the help of the RNA polymerase to the RNA oligonucleotides, which are available for a further selection step.

It has turned out that the desired high specificities and high bonding affinities can be obtained if the selection steps are repeated several times. Rarely will the desired oligonucleotide sequence be obtained as early as after one or two selection steps. As soon as the desired specificity and bonding affinity between target structure and oligonucleotide is obtained, the oligonucleotide(s) can be sequenced and as a result, the sequence of the specifically bonding oligonucleotides can be determined.

Especially advantageous in this process is that this process can be used not only with suitable proteins, but also in vivo. But the above-mentioned selection process can also be performed on purified target structures. But it is essential, especially for the in vivo diagnosis, that the specificity of the oligonucleotides is provided for the target structure in the living environment. Therefore, the selection processes can also be performed on cells or cell cultures, on tissues or tissue sections, on perfused organs and even on living organisms.

In this case, it is advantageous that the modified oligonucleotides can withstand the degradation by the almost omnipresent RNAs. As a result, the desired oligonucleotide sequences are themselves accumulated in selection processes on living organisms, since corresponding naturally occurring oligonucleotides would be degraded by the RNAs.

Oligonucleotide radical N can exhibit one or more connecting components B, or substituents B-K, which can be selected independently of one another. Claimed are oligonucleotide conjugates, which contain 1 to 10 identical or 2 to 10 different connecting components B. Especially preferred are oligonucleotide conjugates with one or two connecting components B.

Connecting component B connects oligonucleotide radical N with a complexing agent or complex K.

Advantageously, polydentate, open-chain or cyclic complexing ligands with O, S and N can be used as donor atoms.

As examples for complexing-agent radicals K, there can be mentioned the polyaminopolycarboxylic acids reduced by a hydrogen atom, a hydroxy group and/or an acetic acid group, ethylenediaminetetraacetic acid, diethylenetriaminepentaacetic acid, trans-1,2-cyclohexanediaminetetraacetic acid, 1,4,7,10-tetraazacyclododecanetetraacetic acid, 1,4,7-triazacyclononanetriacetic acid, 1,4,8,11-tetraazatetradecanetetraacetic acid, 1,5,9-triazacyclododecanetriacetic acid, 1,4,7,10-tetraazacyclododecanetriacetic acid and 3,6,9,15-tetraazabicyclo-[9,3,1]-pentadeca-1(15),11,13-trienetriacetic acid.

Suitable complexing agents are described, e.g., in EP 0 485 045, EP 0 071 564 and EP 0 588 229, in DE 43 10 999 and DE 43 11 023 as well as US 4,965,392.

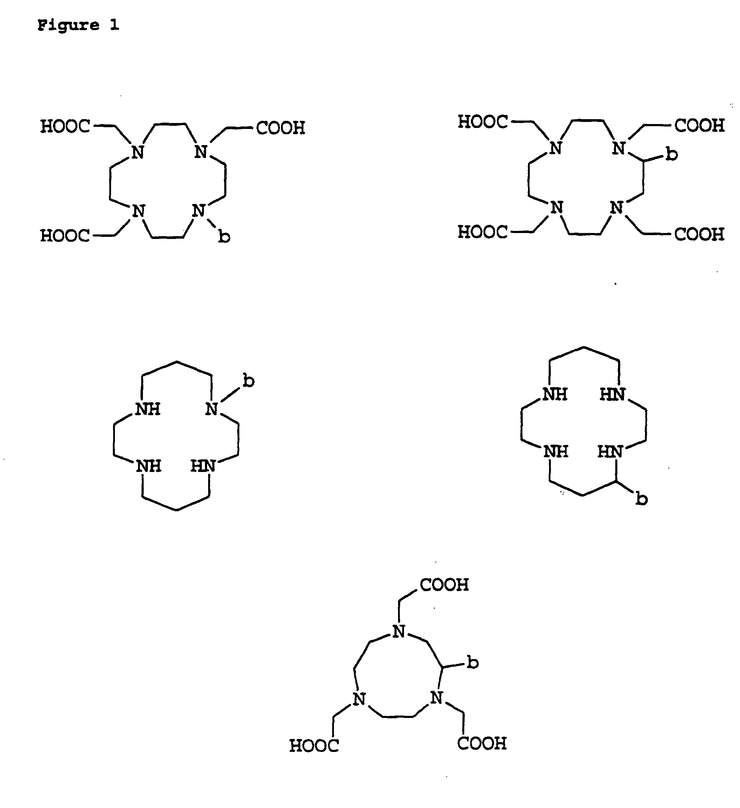

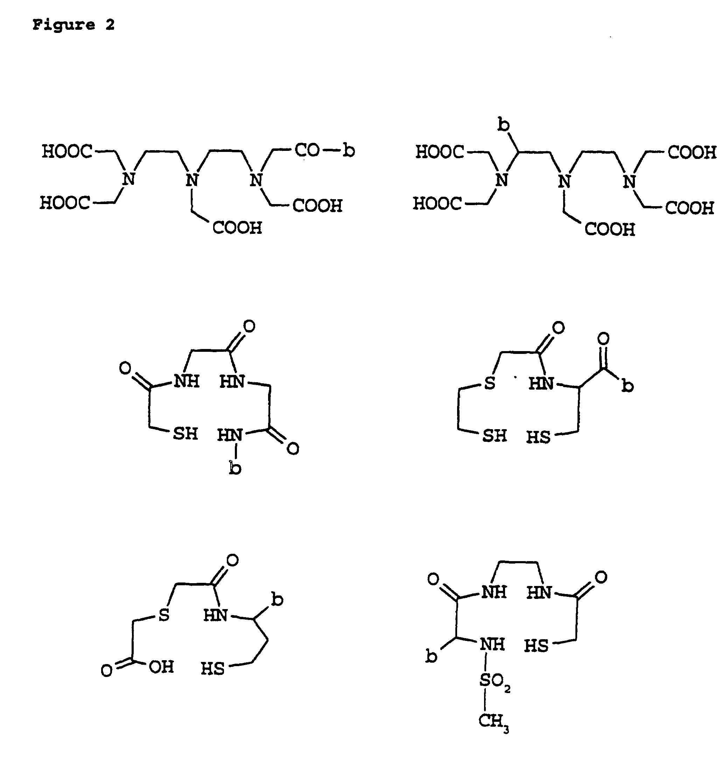

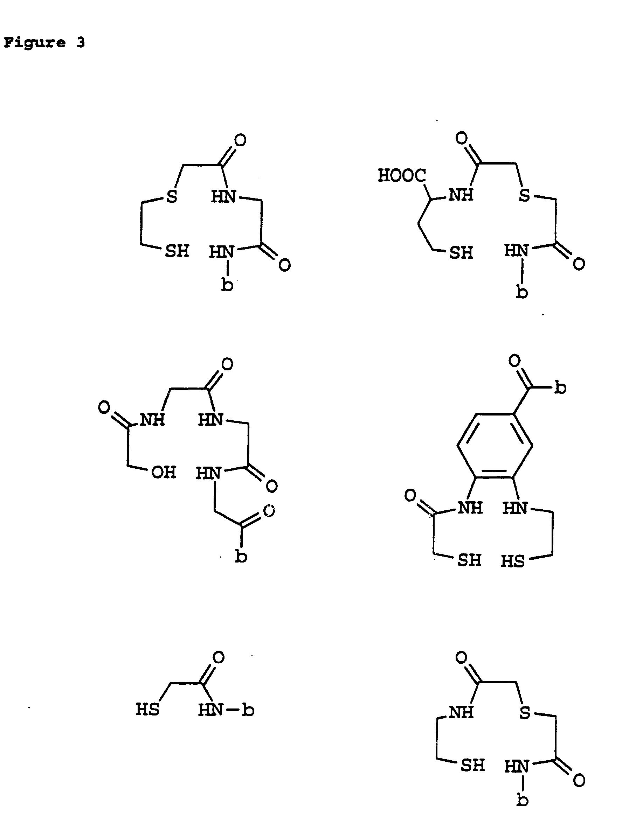

To illustrate the varied possibilities for complexing agents K according to this invention, reference is made to figures 1 to 3, in which some advantageous structures are compiled. These figures are meant as a selection and do not limit this invention in any way to the represented complexing agents.

Complexing agent K can contain all radioactive isotopes, usually used in nuclear medicine for diagnostic and therapeutic purposes, in the form of their metal ions. Stable isotopes, which are excited by external radiation to emit diagnostic or therapeutic radiation, or isotopes, which are converted by radiation from outside to radioactive isotopes, also can be used.

Isotopes suitable according to the invention are selected from the elements of atomic numbers 5, 21-29, 31, 39, 42-44, 49, 57-83 or 85.

For use of the compound according to the invention as a radiopharmaceutical agent, the complexing agent contains a radioactive element. All radioactive elements which are able to achieve a therapeutic or diagnostic effect in vivo or in vitro are suitable for this purpose. Preferred are radioactive isotopes of the elements copper, bismuth, technetium, rhenium or indium. Especially preferred are ^{99m}Tc-complexes.

If the compounds of general formula I according to the invention contain positron-emitting isotopes, such as, e.g., Sc-43, Sc-44, Fe-52, Co-55, Ga-68 or Cu-61, the latter can be used in positron emission tomography (PET).

If the compounds of general formula I according to the invention contain gamma-radiation-emitting isotopes, such as, e.g., Tc-99m or In-111, they can be used in single photon emission tomography (SPECT).

The compounds according to the invention can be used also in radiotherapy in the form of their complexes with radioisotopes, such as, e.g., Ir-192.

The compounds according to the invention can also be used in radioimmunotherapy or radiation therapy. The latter are distinguished from the corresponding diagnosis only by the amount and type of the isotope used. In this case, the purpose is the destruction of tumor cells by high-energy shortwave radiation with a smallest possible range. Suitable β-emitting ions are, for example, Sc-46, Sc-47, Sc-48, Ga-72, Ga-73, Y-90, Re-186 or Re-188. Suitable α-emitting ions exhibiting small half-lives are, for example, At-209, At-211, Bi-211, Bi-212, Bi-213 and Bi-214, and Bi-212 is preferred. A suitable photon- and electron-emitting ion is ¹⁵⁸Gd, which can be obtained from ¹⁵⁷Gd by neutron capture.

If the agent according to the invention is intended for use in the variant of radiation therapy proposed by R. L. Mills et al. (Nature 336, 787 (1988)), the central ion must be derived from a Mössbauer isotope, such as, for example, ⁵⁷Fe or ¹⁵¹Eu.

Those carboxylic acid groups that are not required for complexing the metal ions of the elements of atomic numbers 21 to 29, 31, 39, 42 to 44, 49, 57 to 83 or 85 can optionally be present as salts of an inorganic or organic base, such as alkali- or alkaline-earth metal hydroxides and carbonates, especially sodium and potassium hydroxide, or ammonia and alkylamines, or amino acid or as ester or amide.

Further, compounds that are excited by neutrons to emit particles and/or radiation can be used. Especially effective in this case is gadolinium. Advantageously, those compounds can also be used that contain the isotope boron-10. In such cases, K can have the structure 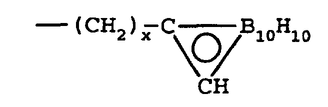 in which x stands for a whole number from 1 to 10.

The invention further relates to processes for the production of the conjugates according to the invention.

Thus, conjugates in which connecting component B is bound on the 5'-end of the oligonucleotide can be obtained by reaction of the oligonucleotide with a phosphoramidite derivative (Tetrahedron 49, 1925-1963 (1993)). To this end, the 5'-hydroxy group of the oligonucleotide is reacted with a phosphoramidite of general formula PR'(NR₂")OR"'. In this case, R' stands for an alkyl, alkoxy or arylalkoxy group, optionally containing N, NO₂, Si or SO₂, with 1 to 20 C atoms, such as methyl, ethyl, propyl, butyl, pentyl, hexyl, methoxy, ethoxy, propyloxy, butyloxy, benzyloxy or phenylethoxy, which optionally can be substituted. As substituents, especially cyano and nitro groups are used. Advantageously, for example, methoxy, β-cyanoethoxy or nitrophenylethoxy groups can be used. Especially preferred are β-cyanoethoxy groups. R" is a C₁-C₄ alkyl radical, and ethyl and propyl radicals are especially suitable. Preferred are isopropyl radicals. R''' is an alkyl or arylalkyl group, optionally containing S, O, N, CN, NO₂ or halogen, with 1 to 20 C atoms. Preferably, protected amino and thioalkyl radicals as well as protected amino and thiooxaalkyl radicals are used. Especially preferred are 6-amino-hexyl, 6-thiohexyl, 3,6,9-trioxa-11-amino-undecyl and 3,6-dioxa-8-amino-octanyl groups. As protective groups, generally usual N- or S-protective groups can be used. For example, trifluoroacetyl, phthalimido and monomethoxytrityl groups are suitable.

In an especially preferred embodiment of this invention, β-cyanoethyl-N,N-diisopropylamino-6-(trifluoroacetamido)-1-hexyl-phosphoramidite is used as phosphoramidite derivative.

In another preferred embodiment of this invention, β-cyanoethyl-N,N-diisopropylamino-(3,6,9-trioxa-11-phthalimido-1-undecyl)-phosphoramidite is used as phosphoramidite derivative.

In another embodiment of the invention, connecting component B is bound on the 3'-end of oligonucleotide N in a way analogous to the one described above by a phosphorus-containing group.

The above-described reaction between oligonucleotide and phosphoramidite can take place as solid-phase reaction, and the oligonucleotide is still on the column of an automatic synthesizer. After an oligonucleotide of the desired sequence has been obtained and exposure of the 5'-hydroxy group of the oligonucleotide has taken place, e.g., with trichloroacetic acid, it is reacted with the phosphoramidite and the reaction product is oxidized and released. Then, the thus obtained oligonucleotide derivative is coupled on the terminal amino or thiol group with the complexing agent or complex K optionally by another linker group. The radical bound in the first step by the phosphorus-containing group on the oligonucleotide then forms, together with the optionally present additional linker group, connecting component B.

The linkage between oligonucleotide and the complexing agent can also take place so that the free 5'-hydroxyl group of the oligonucleotide is reacted with a complexing agent or complex, which terminally carries a bondable phosphorus radical. Such a one can be described by the formula
a) 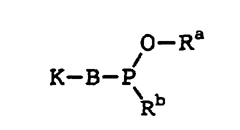 in which
   R^{a} stands for a C₁-C₆ alkyl radical, which optionally carries a cyano group in β-position,
   R^{b} stands for a secondary amino group and
   K and B have the indicated meaning
   or the formula
b) 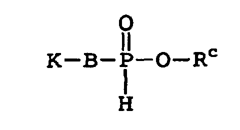 in which
   R^{c} stands for a trialkylammonium cation and K and B have the mentioned meaning,
   or the formula
c) 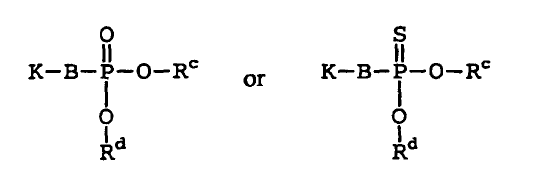 in which
   R^{d} stands for an aryl radical, optionally substituted with one or more halogen atom(s) and/or one or more nitro group(s), or a C₁-C₆ alkyl radical, which optionally is substituted in β-position with a cyano group, and K, B and R^{c} have the mentioned meaning, and when using a radical of formula a), an oxidation step to phosphate takes place after completion of the coupling reaction. In both cases, radical - -OR^{a} or -OR^{c} optionally can be cleaved off in a hydrolysis.

The linkage of the oligonucleotide derivative by the linker with the complexing agent or complex K can take place also as a solid-phase reaction on the column of an automatic synthesizer. The compound according to the invention can then be isolated from the solid vehicle by detaching.

The linkage of the oligonucleotide with the linker can take place not only by the 5'-OH group of the sugar of the terminal nucleotide, but also by other functional groups, which can be generated from the 5'-OH group, such as, e.g., an amino or carboxy group. Such nucleotides carrying amino or carboxy groups are known and can be produced easily. The synthesis of a 5'-deoxy-5'-aminouridine is described in J. Med. Chem. 22, 1273 (1979) as well as in Chem. Lett. 6, 601 (1976). 4'-Carboxy-5'-deoxyuridine is available as described in J. Med. Chem. 21, 1141 (1978), or Nucleic Acids Symp. Ser. 9, 95 (1981).

The linkage with the complexing agent then takes place by a linker carrying a carboxylic acid or amino group in a way known to one skilled in the art. The linker then forms connecting component B together with the -NH-CH₂-4' or the -CO-4' group.

It can be pointed out that the distribution of the conjugates according to the invention into a nucleotide radical, a connecting component and a complexing agent or complex takes place purely formally and thus independently of the actual synthetic structure. Thus, e.g., in the above-mentioned case, the group -NH-CH₂-4' or -CO-4' is considered as belonging to connecting component B, while the oligonucleotide reduced in 4'-position by a CH₂-OH group is designated as oligonucleotide radical N.

A process for the production of conjugates, in which the connecting component to the phosphodiester or phosphorothioate bridges reduced by the OH groups takes place, consists in that first two sugar units are linked to a dinucleotide (see, e.g., Chem. Lett. 1305 (1993)). In this case, there first results a triester of formula 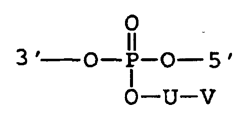 in which U stands for a corresponding alkylene radical and V stands for a protected amino or sulfur group. After cleavage, e.g., of the amino protective group, the complexing agent can optionally be linked, in a way known to one skilled in the art, by a linker with the amino group - e.g., in the form of an amide bond. The linker then forms connecting component B together with group O-U-V' (in which V' stands for a group-NH).

An alternative process consists in that the_phosphotriester passing through intermediately (e.g., by reaction with 1,5-diaminopentane) is subjected to an aminolysis (see Biochemistry 27, 7237 (1988) or J. Am. Chem. Soc. 110, 4470 (1988)).

The thus obtained compounds of formula 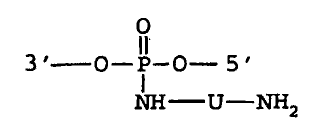 can be linked as described above with the complexing agent optionally by a linker.

For coupling purposes, dinucleoside-phosphate-monothiotriesters are also suitable (see J. Am. Chem. Soc. 111, 9117 (1983) and Nucl. Acids Res. 20, 5205 (1992)).

The nucleobases offer an especially great variety to link the complexing agents with the nucleotides. A linkage by amino groups in 2-position in the purines and in 4-position in the pyrimidines can take place directly. But it is often more advantageous first to modify the purines or pyrimidines and to link these derivatized bases with the complexing agents (optionally by additional linkers). Suitable derivatized nucleobases are described, e.g., in Biochemie [Biochemistry] 71, 319 (1989), Nucl. Acids Res. 16, 4937 (1988) or Nucleosides Nucleotides 10, 633 (1991).

An alternative process for linking by the nucleobases consists in the palladium-catalyzed coupling of bromine or iodine nucleobases with functionalized radicals (Biogenic and Medical Chemistry Letter V, 361 (1994)). By these functionalized radicals, the complexing agent can then optionally be linked with the nucleobase by another linker according to known methods. As functionalized radicals in 5-position of the pyrimidine and in 8-position of the purine, an acrylic ester or an allylamine can be mentioned as examples (see Nucl. Acids Res. 14, 6115 (1986) and Nucl. Acids Res. 16, 4077 (1988)). Another alternative process for preparing 5-position modified pyrimidines, especially for introducing functional groups such as carbonyl, alkenyl or aryl groups at the 5-position, and an improved palladium catalyst capable of coupling modifying groups at the 5-position of pyrimidines is described in U.S. patent application Ser. No. 08/076,735, filed June 14, 1993. The halogen derivatives used as precursor can be obtained as described, e.g., in Biophys. J. 44, 201 (1983), J. Am. Chem. Soc. 86, 1242 (1964) or Chem. Commun. 17 (1967).

The production of the metal complexes according to the invention from the metal-free oligonucleotide conjugates takes place as disclosed in DE 34 01 052, by the metal oxide or a metal salt (for example, the nitrate, acetate, carbonate, chloride or sulfate) of the desired metal isotope being dissolved or suspended in water and/or a lower alcohol (such as methanol, ethanol or isopropanol) and reacted with the solution or suspension of the equivalent amount of the oligonucleotide conjugate containing the complexing agent and then, if desired, present acidic hydrogen atoms being substituted by cations of inorganic and/or organic bases or amino acids or free carboxylic acid groups being converted to amino acid amides.

The neutralization of possibly still present free acid groups takes place with the help of inorganic bases (for example, hydroxides, carbonates or bicarbonates) of, for example, sodium, potassium, lithium, magnesium or calcium and/or organic bases, such as, among others, primary, secondary and tertiary amines, such as, for example, ethanolamine, morpholine, glucamine, N-methyl- and N,N-dimethyl-glucamine, as well as basic amino acids, such as, for example, lysine, arginine and omithine, or of amides of originally neutral or acidic amino acids.

The production of the pharmaceutical agents according to the invention takes place also in a way known in the art, by the oligonucleotide conjugates according to the invention -- optionally by adding the additives usual in galenicals -- being suspended or dissolved in aqueous medium and then the suspension or solution optionally being sterilized or sterilized by filtration. Suitable additives are, for example, physiologically harmless buffers (such as, for example, tromethamine), additives of complexing agents (such as, for example, diethylenetriaminepentaacetic acid) or -- if necessary -- electrolytes, such as, for example, sodium chloride or -- if necessary -- antioxidants, such as, for example, ascorbic acid, or, especially for oral forms of administration, mannitol or other osmotically active substances.

If suspensions or solutions of the agents according to the invention in water or physiological salt solution are desired for enteral administration or other purposes, they can be mixed with one or more adjuvant(s) usual in galenicals (for example, methyl cellulose, lactose, mannitol) and/or surfactant(s) (for example, lecithins, Tween^{TR}, Myrj^{TR}).

The pharmaceutical agents according to the invention preferably contain 0.1 µ mol/l to 3 mmol/l of the oligonucleotide conjugates according to the invention and are generally dosed in amounts of 0.01 nmol/kg - 60 µmol/kg. They are intended for enteral and parenteral administration.

In nuclear medical in vivo use, the labeled compounds generally are dosed in amounts smaller than 10⁻¹⁰ mol/kg of body weight, and the exact dose can vary greatly as a function of the body region studied but especially also as a function of the respectively selected method of study. Starting from an average body weight of 70 kg, the amount of radioactivity for diagnostic uses is between 40 and 1100 MBq, preferably 200-800 MBq, for therapeutic uses 1-500 MBq, preferably 10-100 MBq per administration. The administration takes place normally intravenously, intra-arterially, interstitially, peritoneally or intratumorally, and the intravenous administration is preferred. In general, 0.1 to 20 ml of the agent in question is administered per study.

This invention further relates to a process for detecting target structures. In this case, one or more of the above-described compounds are brought together with the sample to be studied in vivo or in vitro. In this case, oligonucleotide radical N bonds specifically and with high bonding affinity to the target structure to be detected.

If the target structure is present in the sample, it can be detected there based on the signal. The process is especially suitable for a noninvasive diagnosis of diseases. In this case, one or more of the above-described compounds is administered in vivo and it can be detected by the signal whether the target structure, on which oligonucleotide radical N bonds specifically and with high affinity, is present in the organism to be studied.

But in addition to the mere detection of target structures in samples to be studied, the latter can also be specifically destroyed. In this respect, the compounds of this invention are suitable especially in radiotherapy, e.g., in cancer therapy.

Another embodiment of this invention comprises a diagnosis kit for in vivo detection of target structures, which contains one or more of the above-mentioned compounds.

The conjugates and agents according to the invention meet the many requirements that are to be made on a pharmaceutical agent for radiotherapy and diagnosis. They are distinguished especially by a high specificity or affinity relative to the target structure in question. Relative to known oligonucleotide conjugates, the conjugates according to the invention exhibit an especially high in vivo stability. This was achieved by a substitution of the 2'-hydroxyl group and the incorporation of modified thymidine sequences on the terminal hydroxyl groups of the nucleotides. Surprisingly, the specificity of the oligonucleotide is not significantly impaired either by this modification or by the coupling with the complexing agent. Other advantages are the controllable pharmacokinetics as well as the necessary compatibility.

### Brief Description of the Drawings

Various other objects, features and attendant advantages of the present invention will be more fully appreciated as the same becomes better understood when considered in conjunction with the accompanying drawings, wherein:
Figure 1 shows a selection of cyclic complexing agents K, which can be used advantageously for this invention. "b" marks the bonding site on connecting component B.
Figures 2 and 3 show a selection of open-chain complexing agents K, which can be used advantageously for this invention.

The following examples are to illustrate these inventions in more detail.

The polynucleotides described in the examples contain modified compounds.

They mean:
- A, U, C, G: the nucleotides contain a 2'-OCH₃ group
- *:: the intemucleotide bond is a methyl phosphonate
- **:: the internucleotide bond is a thiophosphonate
- ***:: the intemucleotide bond is a dithiophosphonate

Without further elaboration, it is believed that one skilled in the art can, using the preceding description, utilize the present invention to its fullest extent. The following preferred specific embodiments are, therefore, to be construed as merely illustrative and not limitative of the remainder of the disclosure in any way whatsoever.

In the foregoing and in the following examples, all temperatures are set forth uncorrected in degrees Celsius; and, unless otherwise indicated, all parts and percentages are by weight.

### EXAMPLES

### Example 1

### a) 5'-(6-Amino-hexyl-phosphoric acid ester) of the 35mer-oligonucleotide 5'-CUCAUGGAGCGCAAGACGAAUAGCUACAUAT*T*T*T*T-3'

The 30mer-oligonucleotide 5'-CUCAUGGAGCGCAAGACGAAUAGCUACAUA-3', identified according to the SELEX process, with the modification of a sequence T*T*T*T*T-3' placed upstream is produced in the usual way in an automatic synthesizer of the Pharmacia company (see Oligonucleotides and Analogues, A Practical Approach, Ed. F. Eckstein, Oxford University Press, Oxford, New York, Tokyo, 1991), and the oligonucleotide is also present on the column of the solid vehicle. By reaction with trichloroacetic acid solution in dichloromethane, the 5'-hydroxy group is opened. The loading of the column is about 10 mg of 35mer-oligonucleotide. To join the linker, the column is reacted with an acetonitrile solution of 50 µmol of β-cyanoethyl-N,N-diisopropylamino-6-(trifluoroacetamido)-1-hexyl-phosphoramidite (produced according to Nucl. Acids. Res. 16, 2659-2669 (1988)) in the presence of tetrazole. The oxidation of the formed phosphite to the completely protected phosphotriester takes place with iodine in tetrahydrofuran. Then, the column is washed in succession with methanol and water. To remove the modified oligonucleotide from the solid vehicle, the contents of the column are conveyed in a multivial, mixed with 5 ml of 30% ammonia solution, the vessel is sealed and shaken overnight at 55°C. It is then cooled to 0°C, centrifuged, the vehicle is washed with 5 ml of water and the combined aqueous phases are subjected to a freeze-drying.

For purification, the solid material is taken up in 2 ml of water, mixed with 2 ml of 0.5 M ammonium acetate solution and mixed with 10 ml ethanol, it is allowed to stand overnight at -20°C, centrifuged, the residue is washed with 1 ml of ethanol (-20°C) and finally dried in a vacuum at room temperature. 8 mg of the title compound is obtained as colorless powder.

### b) 10-[5-(2-Carboxyphenyl)-2-hydroxy-5-oxo-4-aza-pentyl]-1,4,7-tris(carboxymethyl)-1,4,7,10-tetraazacyclododecane

50 g (144.3 mmol) of 1,4,7-tris(carboxymethyl)-1,4,7,10-tetraazacyclododecane (D03A) is dissolved in 250 ml of water and the pH is adjusted to 13 with 5N sodium hydroxide solution. Then, a solution of 38.12 g (187.6 mmol) of N-(2,3-epoxypropyl)-phthalimide in 100 ml of dioxane is instilled within one hour, stirred for 24 hours at 50°C and the pH is kept at 13 by adding 5N sodium hydroxide solution. The solution is adjusted to pH 2 with 10% hydrochloric acid and evaporated to dryness in a vacuum. The residue is dissolved in some water and purified on an ion exchange column (Reillex^{(R)} = poly-(4-vinyl)-pyridine, it is eluted with water). The main fractions are concentrated by evaporation in a vacuum, and the residue is given a final purification by chromatography on RP-18 (LiChroPrep^{(R)}/mobile solvent: gradient of tetrahydrofuran/methanol/water). After concentration by evaporation of the main fractions, 63.57 g (71% of theory) of an amorphous solid is obtained.
Water content: 8.5%

| Elementary analysis (relative to the anhydrous substance): | | | |
|---|---|---|---|
| Cld | C 52.90 | H 6.57 | N 12.34 |
| Fnd | C 52.65 | H 6.68 | N 12.15 |

### c) 10-(3-Amino-2-hydroxy-propyl)-1,4,7-tris(carboxy-methyl)-1,4,7,10-tetraazacyclododecane

50 g (88.1 mmol) of the title compound of example 1b is refluxed in 300 ml of concentrated hydrochloric acid for 24 hours. It is evaporated to dryness, the residue is dissolved in some water and purified on an ion exchange column (Reillex^{(R)} = poly-(4-vinyl)-pyridine (it is eluted with water)). The main fractions are evaporated to dryness.
Yield: 39 g (95% of theory) of a vitreous solid.
Water content: 10.3%

| Elementary analysis (relative to the anhydrous substance): | | | |
|---|---|---|---|
| Cld | C 48.68 | H 7.93 | N 16.70 |
| Fnd | C 48.47 | H 8.09 | N 16.55 |

### d) 10-[7-(4-Nitrophenyl)-2-hydroxy-5-oxo-7-(carboxy-methyl)-4-aza-heptyl)-1,4,7-tris(carboxymethyl)-1,4,7,10-tetraazacyclododecane

9.84 g (41.8 mmol) of 3-(4-nitrophenyl)-glutaric anhydride (J. Org. Chem. 26, 3856 (1961)) is added to 14.62 g (34.86 mmol) of the title compound of example 1c) in 200 ml of dimethylformamide/20 ml of triethylamine and stirred overnight at room temperature. It is evaporated to dryness in a vacuum. The residue is recrystallized from isopropanol/acetic acid 95:5.
Yield: 21.68 g (95% of theory) of a yellowish solid
Water content: 0.9%

| Elementary analysis (relative to anhydrous substance): | | | |
|---|---|---|---|
| Cld: | C 51.37 | H 6.47 | N 12.84 |
| Fnd: | C 51.18 | H 6.58 | N 12.67 |

### e) 10-[7-(4-Aminophenyl)-2-hydroxy-5-oxo-7-(carboxy-methyl)-4-aza-heptyl]-1,4,7-tris(carboxymethyl)-1,4,7,10-tetraazacyclododecane

21.0 g (32.07 mmol) of the title compound of example 1d) is dissolved in 250 ml of methanol and 5 g of palladium catalyst (10% Pd on C) is added. It is hydrogenated overnight at room temperature. The catalyst is filtered off and the filtrate is evaporated to dryness in a vacuum.
Yield: 19.63 g (98% of theory) of a cream-colored solid
Water content: 0.8%

| Elementary analysis (relative to anhydrous substance): | | | |
|---|---|---|---|
| Cld | C 53.84 | H 6.35 | N 12.60 |
| Fnd | C 53.73 | H 6.45 | N 12.51 |

### f) 10-[7-(4-Isothiocyanatophenyl)-2-hydroxy-5-oxo-7-(carboxymethyl)-4-aza-heptyl]-1,4,7-tris(carboxy-methyl)-1,4,7,10-tetraazacyclododecane

12.4 g (19.27 mmol) of the title compound of example 1e) is dissolved in 200 ml of water and 6.64 g (57.8 mmol) of thiophosgene in 50 ml of chloroform is added. It is stirred for 1 hour at 50°C. It is cooled to room temperature, the organic phase is separated and the aqueous phase is shaken out twice with 100 ml of chloroform. The aqueous phase is evaporated to dryness and the residue is absorptively precipitated in 100 ml of isopropanol at room temperature. The solid is filtered off and washed with ether. After drying overnight in a vacuum (40°C), 12.74 g (97% of theory) of a cream-colored solid is obtained.
Water content: 3.1%

| Elementary analysis (relative to anhydrous substance): | | | | |
|---|---|---|---|---|
| Cld | C 52.24 | H 6.35 | N 12.60 | S 4.81 |
| Fnd | C 52.37 | H 6.44 | N 12.48 | S 4.83 |

### g) Conjugate of 5-(6-amino-hexyl-phosphoric acid ester) of the 35mer oligonucleotide 5'-CUCAUGGAGCGCAAGACGAAUAGCUACAUAT*T*T*T*T-3' and 10-[7-(4-isothiocyanato-phenyl)-2-hydroxy-5-oxo-7-(carboxymethyl)-4-azaheptyl]-1,4,7-tris(carboxymethyl)-1,4,7,10-tetraazacyclododecane

8 mg of the oligonucleotide obtained in example 1a) is dissolved in 2.5 ml of a NaHCO₃/Na₂CO₃ buffer (pH 8.0) and mixed with 1 mg of 10-[7-(4-isothiocyanatophenyl)-2-hydroxy-5-oxo-7-(carboxymethyl)-4-aza-heptyl]-1,4,7-tris-(carboxymethyl)-1,4,7,10-tetraazacyclododecane (title compound of example 1f). It is stirred for 5 hours at room temperature, the pH is adjusted to 7.2 by adding 0.01 M hydrochloric acid and the solution is subjected to an ultrafiltration through a membrane with the exclusion limit 3,000 (Amicon YM3) and then a freeze-drying. 7 mg of the desired conjugate is obtained.

### h) ¹¹¹Indium complex of the thiourea conjugate of 5-(6-amino-hexyl-phosphoric acid ester) of the 35mer oligonucleotide 5-'CUCAUGGAGCGCAAGACGAAUAGCUACAUAT*T*T*T*T-3' and 10-[7-(4-isothiocyanatophenyl)-2-hydroxy-5-oxo-7-(carboxymethyl)-4-azaheptyl]-1,4,7-tris(carboxymethyl)-1,4,7,10-tetraazacyclododecane

15 µl of an ¹¹¹indium(III) acetate solution (350 µCi), (produced from ¹¹¹indium(III) chloride in 2 M sodium acetate solution and adjustment of the pH to 4.0 with 0.1 M hydrochloric acid) is added to 135 µl of a solution of 1 mg of the title compound of example 1g) in MES buffer, pH 6.2 (MES = 2-(N-morpholino)ethylsulfonic acid). The pH is brought to 4.2 by adding 0.01 M hydrochloric acid. It is stirred for 1 hour at 37°C at pH 4.2. It is brought to pH 6 with 2 M sodium acetate solution and 10 µl of a 0.1 M Na₂EDTA = ethylenediamine-tetraacetic acid disodium salt is added to complex excess ¹¹¹indium. The final purification of thus obtained labeled conjugate (1h) takes place by HPLC (exclusion chromatography: TSK-400/MES-buffer). The fractions containing the labeled conjugate are diluted with physiological common salt solution, adjusted to pH 7.2 with 0.01 M sodium hydroxide solution and filtered. A thus produced solution then represents a suitable preparation for radiodiagnosis.

### Example 2

### a) Conjugate of 5'(6-amino- 1-hexyl-phosphoric acid ester) of the 35mer oligonucleotide 5'-CUCAUGGAGCGCAAGACGAAUAGCUACAUAT*T*T*T*T-3' and N-[2-amino-3-(4-isothiocyanatophenyl)-propyl]-trans-cyclohexane-1,2-diamine-N,N'-N',N",N"-pentaacetic acid

8 mg of the oligonucleotide obtained in example 1a) is dissolved in 2.5 ml of a NaHCO₃/Na₂CO₃ buffer (pH 8.0) and 1 mg of N-[2-amino-3-(p-isothiocyanatophenyl)propyl]-trans-cyclohexane-1,2-diamine-N,N',N',N",N"-pentaacetic acid is added (produced according to Bioconjugate Chem. 1, 59 (1990)). It is stirred for 5 hours at room temperature, then adjusted to pH 7.2 with 0.1 M hydrochloric acid and the solution is subjected to an ultrafiltration through a membrane with the exclusion limit 3,000 (Amicon YM3). After freeze-drying, 6 mg of thiourea conjugate 2a) is obtained.

### b) Bismuth-212 complex of the conjugate of 5'(6-amino-1-hexyl-phosphoric acid ester) of the 35mer oligonucleotide 5'-CUCAUGGAGCGCAAGACGAAUAGCUACAUAT*T*T*T*T-3' and N-[2-amino-3-(4-isothiocyanatophenyl)-propyl]-trans-cyclohexane-1,2-diamine-N,N'-N',N",N"-pentaacetic acid

A ²¹²bismuth-tetraiodide solution in 0.1 M of hydroiodic acid is brought to pH 4 with 2 M acetic acid. An aliquot of this solution of the activity of about 3 mCi is added to 1 mg of the title compound of example 2a), dissolved in 0.5 ml of 0.02 M MES-buffer and 0.5 ml of 0.15 M sodium chloride solution is added. It is stirred for 20 minutes at room temperature. It is brought to pH 6 with 2 M sodium acetate solution and 20 µl of a 0.01 M Na₂EDTA solution is added. It is stirred for 20 minutes. The purification of the complex takes place by HPLC (exclusion chromatography: TSK-400/MES-buffer). The radioactive conjugate fractions are combined, diluted with physiological common salt solution, and adjusted to pH 7.2 with 0.01 M sodium hydroxide solution. After filtration, a preparation suitable for radiotherapy is obtained.

### Example 3

### a) Indium-111 complex of the conjugate of 5'(6-amino-1-hexyl-phosphoric acid ester) of the 35mer oligonucleotide 5'-CUCAUGGAGCGCAAGACGAAUAGCUACAUAT*T*T*T*T-3' and N-[2-amino-3-(4-isothiocyanatophenyl)-propyl]-trans-cyclohexane-1,2-diamine-N,N'-N',N",N"-pentaacetic acid

15 ml of a 15 µl of an ¹¹¹indium(III) acetate solution (350 µCi) (produced from ¹¹¹indium(III) chloride in 2 M sodium acetate solution and adjustment of the pH to 4.0 with 0.1 M hydrochloric acid) is added to 0.5 ml of a solution of 1 mg of the title compound of example 2a) in MES-buffer, pH 6.2 (MES = 2-(N-morpholino)ethyl-sulfonic acid). The pH is brought to 5.0 by adding 0.01 M hydrochloric acid. It is stirred for 1 hour at 37°C at pH 5.0. It is brought to pH 6 with 2 M sodium acetate solution and 10 µl of a 0.1 M Na₂EDTA = ethylenediamine-tetraacetic acid disodium salt is added to complex excess ¹¹¹indium. The final purification of thus obtained labeled conjugate (1h) takes place by HPLC (exclusion chromatography: TSK-400/MES-buffer). The fractions containing the labeled conjugate are diluted with physiological common salt solution, adjusted to pH 7.2 with 0.01 M sodium hydroxide solution and filtered. A thus produced solution then represents a suitable preparation for radiodiagnosis.

### Example 4

### a) Conjugate of 5'-(6-amino-1-hexyl-phosphoric acid ester) of the 35mer oligonucleotide 5'-CUCAUGCAGCGCAAGACGAAUAGCUACAUAT*T*T*T*T-3' and 2-(4-isothiocyanato-benzyl)-diethylenetriamine-N,N,N',N",N"-pentaacetic acid

8 mg of the oligonucleotide obtained in example 1a) is dissolved in 2.5 ml of a NaHCO₃/Na₂CO₃ buffer (pH 8.0) and 1 mg of 2-(4-isothiocyanato-benzyl)-diethylenetriamine-N,N',N',N",N"-pentaacetic acid is added (produced according to: Bioconjugate Chem. 2, 187 (1991)). It is stirred for 5 hours at room temperature, then adjusted to pH 7.2 with 0.01 M hydrochloric acid and the solution is subjected to an ultrafiltration through a membrane with the exclusion limit 3,000 (Amicon YM 3). After freeze-drying, 6 mg of the thiourea conjugate is obtained.

### b) Yttrium-90 complex of the conjugate of 5'-(6-amino-1-hexyl-phosphoric acid ester) of the 35mer oligonucleotide 5'-CUCAUGCAGCGCAAGACGAAUAGCUACAUAT*T*T*T*T-3' and 2-(4-isothiocyanato-benzyl)-diethylenetriamine-N,N,N',N",N"-pentaacetic acid

A solution of ⁹⁰yttrium, dissolved in 0.05 M ammonium acetate solution (about 380 mCi), is added to 1 mg of the thiourea derivative of example 4a) in 0.5 ml of 0.05 M ammonium acetate solution of pH 6, adjusted to pH 5.2 with 3 M acetic acid and stirred for 1 hour at room temperature. It is adjusted to pH 7.0 with 0.01 M sodium hydroxide solution and the conjugate is purified by HPLC (TSK-400/MES-buffer). The main fractions are combined, diluted with physiological common salt solution and brought to pH 7.2 with 0.01 M sodium hydroxide solution. After filtration, a preparation suitable for the radiotherapy is obtained.

### Example 5

### a) 5'-(6-Mercapto-1-hexyl-phosphoric acid ester) of the 35mer-oligonucleotide 5'-T*T*T*T*TAGGAGGAGGAGGGAGAGCGCAAAUGAGAUU-3' (modified ligand for serine protease)

The 30mer-oligonucleotide 5'-AGGAGGAGGAGGGAGAGCGCAAAUGAGAUU-3' (seq. no. 13 of US Patent No. 5,270,163) identified according to the SELEX process, modified in the sugar units and by a 5'-linked sequence of 5 tymidines, is produced in the usual way in an automatic synthesizer of the Pharmacia company (see Oligonucleotides and Analogues, A Practical Approach, Ed. F. Eckstein, Oxford University Press, Oxford, New York, Tokyo, 1991), and the oligonucleotide is also present on the column of the solid vehicle. By reaction with trichloroacetic acid solution in dichloromethane, the 5'-hydroxy group is opened. The loading of the column is about 10 mg of 35mer-oligonucleotide. To join the linker, the column is reacted with a solution of 50 µmol of β-cyanoethyl-N,N-diisopropylamino-S-trityl-6-mercapto)-phosphoramidite in acetonitrile in the presence of tetrazole. The oxidation of the formed phosphite to the completely protected phosphotriester takes place with iodine in tetrahydrofuran. Then, the column is washed in succession with methanol and water. To remove the modified oligonucleotide from the solid vehicle, the contents of the column are conveyed in a multivial, mixed with 5 ml of 30% ammonia solution, the vessel is sealed and shaken overnight at 55°C. It is then cooled to 0°C, centrifuged, the vehicle is washed with 5 ml of water and the combined aqueous phases are subjected to a freeze-drying.

For purification, the solid material is taken up in 2 ml of water, mixed with 2 ml of 0.5 M ammonium acetate solution and mixed with 10 ml ethanol, it is allowed to stand overnight at -20°C, centrifuged, the residue is washed with 1 ml of ethanol (-20°C) and finally dried in a vacuum at room temperature.

9 mg of the S-tritylated title compound is obtained. To cleave the trityl protective group, the product is dissolved in 0.5 ml of water, mixed with 0.1 ml of 1 M silver nitrate solution and stirred for 1 hour at room temperature. Then, it is mixed with 0.1 ml of 1 M dithiothreitol solution. After 15 minutes, it is centrifuged, and the supernatant solution is extracted several times with ethyl acetate. After the freeze-drying, 8 mg of the desired title compound is obtained from the aqueous solution.

### b) 4-Benzyloxy-N-methanesulfonyl-phenylalanine-methylester

19.58 g of methanesulfonic acid chloride is instilled in 50 g of 4-benzyloxy-phenylalanine-methylester-hydrochloride in 300 ml of pyridine at 0°C and stirred for 3 hours at 0°C. It is evaporated to dryness in a vacuum and the residue is dissolved in 500 ml of dichloromethane. It is shaken out twice with 300 ml of 5N hydrochloric acid each, dried on magnesium sulfate and concentrated by evaporation in a vacuum. The residue is recrystallized from 150 ml of methanol.
Yield: 53.64 g of a colorless crystalline powder.

### c) 2-(4-Benzyloxybenzyl)-1-methanesulfonyl-1,4,7-triazaheptan-3-one

37.2 g of 4-benzyloxy-N-methanesulfonyl-phenylalanine-methylester and 1.2 l of 1,2-diaminoethane are stirred for 3 hours at 80°C. The residue is evaporated to dryness and absorptively precipitated with 200 ml of water, the precipitate is suctioned off, washed neutral with water and dried overnight at 60°C.
Yield: 37.68 g of a cream-colored, amorphous powder.

### d) 2-(4-Benzyloxybenzyl)-1-methanesulfonyl-7-(tert-butyloxycarbonyl)-1,4,7-triazaheptan-3-one

A solution of 16.23 g of 2-(4-benzyloxybenzyl)-1-methanesulfonyl-1,4,7,-triazaheptan-3-one and 4.76 g of triethylamine in 200 ml of chloroform is mixed at 0°C with a solution of 10.27 g of di-tert-butyl-dicarbonate in 50 ml of chloroform. It is stirred for 5 hours at room temperature, shaken with 5% sodium carbonate solution and water, dried on magnesium sulfate and concentrated by evaporation in a vacuum. The residue is recrystallized from 100 ml of methanol.
Yield: 20.19 g of a foamy solid.

### e) 2-(4-Hydroxybenzyl)-1-methanesulfonyl-7-(tert-butyloxycarbonyl)-1,4,7-triazaheptan-3-one

20 g of 2-(4-benzyloxybenzyl)-1-methanesulfonyl-7-(tert-butyloxycarbonyl)-1,4,7-triazaheptan-3-one, dissolved in 300 ml of dichloromethane, is stirred with 4 g of palladium-carbon (10%) overnight under a hydrogen atmosphere. It is filtered and the solution is concentrated by evaporation in a vacuum.
Yield: 16.17 g of a vitreous foam, which solidifies after a few minutes.

### f) 2-[4-(3-Oxapropionic acid-benzylester)-benzyl]-1-methanesulfonyl-7-(tert-butyloxycarbonyl)-1,4,7-triazaheptan-3-one

15 g of 2-(4-hydroxybenzyl)-1-methanesulfonyl-7-(tert-butyloxycarbonyl)-1,4,7-triazaheptan-3-one, 8.56 g of bromoacetic acid-benzyl ester and 13.18 g of potassium carbonate are refluxed in 300 ml of acetonitrile for 24 hours. It is filtered and evaporated to dryness in a vacuum. The residue is dissolved in 200 ml of dichloromethane, shaken out twice with 50 ml of water each. The organic phase is dried on magnesium sulfate and concentrated by evaporation in a vacuum. The residue is chromatographed with dichloromethane-hexane-acetone (20/10/1) as eluent.
Yield: 10.06 g of foamy solid

### g) 2-[4-(3-Oxapropionic acid-benzylester)-benzyl]-1-methanesulfonyl-1,4,7-triazaheptan-3-one

10 g of 2-[4-(3-oxapropionic acid-benzylester)-benzyl]-1-methanesulfonyl-7-(tert-butyloxycarbonyl)-1,4,7-triazaheptan-3-one is stirred for 1 hour at room temperature with 100 ml of trifluoroacetic acid. It is evaporated to dryness in a vacuum.
Yield: 9.2 g of vitreous foam, which solidifies while standing.

### h) 9-Chloro-1-methanesulfonyl-2-[4-(3-oxapropionic acid-benzylester)-benzyl]-1,4,7-triaza-3,8-dione

9 g of 2-[4-(3-oxapropionic acid-benzylester)-benzyl]-1-methanesulfonyl-1,4,7-triazaheptan-3-one and 1.78 g of triethylamine are dissolved in 200 ml of chloroform. At 0°C, 1.98 g of chloroacetyl chloride, dissolved in 20 ml of chloroform, is instilled within 30 minutes and then stirred for 2 hours at 0°C. It is washed with 100 ml of 5% hydrochloric acid, twice with 50 ml of water each, dried on magnesium sulfate and evaporated to dryness in a vacuum. The residue is chromatographed on silica gel with dichloromethane-ethyl acetate (20/1) as eluent.
Yield: 6.97 g of waxy solid

### i) 9-Chloro-1-methanesulfonyl-2-[4-(3-oxapropionic acid-benzylester)-benzyl]-1,4,7-triaza-3,8-dione

6.5 g of 9-chloro-1-methanesulfonyl-2-[4-(3-oxapropionic acid-benzylester)-benzyl]-1,4,7-triaza-3,8-dione, dissolved in 150 ml of dichloromethane, is stirred with 2 g of palladium-carbon (10%) overnight under a hydrogen atmosphere. It is filtered and the solution is concentrated by evaporation in a vacuum.
Yield: 5.33 g of vitreous solid

### j) 10-Acetyl-2-[4-(3-oxapropionic acid)-benzyl]-1-(methanesulfonyl)-10-thia-1,4,7-triazadecane-3,8-dione

5 g of 9-chloro-1-methanesulfonyl-2-[4-(3-oxapropionic acid-benzylester)-benzyl]-1,4,7-triaza-3,8-dione, dissolved in 80 ml of chloroform, is refluxed with 1.98 g of triethylamine and 0.74 g of thioacetic acid for 10 minutes. The solution is poured in 200 ml of ice-cold 5% hydrochloric acid, the organic phase is separated, dried on magnesium sulfate and concentrated by evaporation in a vacuum. After chromatography on silica gel with hexane-ethyl acetate (3/1), 4.64 g of the desired compound is obtained as vitreous solid.

### k) 10-Acetyl-2-[4-(3-oxapropionic acid-(2,5-dioxo-pyrrolidin-1-yl)-ester)-benzyl]-1-(methanesulfonyl)-10-thia-1,4,7-triazadecane-3,8-dione

4 g of 10-acetyl-2-[4-(3-oxapropionic acid)-benzyl]-1-(methanesulfonyl)-10-thia-1,4,7-triazadecane-3,8-dione, 1.91 g of dicyclohexylcarbodiimide, 4 g of N-hydroxysuccinimide and 30 mg of 4-dimethylaminopyridine are stirred for 24 hours in 20 ml of chloroform at room temperature. It is then mixed with 20 ml of diethyl ether, filtered, and the residue is concentrated by evaporation in a vacuum. The residue is chromatographed on silica gel with dichloromethane-dioxane (10/1) as eluent.
Yield: 3.47 g of a cream-colored solid

| Elementary analysis: | | | | |
|---|---|---|---|---|
| Cld | C 46.15 | H 4.93 | N 9.78 | S 11.20 |
| Fnd | C 46.03 | H 4.83 | N 9.64 | S 11.05 |

### l) 10-Acetyl-2-{4-[3-oxapropionic acid-(6-maleimido-hexanoyl)-hydrazide]-benzyl}-1-methanesulfonyl-10-thia-1,4,7-triazadecane-3,8-dione

3 g of 10-acetyl-2-[4-(3-oxapropionic acid-(2,5-dioxo-pyrrolidin-1-yl)-ester)-benzyl]-1-(methanesulfonyl)-10-thia-1,4,7-triazadecane-3,8-dione and 1.17 g of 6-male-imidocaproic acid hydrazide (Science 261, 212 (1993)) are stirred in 40 ml of dimethylformamide for 5 hours at 60°C. With vigorous stirring, 100 ml of water is instilled and filtered off from the precipitated precipitate. It is dried in a vacuum and the residue is purified by a FLASH chromatography on a silica gel column with dichloromethane/dioxane (10/1) as eluent. 2.8 g of the title compound is obtained as white solid.

| Elementary analysis (relative to anhydrous substance): | | | | |
|---|---|---|---|---|
| Cld | C 49.25 | H 5.61 | N 12.30 | S 9.39 |
| Fnd | C 49.33 | H 5.95 | N 12.43 | S 9.11 |

### m) Conjugate of 5'-(6-mercapto-1-hexyl-phosphoric acid ester) of the 35mer-oligonucleotide 5'-T*T*T*T*TAGGAGGAGGAGGGAGAGCGCAAAUGAGAUU-3' and 10-acetyl-2-{4-[3-oxapropionic acid-(6-maleimido)-hexanoyl)-hydrazide]-benzyl}-1-methanesulfonyl-10-thia-1,4,7-triazadecane-3,8-dione

5 mg of the thiol-containing oligonucleotide produced according to example 5a) is mixed in 2 ml of phosphate buffer (pH 7.4) with 1 mg of the maleimide derivative, produced according to example 51), dissolved in 0.2 ml of dimethylformamide. It is allowed to stand for 2 hours at room temperature, and the solution is subjected to an ultrafiltration through a membrane with the exclusion limit 3,000 (Amicon YM 3) and then a freeze-drying. 5 mg of the desired conjugate is obtained.

### n) Technetium-99m complex of the conjugate of 5'-(6-Mercapto-1-hexyl-phosphoric acid ester) of the 35mer-oligonucleotide 5'-T*T*T*T*TAGGAGGAGGAGGGAGAGCGCAAAUGAGAUU-3' and 10-acetyl-2-{4-[3-oxapropionic acid-(6-maleimido)-hexanoyl)-hydrazide]-benzyl}-1-methanesulfonyl-10-thia-1,4,7-triazadecane-3,8-dione

1 ml of a solution of potassium-D-glucarate (12 mg/ml) and tin(II) chloride (100 µg/ml) in 0.2 M NaHCO₃ are freezed-dried in a vial and then mixed with [Tc-99m]-sodium pertechnetate solution (1 ml, 1 mCi) from an Mo-99/Tc-99m generator. After standing at room temperature for 15 minutes, an aliquot is removed and mixed with the same volume of the conjugate of example 5m) (1 mg/ml) dissolved in 0.2 M NaHCO₃ solution. After 15 minutes, both thin-layer chromatography and HPLC showed that > 98% of the radioactivity was taken up by the conjugate.

### Example 6

### a) Conjugate of 5'-(6-amino-1-hexyl-phosphoric acid ester) of the 35mer-oligonucleotide 5'-CUCAUGGAGCGCAAGACGAAUAGCUACAUAT*T*T*T*T-3' and S-benzoyl MAG₃-2,3,5,6-tetrafluorophenylester

3 mg of S-benzoyl-MAG₃-2,3,5,6-tetrafluorophenylester (produced according to US 4,965,392), dissolved in 0.2 ml of dimethylformamide, is added to 8 mg of the title compound of example 1a), dissolved in 0.5 ml of 0.1 M phosphate buffer (pH 7), and stirred for 3 hours at room temperature. It is diluted with water and the solution is subjected to an ultrafiltration (Amicon YM 3, exclusion limit 3,000). After freeze-drying, 5 mg of conjugate 6a) is obtained.

### b) ^{99m}Technetium complex of the conjugate of 5'-(6-amino-1-hexyl-phosphoric acid ester) of the 35mer-oligonucleotide 5'-CUCAUGGAGCGCAAGACGAAUAGCUACAUAT*T*T*T*T-3' and S-benzoyl MAG₃-2,3,5,6-tetrafluorophenylester

1 mg of the title compound of example 6a) is dissolved in 200 µl of water and mixed with 1 ml of 0.1 M phosphate buffer of pH 8.5. 200 µl of ^{99m}technetium-(V)-gluconate solution (about 15 mCi) is added to this mixture and allowed to stand for 15 minutes at room temperature. The tracer yield (determined by HPLC) is about 95%.

### Example 7

### a) Conjugate of the 5'-(6-amino-1-hexyl-phosphoric acid ester) of the 35mer-oligonucleotide 5'-CUCAUGGAGCGCAAGACGAAUAGCUACAUAT*T*T*T*T-3' and the ^{99m}technetium complex of 2,3,5,6-tetrafluorophenyl-4,5-bis(mercaptoacetamido)-pentanoic acid ester

3 mg of the title compound of example 1a), dissolved in 0.6 ml of phosphate buffer, is added to the ^{99m}technetium complex of 2,3,5,6-tetrafluorophenyl-4,5-bis(mercaptoacetamido)-pentanoic acid ester (produced according to: J. Nucl. Med. 32, 1445 (1991)) about 100 mCi, dissolved in 2 ml of phosphate buffer, pH 7.2. It is adjusted to pH 10 with 1.0 M potassium carbonate buffer and stirred for 20 minutes at room temperature. For final purification, the solution is added to a Sephadex column (Pharmacia) and eluted with 75 mmol of sodium chloride solution. The main fractions are combined, diluted with physiological common salt solution and filtered. The thus obtained solution can be used for radiodiagnostic studies.

### Example 8

### a) Conjugate of 5'-(6-mercapto-1-hexyl-phosphoric acid ester) of the 35mer-oligonucleotide 5'-T*T*T*T*TAGGAGGAGGAGGGAGAGCGCAAAUGAGAUU-3'and 5'(N-maleimido)-3-oxapentyl-{2-[3-carboxybenzoyl)-thio]-acetyl}-glycylglycylglycinate

5 mg of the thio-containing oligonucleotide produced according to example 5a) is mixed under N₂ in 2 ml of phosphate buffer (pH 7.4) with 1 mg of 5-(N-maleimido)-3-oxapentyl-{2-[3-carboxy-benzoyl)-thio]-acetyl}-glycylglycylglycinate (produced according to Bioconj. Chem. 1, 431 (1990)), dissolved in 0.2 ml of dimethylformamide. It is allowed to stir for 2 hours at room temperature and the solution is subjected to an ultrafiltration through a membrane (Amicon YM 3) and then a freeze-drying. 5.5 mg of the desired conjugate is obtained.

### b) Technetium-99m complex of the conjugate 5'-(6-mercapto-1-hexyl-phosphoric acid ester) of the 35mer-oligonucleotide 5'-T*T*T*T*TAGGAGGAGGAGGGAGAGCGCAAAUGAGAUU-3'and 5'(N-maleimido)-3-oxapentyl-{2-[3-carboxybenzoyl)-thio]-acetyl}-glycylglycylglycinate

1 ml of a solution ofpotassium-D-glucarate (12 mg/ml) and tin(II) chloride (100 µg/ml) in 0.2 M NaHCO₃ is freeze-dried in a vial and then mixed with [Tc-99m]-sodium pertechnetate solution (1 ml, 1 mCi) from an Mo-99/Tc-99m generator. After standing at room temperature for 15 minutes, an aliquot is removed and mixed with the same volume of the conjugate of example 8a) (1 mg/ml) dissolved in 0.2 M NaHCO₃ solution. The substance can be isolated by freeze-drying. The tracer yield determined by HPLC is > 96%.

### Example 9

### a) Conjugate of 5'-(6-mercapto-1-hexyl-phosphonic acid ester) of the 35mer-oligonucleotide 5'-T*T*T*T*TAGGAGGAGGAGGGAGAGCGCAAAUGAGAUU-3' and 1-[6-(2-vinyl-6-hexyloxymethyl)-pyridine]-1,4,8,11-tetraazacyclotetradecane

A solution of 4 mg of the thio-containing oligonucleotide, produced according to example 5a), in 2 ml of phosphate buffer (pH 7.4) is mixed under N₂ with 1 mg of 1-[6-(2-vinyl-6-hexyl-oxymethyl)-pyridine]-1,4,8,11-tetraazacyclotetradecane (produced according to EP 0 588 229), dissolved in 0.5 ml of dimethylformamide. It is allowed to stir for 4 hours at 35°C, mixed with 10 ml of ethanol and the product is isolated by centrifuging. The purification takes place by reversed-phase chromatography on a 1 x 25 cm column with a 50 mmol triethylammonium acetate (pH 7) acetonitrile gradient. The combined fractions are freeze-dried, dissolved in 1 ml of water and desalted on a Sephadex G-10 column. The title compound (about 4 mg) is isolated by freeze-drying.

### b) Tc-99m complex of the conjugate of 5'-(6-mercapto-1-hexyl-phosphonic acid ester) of the 35mer-oligonucleotide. 5'-T*T*T*T*TAGGAGGAGGAGGGAGAGCGCAAAUGAGAUU-3' and 1-[6-(2-vinyl-6-hexyloxymethyl)-pyridine]-1,4,8,11-tetraazacyclotetradecane

The procedure is performed as described in example 8b). The tracer yield, determined by HPLC, is 92%.

### Example 10

### a) Conjugate of 5'-(6-mercapto-1-hexyl-phosphonic acid ester) of the 35mer oligonucleotide 5'-T*T*T*T*TAGGAGGAGGAGGGAGAGCGCAAAUGAGAUU-3' and N-[4-hydroxy-3-(1,4,8,11-tetraaza-cyclotetradec-5-yl)-benzyl]-2-(6-vinyl-pyridin-2-ylmethoxy)-acetamide

A solution of 6.5 mg of the thiol-containing oligonucleotide, produced according to example 5a), in 2 ml of phosphate buffer (pH 8.0) is mixed with 1.2 mg of N-[4-hydroxy-3-(1,4,8,11-tetraaza-cyclotetradec-5-yl)-benzyl-2-(6-vinyl-pyridin-2-ylmethoxy)-acetamide (produced according to J. Chem. Soc., Chem. Commun. 156 (1988)), dissolved in 0.1 ml of dimethylformamide. It is stirred for 4 hours under N₂ at 35°C, mixed with 10 ml of ethanol and the product is isolated by centrifuging. The purification takes place by reversed-phase chromatography on a 1 x 25 cm column with a 50 mmol triethylammonium acetate (pH 7)/acetonitrile gradient. The combined fractions are desalted on a Sephadex-G-10 column. By freeze-drying, 5 mg of the title compound is obtained as white powder.

### b) Copper-64 complex of the conjugate of 5'-(6-mercapto-1-hexyl-phosphonic acid ester) of the 35mer oligonucleotide 5'-T*T*T*T*TAGGAGGAGGAGGGAGAGCGCAAAUGAGAUU-3' and N-[4-hydroxy-3-(1,4,8,11-tetraaza-cyclotetradec-5-yl)-benzyl]-2-(6-vinyl-pyridin-2-ylmethoxy)-acetamide

1 mg of the conjugate obtained according to 10a) is incubated in 1 ml of phosphate buffer (pH 8) with ⁶⁴CuCl₂ (0.2 mCi). The tracer yield, determined after 1 hour by HPLC, is > 98%. The product is isolated by freeze-drying.

### Example 11

### a) Conjugate of 5'-(6-mercapto-1-hexyl-phosphoric acid ester) of the 35mer oligonucleotide 5'-T*T*T*T*TAGGAGGAGGAGGGAGAGCGCAAAUGAGAUU-3' and 1,4,7,10-tetraazacyclododecane-2-[(5-aza-8-maleimido-6-oxo)-octane]-1,4,7,10-tetraacetic acid

1 mg of 1,4,7,10-tetraazacyclododecane-2-[(5-aza-8-maleimido-6-oxo)-octane]-1,4,7,10-tetraacetic acid (produced according to J. Chem. Soc., Chem. Commun. 796, (1989)) is added to a solution of 5 mg of the thiol-containing oligonucleotide, produced according to example 5a), in 2 ml of phosphate buffer (pH 8) under N₂. It is stirred for 3 hours at 35°C, mixed with 10 ml of isopropyl alcohol and the product is isolated by centrifuging. The purification takes place by reversed-phase chromatography on a 1 x 25 cm column with a 25 mmol triethylammonium acetate (pH 7)/acetonitrile gradient. The combined fractions are gently concentrated by evaporation in a vacuum, dissolved in a little water and desalted with the help of a Sephadex-G-10 column. By freeze-drying, 4 mg of the title compound is obtained as white powder.

### b) Yttrium-90 complex of the conjugate of 5'-(6-mercapto-1-hexyl-phosphonic acid ester) of the 35mer oligonucleotide 5'-T*T*T*T*TAGGAGGAGGAGGGAGAGCGCAAAUGAGAUU-3' and 1,4,7,10-tetraazacyclododecane-2-[(5-aza-8-maleimido-6-oxo)-octane]-1,4,7,10-tetraacetic acid

⁹⁰Y-acetate (1 mCi), dissolved in 1 ml of 0.05 M ammonium acetate solution, is mixed with 1 mg of the conjugate produced according to example 11a) and heated for 1 hour to 85°C. The tracer yield, determined by HPLC, is > 95%. The product is isolated by freeze-drying.

### Example 12

### a) Conjugate of 5'-(6-mercapto-1-hexyl-phosphonic acid ester) of the 35mer oligonucleotide 5'-T*T*T*T*TAGGAGGAGGAGGGAGAGCGCAAAUGAGAUU-3' and 1,4,7-triazacyclononane-2-[(5-aza-8-maleimido-6-oxo)-octane]-1,4,7-triacetic acid

1 mg of 1,4,7,10-triazacyclononane-2-[(5-aza-8-maleimido-6-oxo)-octane]-1,4,7-triacetic acid (produced according to J. Chem. Soc., Chem. Commun. 794, (1989)) is added to a solution of 5 mg of the thiol-containing oligonucleotide, produced according to example 5a), in 2 ml of phosphate buffer (pH 8) under N₂. It is stirred for 6 hours at 35°C, mixed with 10 ml of isopropanol and the product is isolated by centrifuging. The purification takes place by reversed-phase chromatography on a 1 x 25 cm column with a 25 mmol triethylammonium acetate (pH 7)/acetonitrile gradient. The combined fractions are gently concentrated by evaporation in a vacuum, dissolved in a little water and desalted with the help of a Sephadex G-10 column. By freeze-drying, 3 mg of the title compound is obtained as white powder.

### b) Gallium-67 complex of the conjugate of 5'-(6-mercapto-1-hexyl-phosphonic acid ester) of the 35mer oligonucleotide 5'-T*T*T*T*TAGGAGGAGGAGGGAGAGCGCAAAUGAGAUU-3' and 1,4,7-triazacyclononane-2-[(5-aza-8-maleimido-6-oxo)-octane]-1,4,7-triacetic acid

20 mg of the conjugate produced according to example 12a) is dissolved in 0.5 ml of 0.1 M citrate buffer of pH 4.5 and mixed with 0.1 ml of a gallium-67-citrate solution (0.2 mCi). It is allowed to stand for 2 hours at room temperature and the product is desalted on a Sephadex-G-10 column. After freeze-drying, 17 mg of the title compound is obtained as white powder.

### Example 13

### a) Phosphitylation of 5'-0-(4,4'-dimethoxytrityl)-5-(prop-2-en-1-one)-2'-deoxyuridine

50 mg of 4-dimethylaminopyridine, 3 ml of diisopropylethylamine and 962 µl (4.31 mmol) of 2-cyanoethyl-N,N-diisopropylchlorophosphoramidite are added in succession to a stirred solution of 2.1 g (3.59 mmol) of 5'-0-(4,4'-dimethoxytrityl)-5-(prop-2-en-1-one)-2'-deoxyuridine (produced according to Nucleosides & Nucleotides 13, 939-944, (1994)) in 50 ml of tetrahydrofuran. After about 30 min, a white precipitate is formed. It is filtered, the solution is concentrated by evaporation in a vacuum and the residue is spread between dichloromethane and 5% sodium bicarbonate solution. The dichloromethane phase is dried on magnesium sulfate and concentrated by evaporation in a vacuum. The residue is purified by quick chromatography on silica gel, and it is eluted with dichloromethane/hexane/diisopropylethylamine (80:18:2). 1.8 g of the desired compound is obtained as a white foam.

| Elementary analysis: | | | | |
|---|---|---|---|---|
| Cld | C 64.28 | H 6.29 | N 7.14 | P 3.95 |
| Fnd | C 64.02 | H 6.60 | N 7.21 | P 4.09 |

### b) Conjugate of the 36mer oligonucleotide U*T*T*T*T*TCUCAUGGAGCCAAGACGAAUAGCUACAUA-3' and 10-(4-aza-2-hydroxy-5-imino-8-mercapto-octane-1,4,7-tris(carboxymethyl)-1,4,7,10-tetraazacyclododecane

U*: 5-(prop-2-en-1-one)-2'-deoxyuridine

The 30mer-oligonucleotide identified according to the SELEX process is produced with the modification of a 5'-linked sequence 5'-T*T*T*T*T in the usual way in an automatic synthesizer of the Pharmacia company (see Oligonucleotides and Analogues, A Practical Approach, Ed. F. Eckstein, Oxford University Press, Oxford, New York, Tokyo, 1991), and the oligonucleotide is also present on the column of the solid vehicle. By reaction with trichloroacetic acid solution in dichloromethane, the 5'-hydroxy group is opened. The load on the column is about 10 mg of the 35mer-oligonucleotide. The 5'-hydroxy group is reacted in the presence of tetrazole with the phosphoramidite obtained according to example 13a). Then, the phosphite is converted to the phosphotriester by treatment with iodine solution and the terminal DMT radical is cleaved by reaction with trichloroacetic acid solution in dichioromethane. For addition of a thiol group to the α,β-unsaturated carbonyl system present on the terminal 2'-deoxyuridine, it is reacted with a solution of 10-(4-aza-2-hydroxy-5-imino-8-mercaptooctane)-1,4,7-tris(carboxymethyl)-1,4,7,10-tetraazacyclododecane*) in tetrahydrofuran and washed in succession with methanol and water. To remove the modified oligonucleotide from the solid vehicle, the contents of the column are conveyed in a multivial, mixed with 5 ml of 30% ammonia solution, the vessel is sealed and shaken overnight at 55°C. It is then cooled to 0°C, centrifuged, the vehicle is washed with 5 ml of water and the combined aqueous phases are subjected to a freeze-drying.

For purification, the solid material is taken up in 2 ml of water, mixed with 2 ml of 0.5 M ammonium acetate solution and mixed with 10 ml of ethanol, it is allowed to stand overnight at -20°C, centrifuged, the residue is washed with 1 ml of ethanol (-20°C) and finally dried in a vacuum at room temperature.

6 mg of the title compound is obtained as colorless powder.

### *) 10-(4-Aza-2-hydroxy-5-imino-8-mercapto-octane)-1,4,7-tris-(carboxymethyl)-1,4,7,10-tetraazacyclododecane is obtained as described below:

15.7 ml of 1N sodium hydroxide solution and 480 mg (3.49 mmol) of 2-iminotetrahydrothiophenehydrochloride are added to a solution of 1.46 g (3.49 mmol) of 10-(3-amino-2-hydroxy-propyl)-1,4,7-tris-(carboxymethyl)-1,4,7,10-tetraazacyclododecane (see example 1c) in a mixture of 50 ml of water and 50 ml of methanol and stirred for 3 hours at room temperature, concentrated by evaporation in a vacuum to about 1/4 of the initial volume and mixed with stirring with an anion exchanger (IRA 410) until a pH of 11 is reached. The solution is filtered and mixed with stirring in small portions with enough cation exchanger IRC 50 until a pH of 3.5 is reached. After filtering, the solution is freeze-dried. 1.39 g of the desired substance is obtained as white powder with a water content of 4.9%.

| Elementary analysis (relative to the anhydrous substance): | | | | |
|---|---|---|---|---|
| Cld | C 48.45 | H 7.74 | N 16.14 | S 6.16 |
| Fnd | C 48.30 | H 7.98 | N 16.05 | S 6.44 |

### c) Yttrium-90 complex of the conjugate of the 36mer oligonucleotide U*T*T*T*T*TCUCAUGGAGCCAAGACGAAUAGCUACAUA-3' and 10-(4-aza-2-hydroxy-5-imino-8-mercapto-octane)-1,4,7-tris(carboxymethyl)-1,4,7,10-tetraazacyclododecane

U*: 5-(prop-2-en-1-one)-2'-deoxyuridine

A solution of ⁹⁰yttrium, dissolved in 0.05 M ammonium acetate solution (about 380 mCi), is added to 1 mg of the thio derivative of example 13b) in 0.5 ml of 0.05 M ammonium acetate solution of pH 6, adjusted to pH 5.2 with 3 M acetic acid and stirred for 1 hour at room temperature. It is adjusted to pH 7.0 with 0.01 M sodium hydroxide solution and the conjugate is purified by HPLC (TSK-400/MES-buffer). The main fractions are combined, diluted with physiological common salt solution and brought to pH 7.2 with 0.01 M sodium hydroxide solution. After filtration, a preparation suitable for radiotherapy is obtained.

### Example 14

### a) S-(Triphenylmethyl-mercaptoacetyl)-glycyl-glycine methyl ester

3.34 g (10 mmol) of 8-triphenylmethyl mercaptoacetic acid and 1.83 g (10 mmol) of glycylglycine methyl ester hydrochloride are suspended in 250 ml of absolute dichloromethane. After adding 1.01 g (10 mmol) of triethylamine, 2.06 g (10 mmol) of dicyclohexylcarbodiimide, dissolved in 50 ml of absolute dichloromethane, is instilled with ice cooling. It is stirred for 1 hour at 0°C and for 18 hours at room temperature. It is filtered, concentrated by evaporation and chromatographed on silica gel (eluent: CH₂Cl₂/MeOH: 10%-30%).
Yield: 3.56 g (77.0% of theory), white powder

| Elementary analysis: | | | | | |
|---|---|---|---|---|---|
| Cld | C 67.51 | H 5.67 | N 6.06 | O 13.84 | S 11.20 |
| Fnd | C 67.37 | H 6.02 | N 5.91 | | S 6.73 |

### b) [S-(Triphenylmethyl-mercaptoacetyl)-glycyl-glycinamidyl]-6-hexanol

4.63 g (10 mmol) of S-(triphenyl-methyl-mercaptoacetyl)-glycyl-glycine methyl ester, produced under example 14a), is heated to 100°C in 11.72 g (100 mmol) of 6-aminohexanol/50 ml of 1,4-dioxane under argon atmosphere for 2 hours. Then, the reaction batch is poured on a mixture of 100 ml of dichloromethane and 100 ml of water. With stirring and ice cooling, a pH of 6 is adjusted by 10 M hydrochloric acid, the organic phase is separated and dried on sodium sulfate. After evaporation of the solvent, the crude product is purified on silica gel (eluent: CH₂Cl₂/MeOH: 10%-50%).
Yield: 2.97 g (54.2% of theory), white powder

| Elementary analysis: | | | | | |
|---|---|---|---|---|---|
| Cld | C 67.98 | H 6.81 | N 7.67 | O 11.68 | S 5.85 |
| Fnd | C 67.72 | H 6.93 | N 7.93 | | S 5.64 |

### c) O-{[S-(Triphenylmethyl-mercaptoacetyl)-glycyl-glycinamidyl]-6-hex-1-yl}-diisopropylamide-O'-methyl-phosphorous acid diester

5.48 g (10 mmol) of the [S-(triphenylmethyl-mercaptoacetyl)-glycyl-glycinamidyl]-6-hexanol produced under example 14b) is dissolved in 100 ml of absolute dichloromethane. 5.17 g (40 mmol) of diisopropylethylamine is added under argon atmosphere and 3.95 g (20 mmol) of phosphorous acid monomethylester diisopropylamide chloride, dissolved in 50 ml of absolute dichloromethane, is instilled at 0°C. It is stirred for 0.5 hour at 0°C and then for 2 hours at room temperature. For working-up, it is mixed under ice cooling with 320 mg (10 mmol) of absolute methanol, and after concentration, chromatographed on silica gel (eluent: CH₂Cl₂/MeOH: 95% : 5/5% triethylamine).
Yield: 1.98 g (27.9% of theory), colorless oil

### d) 5'-[(Mercaptoacetyl-glycyl-glycyl-amidyl)-6-hex-1-yl]-phosphoric acid ester of the 35mer oligonucleotide 5'-T*T*T*T*TCUCAUGGAGCGCAAGACGAAUAGCUACAUA-3'

A 30mer-oligonucleotide, identified according to the SELEX process, with the modification of a 5'-linked sequence 5'-T*T*T*T*T is produced with the help of an automatic synthesizer of the Pharmacia company (see Oligonucleotides and Analogues, A Practical Approach, Ed. F. Eckstein, Oxford University Press, Oxford, New York, Tokyo, 1991), and the oligonucleotide in protected form is also present on the column of the solid vehicle. The load on the column is about 15 mg of 35mer-oligonucleotide. After cleavage of the 5'-DMT-protective group (trichloroacetic acid/dichloromethane), it is coupled according to the standard methods with the phosphoramidite described under example 14c). After oxidation with iodine in tetrahydrofuran, the conjugate is cleaved off from the vehicle. In this case, the material is mixed with 10 ml of 30% ammonia solution, the vessel is sealed and shaken overnight at 55°C. It is cooled to 0°C, centrifuged, the vehicle is washed with 10 ml of water and the combined aqueous phases are subjected to a freeze-drying.

For purification, the material is taken up in 5 ml of water, mixed with 4 ml of 0.5 M ammonium acetate solution and mixed with 20 ml of ethanol. For completion of the precipitation, it is cooled overnight (-20°C), centrifuged, the residue is washed with 1 ml of ethanol (-20°C) and dried in a vacuum. 9 mg of a white powder is obtained.

For cleavage of the S-trityl protective group, the material is taken up in 5 ml of 50 mmol triethylammonium acetate solution (pH 7.0) and incubated with 500 µl of 0.1 M silver nitrate solution for 30 minutes. Then, 500 µl of 0.14 M dithiothreitol solution is added and incubated for another 30 minutes. After centrifuging, the clear supernatant is desalted on Sephadex G 10. The fractions containing the product are freeze-dried. 4 mg of the conjugate is obtained as white powder.

### e) Technetium-99m complex of the conjugate 5'-[(mercaptoacetyl-glycyl-glycyl-amidyl)-6-hex-1-yl)-phosphoric acid ester of the 35mer oligonucleotide 5'-T*T*T*T*TCUCAUGGAGCGCAAGACGAAUAGCUACAUA-3'

1 mg of conjugate 14d) is dissolved in 1 ml of 0.1 M disodium hydrogen phosphate buffer (pH = 9.5). After adding 10 mg of disodium tartrate, it is mixed with sodium pertechnetate solution (1 mCi) and then with 10 ml of tin(II) chloride solution (5 mg of SnCl₂/1 ml of 0.01 M HCl). The tracer yield (about 93%) is determined by HPLC.

### Example 15

### a) O-{[S-(Triphenylmethyl-mercaptoacetyl)-glycyl-glycinamidyl]-6-hex-1-yl}-toluenesulfonic acid ester

5.48 g (10 mmol) of the [S-(triphenylmethyl-mercaptoacetyl)-glycyl-glycinamidyl]-6-hexanol produced under example 14b) is dissolved in 100 ml of absolute dichloromethane. 1.01 g (10 mmol) of triethylamine and 1.91 g (10 mmol) of p-toluenesulfonic acid chloride are added and stirred for 24 hours at room temperature. Then, it is concentrated and chromatographed on silica gel (eluent: CH₂Cl₂/MeOH: 95:5).
Yield: 4.32 g (61.5% of theory), colorless oil

| Elementary analysis: | | | | | |
|---|---|---|---|---|---|
| Cld | C 65.03 | H 6.18 | N 5.99 | O 13.68 | S 9.14 |
| Fnd | C 64.93 | H 6.32 | N 5.78 | | S 8.87 |

### b) 5'-[(Mercaptoacetyl-glycyl-glycinamidyl)-6-hex-1-yl]-phosphoric acid ester of the 35mer oligonucleotide 5'-T*T*T*T*TCUCAUGGAGCGCAAGACGAAUAGCUACAUA-3'

A 30mer-oligonucleotide, identified according to the SELEX process, with the modification of a 5'-linked sequence 5'-T*T*T*T*T is produced with the help of an automatic synthesizer of the Pharmacia company (see Oligonucleotides and Analogues, A Practical Approach, Ed. F. Eckstein, Oxford University Press, Oxford, New York, Tokyo, 1991), and the oligonucleotide in protected form is also present on the column of the solid vehicle. The load on the column is about 15 mg of 35mer-oligonucleotide. After cleavage of the 5'-DMT-protective group (trichloroacetic acid/dichloromethane), it is coupled according to the standard methods with S-trityl-6-mercapto-hexyl-phosphoramidite. After oxidation with iodine in tetrahydrofuran, the trityl-protected compound is cleaved off from the vehicle, isolated and purified (see example 14d).

The cleavage of the S-trityl protective group, the isolation of the SH-group-carrying oligonucleotide and the purification take place as described under example 14d) (6 mg).

For coupling, the SH-group-carrying 35mer-oligonucleotide (6 mg) in 500 µl of 0.1 M sodium carbonate solution is mixed under argon atmosphere with 100 mg of toluenesulfonic acid ester 15a), dissolved in 500 µl of dimethylformamide. After 30 minutes, it is neutralized and diluted with water to a volume of 5 ml. After the centrifuging, the clear supernatant is freeze-dried.

For cleavage of the S-trityl groups, the procedure is performed as described under 14d). After purification, 4 mg of the conjugate is obtained.

### c) Technetium-99m complex of the conjugate 5'-[(mercaptoacetyl-glycyl-glycinamidyl)-6-hex-1-yl]-phosphoric acid ester of the 35mer oligonucleotide 5'-T*T*T*T*TCUCAUGGAGCGCAAGACGAAUAGCUACAUA-3'

1 mg of the conjugate described under example 15b) is dissolved in 1 ml of 0.1 M disodium hydrogen phosphate buffer (pH = 9.5). After adding 10 mg of disodium tartrate, it is mixed with sodium pertechnetate solution (1 mCi) and then with 10 µl of tin(II) chloride solution (5 mg of SnCl₂/1 ml of 0.01 M HCl). The tracer yield (about 95%) is determined by HPLC.

### Example 16

### a) N-[3-Thia-5-(triphenylmethylmercapto)-1-oxo-pent-1-yl]-S-triphenylmethyl-cysteine methyl ester

2.69 g (10 mmol) of N-[3-thia-5-(triphenylmethylmercapto)-1-oxo-pent-1-yl]-S-triphenylmethyl-cysteine methyl ester (production according to DE 43 10 999) is dissolved together with 5.58 g (20 mmol) of triphenylmethyl chloride in 100 ml of absolute dichloromethane. After adding 2.02 g (20 mmol) of triethylamine, it is allowed to stir overnight under argon atmosphere at room temperature. For working-up, the organic phase is washed three times in each case with 1% citric acid solution, saturated sodium bicarbonate solution and with water. After drying on sodium sulfate, it is concentrated by evaporation and purified on silica gel (eluent: CH₂Cl₂/MeOH: 95:5).
Yield: 4.53 g (60.1% of theory) of colorless oil

| Elementary analysis: | | | | | |
|---|---|---|---|---|---|
| Cld | C 73.27 | H 5.75 | N 1.86 | O 6.37 | S 12.76 |
| Fnd | C 73.31 | H 5.48 | N 1.63 | | S 12.49 |

### b) N-[3-Thia-5-(triphenylmethylmercapto)-1-oxo-pent-1-yl]-S-triphenylmethyl-N'-(6-hydroxy-hex-1-yl)cysteinamide

7.54 g (10 mmol) of the N-[3-thia-5-(triphenylmethylmercapto)-1-oxo-pent-1-yl]-S-cysteine methyl ester described under example 16a) is heated to 100°C in 11.72 g (100 mmol) of 6-aminohexanol/50 ml of 1,4-dioxane under argon atmosphere for 2 hours. Then, the reaction batch is poured on a mixture of 100 ml of dichloromethane and 100 ml of water. With stirring and ice cooling, it is adjusted to a pH of 6 with 10 M hydrochloric acid, the organic phase is separated and dried on sodium sulfate. After evaporation of the solvent, the crude product is purified on silica gel (eluent: CH₂Cl₂/MeOH: 5%-50%)

| Elementary analysis: | | | | | |
|---|---|---|---|---|---|
| Cld | C 72.99 | H 6.49 | N 3.34 | O 5.72 | S 11.46 |
| Fnd | C 72.73 | H 6.62 | N 3.11 | | S 11.17 |

### c) O-{{[N-[3-Thia-5-(triphenylmethylmercapto)-1-oxo-pent-1-yl]-S-triphenylmethyl-cysteinyl}-2-amino-eth-1-yl}-diisopropylamide-O'-methylphosphorous acid diester

8.39 g (10 mmol) of the N-[3-thia-5-(triphenylmethylmercapto)-1-oxo-pent-1-yl]-S-triphenylmethyl-N'-(6-hydroxy-hex-1-yl)cysteinamide, produced under example 16b), is dissolved in 200 ml of absolute dichloromethane. 5.17 g (40 mmol) of diisopropylethylamine is added under argon atmosphere and 3.95 g (20 mmol) of phosphorous acid monomethylester-diisopropylamide-chloride, dissolved in 50 ml of absolute dichloromethane, is instilled at 0°C. It is stirred for 0.5 hour at 0°C and then for 1.5 hours at room temperature. For working-up, it is mixed, with ice cooling, with 320 mg (10 mmol) of absolute methanol and after concentration, chromatographed on silica gel (eluent: CH₂Cl₂/MeOH: 95:5/5% (triethylamine)).
Yield: 5.37 g (53.7% of theory) of yellowish oil

### d) 5'-[N-(3-Thia-5-mercapto-1-oxo-pent-1-yl]-cysteine-N'-(6-hydroxy-hex-1-yl)-amide]-phosphoric acid ester of the 35mer oligonucleotide 5'-CUCAUGGAGCGCAAGACGAAUAGCUACAUAT*T*T*T*T-3'

A 30mer-oligonucleotide, identified according to the SELEX process, is produced with the modification of a sequence T*T*T*T*T-3' placed upstream with the help of an automatic synthesizer of the Pharmacia company (see Oligonucleotides and Analogues, A Practical Approach, Editor F. Eckstein, Oxford University Press, Oxford, New York, Tokyo, 1991), and the oligonucleotide in protected form is also present on the column of the solid vehicle. The load on the column is about 15 mg of 35mer-oligonucleotide. After cleavage of the 5'-DMT-protective group (trichloroacetic acid/dichloromethane), it is coupled according to the standard methods with phosphoramidite (16c) and then oxidized.

The cleavage from the vehicle of the base protective groups and the purification of the bis-S-trityl-protected conjugate take place as described under 14d) (about 12 mg).

For cleavage of the S-trityl protective groups, the material is taken up in 5 ml of 50 mmol triethylammonium acetate solution (pH = 7.0) and incubated with 500 µl of 0.1 M silver nitrate solution for 30 minutes. Then, 500 µl of 0.14 M dithiothreitol solution is added and incubated for another 30 minutes. After centrifuging, the clear supernatant is desalted on Sephadex G 10. The fractions containing the product are freeze-dried. 5 mg of white powder is obtained.

### e) Technetium-99m complex of the conjugate 5'-[N-(3-thia-5-mercapto-1-oxo-pent-1-yl]-cysteine-N'-(6-hydroxy-hex-1-yl)-amide]-phosphoric acid ester of the 35mer oligonucleotide 5'-CUCAUGGAGCGCAAGACGAAUAGCUACAUAT*T*T*T*T-3'

1 mg of the conjugate described under example 16d is dissolved in 1 ml of 0.1 M disodium hydrogen phosphate buffer (pH = 9.5). After adding 10 mg of disodium tartrate, it is mixed with sodium pertechnetate solution (1 mCi) and then with 10 ml of tin(II) chloride solution (5 mg of SnCl₂/1 ml of 0.01 M HCl). The tracer yield (about 96%) is determined by HPLC.

### Example 17

### a) O-{N-[3-Thia-5-(triphenylmethylmercapto)-1-oxo-pent-1-yl]-S-triphenylmethyl-N'-(6-hydroxy-hex-1-yl)cystein-imidyl}-p-toluenesulfonic acid ester

8.39 g (10 mmol) of the N-[3-thia-5-(triphenylmethylmercapto)-1-oxo-pent-1-yl]-S-triphenylmethyl-N'-(6-hydroxy-hex-1-yl)cysteinamide, produced under example 16b, is dissolved in 200 ml of absolute dichloromethane. 1.01 g (10 mmol) of triethylamine, 1.91 g (10 mmol) of p-toluenesulfonic acid chloride are added and stirred for 20 hours at room temperature. Then, it is concentrated and chromatographed on silica gel. (Eluent: CH₂Cl₂/MeOH: 97:3).
Yield: 6.44 g (67.0% of theory) of yellowish oil

| Elementary analysis: | | | | | |
|---|---|---|---|---|---|
| Cld | C 72.47 | H 6.29 | N 2.91 | O 8.32 | S 10.01 |
| Fnd | C 72.19 | H 6.47 | N 2.68 | | S 9.83 |

### b) 5'-[(Mercaptoacetyl-glycyl-glycinamidyl)-13-tridec-7-thio-1-ylj-phosphoric acid ester of the 35mer oligonucleotide 5'-CUCAUGGAGCGCAAGACGAAUAGCUACAUAT*T*T*T*T-3'

A 30mer-oligonucleotide, identified according to the SELEX process, is produced with the modification of a sequence T*T*T*T*T-3' placed upstream with the help of an automatic synthesizer of the Pharmacia company (see Oligonucleotides and Analogues, A Practical Approach, Editor F. Eckstein, Oxford University Press, Oxford, New York, Tokyo, 1991), and the oligonucleotide in protected form is also present on the column of the solid vehicle. The load on the column is about 15 mg of 35mer-oligonucleotide. After cleavage of the 5'-DMT-protective group (trichloroacetic acid/dichloromethane), it is coupled according to the standard methods with S-trityl-6-mercaptohexyl-phosphoramidite.

After oxidation with iodine in tetrahydrofuran, the trityl-protected compound is cleaved off from the vehicle, isolated and purified (see example 14d).

The cleavage of the S-trityl protective group, the isolation of the SH-group-carrying oligonucleotide and the purification take place as described under example 14d) (7.5 mg).

For coupling, the SH-group-carrying 30mer-oligonucleotide (7 mg) in 550 µl of 0.1 M sodium carbonate solution is mixed under argon atmosphere with 180 mg of toluenesulfonic acid ester 17a), dissolved in 500 µl of dimethylformamide. After 30 minutes, it is neutralized and diluted with water to a volume of 5 ml. After the centrifuging, the clear supernatant is freeze-dried. For cleavage of the S-trityl groups, the procedure is performed as described under 14d). After purification, 4.3 mg of conjugate is obtained.

### c) Technetium-99m complex of the conjugate 5'-[(mercaptoacetyl-glycyl-glycinamidyl)-13-trideca-7-thio-1-yl]-phosphoric acid ester of the 35mer oligonucleotide 5'-CUCAUGGAGCGCAAGACGAAUAGCUACAUAT*T*T*T*T-3'

1 mg of the conjugate described under example 17b) is dissolved in 1 ml of 0.1 M disodium hydrogen phosphate buffer (pH = 9.5). After adding 10 mg of disodium tartrate, it is mixed with sodium pertechnetate solution (1 mCi) and then with 10 µl of tin(II) chloride solution (5 mg of SnCl₂/1 ml of 0.01 M HCl). The tracer yield (about 93%) is determined by HPLC.

### Example 18

### a) Conjugate of 5'-(6-amino-1-hexyl-phosphoric acid ester) of the 35mer oligonucleotide 5'-CUCAUGGAGCGCAAGACGAAUAGCUACAUAT*T*T*T*T-3' and N-(tetrahydro-2-oxo-thiophen-3-yl)-thiodiglycolic acid monoamide

A 30mer-oligonucleotide, identified according to the SELEX process, is produced with the modification of a sequence T*T*T*T*T-3' placed upstream with the help of an automatic synthesizer of the Pharmacia company (see Oligonucleotides and Analogues, A Practical Approach, Editor F. Eckstein, Oxford University Press, Oxford, New York, Tokyo, 1991), and the oligonucleotide in protected form is also present on the column of the solid vehicle. The load on the column is about 15 mg of 35mer-oligonucleotide. After cleavage of the 5'-DMT-protective group (trichloroacetic acid/dichloromethane), it is coupled according to the standard methods with N-trifluoroacetylaminohexylphosphoramidite.

After oxidation with iodine in tetrahydrofuran, the amino group-carrying oligonucleotide is cleaved off from the vehicle and isolated and purified as described under 1b) (9.3 mg).

For coupling with the N-(tetrahydro-2-oxo-thiophen-3-yl)-thiodiglycolic acid monoamide (DE 43 11 023), 5 mg of the amino group-carrying oligonucleotide is dissolved in 1 ml of 2 M sodium carbonate solution. After adding 100 mg of the thiolactone derivative, it is incubated for 4 hours at room temperature. Then, it is neutralized and the desalting is achieved by ultrafiltration through a membrane with an exclusion limit of 3,000 (Amicon YM3). After lyophilization, it is subjected to a freeze-drying. 6.3 mg of the desired conjugate is obtained.

### b) Technetium-99m complex of the conjugate 5'-(6-amino-1-hexyl-phosphoric acid ester) of the 35mer oligonucleotide 5'-CUCAUGGAGCGCAAGACGAAUAGCUACAUAT*T*T*T*T-3' and N-(tetrahydro-2-oxo-thiophen-3-yl)-thiodiglycolic acid monoamide

1 mg of the SH-group-carrying conjugate described under example 18a) is dissolved in 1 ml of 0.1 M disodium hydrogen phosphate buffer (pH = 9.5). After adding 10 mg of disodium tartrate, it is mixed with sodium pertechnetate solution (1 mCi) and then with 10 µl of tin(II) chloride solution (5 mg of SnCl₂/1 ml of 0.01 M HCl). The tracer yield (94%) is determined by HPLC.

### Example 19

### a) 5'-(6-Amino-1-hexyl-phosphoric acid ester) of the 32mer-oligonucleotide 5'-CUCAUGGAGCGCAAGACGAAUAGCUACAUAT-^{3'-3'}T-5'

The 30mer-oligonucleotide 5'-CUCAUGGAGCGCAAGACGAAUAGCUACAUA-3', identified according to the SELEX process, with the modification of a thymidine sequence -T^{3'-3'}T-5', placed upstream, which is bound by 5'-position on the vehicle, is produced in the usual way in an automatic synthesizer of the Pharmacia company (see Oligonucleotides and Analogues, A Practical Approach, Editor F. Eckstein, Oxford University Press, Oxford, New York, Tokyo, 1991), and the oligonucleotide is also present on the column of the solid vehicle. By reaction with trichloroacetic acid solution in dichloromethane, the 5'-hydroxy group is opened. The load on the column is about 10 mg of the 32mer-oligonucleotide. To join the linker, the column is reacted with an acetonitrile solution of 50 µmol of β-cyanoethyl-N,N-diisopropylamino-6-(trifluoroacetamido)-1-hexylphosphoramidite (produced according to Nucl. Acids. Res. 16,2659-2669 (1988)) in the presence of tetrazole. The oxidation of the formed phosphite to the completely protected phosphotriester takes place with iodine in tetrahydrofuran. Then, the column is washed in succession with methanol and water. To remove the modified oligonucleotide from the solid vehicle, the contents of the column are conveyed in a multivial, mixed with 5 ml of 30% ammonia solution, the vessel is sealed and shaken overnight at 55°C. It is then cooled to 0°C, centrifuged, the vehicle is washed with 5 ml of water and the combined aqueous phases are subjected to a freeze-drying.

For purification, the solid material is taken up in 2 ml of water, mixed with 2 ml of 0.5 M ammonium acetate solution and mixed with 10 ml of ethanol, it is allowed to stand overnight at -20°C, centrifuged, the residue is washed with 1 ml of ethanol (-20°C) and finally dried in a vacuum at room temperature.

8 mg of the title compound is obtained as colorless powder.

### b) Conjugate of 5'-(6-amino-1-hexyl-phosphoric acid ester) of the 32mer oligonucleotide 5'-CUCAUGGAGCGCAAGACGAAUAGCUACAUAT-^{3'-3'}T-5' and 10-[7-(4-isothiocyanatophenyl)-2-hydroxy-5-oxo-7-(carboxymethyl)-4-azaheptyl]-1,4,7-tris-(carboxymethyl)-1,4,7,10-tetraazacyclododecane

8 mg of the oligonucleotide obtained in example 19a) is dissolved in 2.5 ml of a NaHCO₃/Na₂CO₃ buffer (pH 8.0) and mixed with 1 mg of 10-[7-(4-isothiocyanatophenyl)-2-hydroxy-5-oxo-7-(carboxymethyl)-4-aza-heptyl]-1,4,7-tris-(carboxymethyl)-1,4,7,10-tetraazacyclododecane (title compound of example 1f). It is stirred for 5 hours at room temperature, the pH is adjusted to 7.2 by adding 0.01 M hydrochloric acid and the solution is subjected to an ultrafiltration through a membrane with the exclusion limit 3,000 (Amicon YM3) and then a freeze-drying. 7 mg of the desired conjugate is obtained.

### c) ¹¹¹Indium complex of the conjugate of 5'-(6-amino-1-hexyl-phosphoric acid ester) of the 32mer oligonucleotide 5'-CUCAUGGAGCGCAAGACGAAUAGCUACAUAT-^{3'-} ^{3'}T-5' and 10-[7-(4-isothiocyanatophenyl)-2-hydroxy-5-oxo-7-(carboxymethyl)-4-aza-heptyl]-1,4,7-tris-(carboxymethyl)-1,4,7,10-tetraazacyclododecane

15 µl of an ¹¹¹indium(III) acetate solution (350 µCi), (produced from ¹¹¹indium(III) chloride in 2 M sodium acetate solution and adjustment of the pH to 4.0 with 0.1 M hydrochloric acid) is added to 135 µl of a solution of 1 mg of the title compound of example 19b) in MES buffer, pH 6.2 (MES = 2-(N-morpholino)ethylsulfonic acid). The pH is brought to 4.2 by adding 0.01 M hydrochloric acid. It is stirred for 1 hour at 37°C at pH 4.2. It is brought to pH 6 with 2 M sodium acetate solution and 10 µl of a 0.1 M Na₂EDTA solution (Na₂EDTA = ethylenediaminetetraacetic acid disodium salt) is added to complex excess ¹¹¹indium. The final purification of thus obtained labeled conjugate (1h) takes place by HPLC (exclusion chromatography: TSK-400/MES-buffer). The fractions containing the labeled conjugate are diluted with physiological common salt solution, adjusted to pH 7.2 with 0.01 M sodium hydroxide solution and filtered. A thus produced solution then represents a suitable preparation for radiodiagnosis.

### Example 20

### a) 5'-(6-Amino-1-hexyl-phosphoric acid ester) of the 35mer oligonucleotide 5'-T***T***T***T***TAGGAGGAGGAGGGAGAGCGCAAAUGAOAUU-3'

The 30mer-oligonucleotide 5'-TAGGAGGAGGAGGGAGAGCGCAAAUGAGAUU-3' (seq. no. 13 of US Patent No. 5,270,163), identified according to the SELEX process, is produced in the usual way in an automatic synthesizer of the Pharmacia company (see Oligonucleotides and Analogues, A Practical Approach, Ed. F. Eckstein, Oxford University Press, Oxford, New York, Tokyo, 1991), and the oligonucleotide is also present on the column of the solid vehicle. The four final thymidines are bound by phosphorodithioates to the thymidine present on the 5'-end according to the technique described by G. Blaton et al. in "Oligonucleotides and Analogues," pp. 109-135. For this purpose, first the 5'-hydroxy group is opened by treatment with trichloroacetic acid. Then, the 3'-hydroxy group of a 5'-DMTO-thyridine is converted with tris-pyrrolidinophosphine and tetrazole in the diamidite, which is converted to the phosphorothioamidite by adding 2,4-dichlorobenzyl mercaptan. This compound is activated with tetrazole and reacted with the 5'-hydroxy group of the oligonucleotide to thiophosphatetriester. The oxidation to phosphorodithioate takes place with elementary sulfur in a solution of carbon disulfide, pyridine, triethylamine 95:95:10. The benzyl groups are not yet removed. In an analogous way, 3 additional thymidines are also bound. The load on the column is about 10 mg of the 35mer-oligonucleotide. Then, a 5'-hydroxy group is again opened.

To join the linker, the column is reacted with an acetonitrile solution of 50 µmol of 2-cyanoethyl-N,N'-diisopropylamino-6-(trifluoroacetamido)-1-hexyl phosphoramidite (lit. in example in the presence of tetrazole. The oxidation of the formed phosphite to the phosphotriester takes place with iodine in tetrahydrofuran. Then, the column is washed in succession with methanol and water. Then, the protective groups of the dithionate bonds are removed by a solution of thiophenol/triethylamine/dioxane 1:2:2, which takes place in 2 hours. Thereupon, the column is washed in each case with 3 times its volume with methanol, then washed with ether and dried. To remove the modified oligonucleotide from the solid vehicle, the contents of the column are conveyed in a multivial, mixed with 5 ml of 30% ammonia solution, the vessel is sealed and shaken overnight at 55°C. It is then cooled to 0°C, centrifuged, the vehicle is washed with 5 ml of water and the combined aqueous phases are subjected to a freeze-drying.

For purification, the solid material is taken up in 2 ml of water, mixed with 2 ml of 0.5 M ammonium acetate solution and mixed with 10 ml of ethanol, it is allowed to stand overnight at -20°C, centrifuged, the residue is washed with 1 ml of ethanol (-20°C) and finally dried in a vacuum at room temperature.

8 mg of the title compound is obtained as colorless powder.

### b) Conjugate of 5'-(6-amino-1-hexyl-phosphoric acid ester) of the 35mer oligonucleotide 5'-T***T***T***T***TAGGAGGAGGAGGGAGAGCGCAAAUGAGAUU-3' and 10-[7-(4-isothiocyanato-phenyl)-2-hydroxy-5-oxo-7-(carboxymethyl)-4-aza-heptyl]-1,4,7-tris(carboxymethyl)-1,4,7,10-tetraazacyclododecane

8 mg of the oligonucleotide obtained in example 20a) is dissolved in 2.5 ml of a NaHCO₃/Na₂CO₃ buffer (pH 8.0) and mixed with 1 mg of 10-[7-(4-isothiocyanatophenyl)-2-hydroxy-5-oxo-7-(carboxymethyl)-4-aza-heptyl]-1,4,7-tris-(carboxymethyl)-1,4,7,10-tetraazacyclododecane (title compound of example 1f). It is stirred for 5 hours at room temperature, the pH is adjusted to 7.2 by adding 0.01 M hydrochloric acid and the solution is subjected to an ultrafiltration through a membrane with the exclusion limit 3,000 (Amicon YM3) and then a freeze-drying.

7 mg of the desired conjugate is obtained.

### c) ¹¹¹Indium complex of the conjugate 5'-(6-amino-1-hexyl-phosphoric acid ester) of the 35mer oligonucleotide 5'-T***T***T***T***TAGGAGGAGGAGGGAGAGCGCAAAUGAGAUU-3' and 10-[7-(4-isothiocyanatophenyl)-2-hydroxy-5-oxo-7-(carboxymethyl)-4-aza-heptyl]-1,4,7-tris(carboxymethyl)-1,4,7,10-tetraazacyclododecane

15 µl of an ¹¹¹indium(III) acetate solution (350 µCi), (produced from ¹¹¹indium(III) chloride in 2 M sodium acetate solution and adjustment of the pH to 4.0 with 0.1 M hydrochloric acid) is added to 135 µl of a solution of 1 mg of the title compound of example 20b) in MES buffer, pH 6.2 (MES = 2-(N-morpholino)ethylsulfonic acid). The pH is brought to 4.2 by adding 0.01 M hydrochloric acid. It is stirred for 1 hour at 37°C at pH 4.2. It is brought to pH 6 with 2 M sodium acetate solution and 10 µl of a 0.1 M Na₂EDTA = ethylenediamine-tetraacetic acid disodium salt is added to complex excess ¹¹¹indium. The final purification of thus obtained conjugate (1h) takes place by HPLC (exclusion chromatography: TSK-400/MES-buffer). The fractions containing the labeled conjugate are diluted with physiological common salt solution, adjusted to pH 7.2 with 0.01 M sodium hydroxide solution and filtered. A thus produced solution then represents a suitable preparation for radiodiagnosis.

### Example 21

### a) 5'-(6-Amino-1-hexyl-phosphoric acid ester) of the 35mer oligonucleotide 5'-CUCAUGGAGCGCAAGACGAAUAGCUACAUAT**T**T**T**T-3'

The 30mer-oligonucleotide 5'-CUCAUGGAGCGCAAGACGAAUAGCUACAUA-3', identified according to the SELEX process, with the modification of a sequence T**T**T**T**T-3' placed upstream, is obtained by first a sequence of 5 thymidines connected by cyanoethylphosphate groups being produced on the vehicle by 3'. By reaction of this compound with a 0.5 M solution of tetraethylthiuram disulfide in acetonitrile, the sulfonation to phosphonothioate takes place within 15 minutes, which then with free 5'-hydroxyl group is starting material for the 35mer oligonucleotide. The entire synthesis takes place in the usual way in an automatic synthesizer of the Pharmacia company (see Oligonucleotides and Analogues, A Practical Approach, Editor F. Eckstein, Oxford University Press, Oxford, New York, Tokyo, 1991), and the oligonucleotide is still present on the column of the solid vehicle. By reaction with trichloroacetic acid solution in dichloromethane, the 5'-hydroxy group is opened. The load on the column is about 10 mg of the 35mer-oligonucleotide. To join the linker, the column is reacted with an acetonitrile solution of 50 µmol of β-cyanoethyl-N,N-diisopropylamino-6-(trifluoroacetamido)-1-hexylphosphoramidite (produced according to Nucl. Acids. Res. 16, 2659-2669 (1988)) in the presence of tetrazole. The oxidation of the phosphite formed in such a way to the completely protected phosphotriester takes place with iodine in tetrahydrofuran. Then, the column is washed in succession with methanol and water. To remove the modified oligonucleotide from the solid vehicle and to cleave the cyanoethyl groups, the contents of the column are conveyed in a multivial, mixed with 5 ml of 30% ammonia solution, the vessel is sealed and shaken overnight at 55°C. It is then cooled to 0°C, centrifuged, the vehicle is washed with 5 ml of water and the combined aqueous phases are subjected to a freeze-drying.

For purification, the solid material is taken up in 2 ml of water, mixed with 2 ml of 0.5 M ammonium acetate solution and mixed with 10 ml of ethanol, it is allowed to stand overnight at -20°C, centrifuged, the residue is washed with 1 ml of ethanol (-20°C) and finally dried in a vacuum at room temperature.

8 mg of the title compound is obtained as colorless powder.

### b) Conjugate of 5'-(6-amino-1-hexyl-phosphoric acid ester) of the 35mer oligonucleotide 5'-CUCAUGGAGCGCAAGACGAAUAGCUACAUAT**T**T**T**T-3' and 10-[7-(4-isothiocyanatophenyl)-2-hydcoxy-5-oxo-7-(carboxymethyl)-4-aza-heptyl]-1,4,7-tris(carboxymethyl)-1,4,7,10-tetraazacyclododecane

8 mg of the oligonucleotide obtained in example 20a) is dissolved in 2.5 ml of a NaHCO₃/Na₂CO₃ buffer (pH 8.0) and mixed with 1 mg of 10-[7-(4-isothiocyanatophenyl)-2-hydroxy-5-oxo-7-(carboxymethyl)-4-aza-heptyl]-1,4,7-tris-(carboxymethyl)-1,4,7,10-tetraazacyclododecane (title compound of example 1f). It is stirred for 5 hours at room temperature, the pH is adjusted to 7.2 by adding 0.01 M hydrochloric acid and the solution is subjected to an ultrafiltration through a membrane with the exclusion limit 3,000 (Amicon YM3) and then a freeze-drying.

7 mg of the desired conjugate is obtained.

### c) ¹¹¹Indium complex of the conjugate 5'-(6-amino-1-hexyl-phosphoric acid ester) of the 35mer oligonucleotide 5'-CUCAUGGAGCGCAAGACGAAUAGCUACAUAT**T**T**T**T-3' and 10-[7-(4-isothiocyanatophenyl)-2-hydroxy-5-oxo-7-(carboxymethyl)-4-aza-heptyl]-1,4,7-tris(carboxymethyl)-1,4,7,10-tetraazacyclododecane

15 µl of an ¹¹¹indium(III) acetate solution (350 µCi), (produced from ¹¹¹indium(III) chloride in 2 M sodium acetate solution and adjustment of the pH to 4.0 with 0.1 M hydrochloric acid) is added to 135 µl of a solution of 1 mg of the title compound of example 21b) in MES buffer, pH 6.2 (MES = 2-(N-morpholino)ethylsulfonic acid). The pH is brought to 4.2 by adding 0.01 M hydrochloric acid. It is stirred for 1 hour at 37°C at pH 4.2. It is brought to pH 6 with 2 M sodium acetate solution and 10 µl of a 0.1 M Na₂EDTA = ethylenediamine-tetraacetic acid disodium salt is added to complex excess ¹¹¹indium. The final purification of thus obtained conjugate (1h) takes place by HPLC (exclusion chromatography: TSK-400/MES-buffer). The fractions containing the labeled conjugate are diluted with physiological common salt solution, adjusted to pH 7.2 with 0.01 M sodium hydroxide solution and filtered. A thus produced solution then represents a suitable preparation for radiodiagnosis.

### Example 22

### a) N-(5-Mercapto-3-thia-1-oxo-pent-1-yl)-glycine methyl ester

12.56 g (0.1 mol) of glycine methyl ester hydrochloride, 13.42 g (0.1 mol) of 2,5-dithia-cyclohexanone and 10.12 g (0.1 mol) of triethylamine are dissolved under argon atmosphere in 500 ml of absolute dichloromethane. It is stirred for 24 hours at room temperature and the batch is then poured on 250 ml of 5% aqueous citric acid. It is well-stirred, the organic phase is separated and dried on sodium sulfate. After evaporation of the solvent in a vacuum, the oily residue is chromatographed on silica gel (mobile solvent: dichloromethane/methanol, methanol 0-10%)
Yield: 18.9 g (84.6%), colorless oil

| Elementary analysis: | | | | | |
|---|---|---|---|---|---|
| Cld | C 37.65 | H 5.87 | N 6.27 | O 21.49 | S 28.71 |
| Fnd | C 37.43 | H 6.02 | N 6.12 | | S 28.48 |

### b) N-[5-(Triphenylmethylmercapto)-3-thia-1-oxo-pent-1-yl]-glycine methyl ester

11.17 g (50 mmol) of N-(5-mercapto-3-thia-1-oxo-pent-1-yl)-glycine methyl ester (example 22a), 13.94 g (50 mmol) of triphenylmethyl chloride and 5.06 g (50 mmol) of triethylamine are dissolved under argon atmosphere in 500 ml of absolute dichloromethane. It is stirred for 16 hours at room temperature and the batch is then poured on 150 ml of 5% aqueous citric acid. It is well-stirred, the organic phase is separated and dried on sodium sulfate. After evaporation of the solvent in a vacuum, the oily residue is chromatographed on silica gel (mobile solvent: dichloromethane/methanol, 95:5).
Yield: 15.7 g (67.4%), colorless oil

| Elementary analysis: | | | | | |
|---|---|---|---|---|---|
| Cld | C 67.07 | H 5.85 | N 3.01 | O 10.31 | S 13.77 |
| Fnd | C 67.01 | H 6.11 | N 2.93 | | S 13.49 |

### c) N-[5-(Triphenylmethylmercapto)-3-thia-1-oxo-pent-1-yl]-glycine-N'-(6-hydroxyhexyl)-amide

11.64 g (25 mmol) of N-[5-triphenylmethylmercapto)-3-thia-1-oxo-pent-1-yl]-glycine methyl ester (example 22b) and 29.3 g (250 mmol) of 6-aminohexanol are melted together under argon atmosphere for 16 hours at 100°C. After cooling down the reaction batch, it is taken up in 500 ml of dichloromethane and poured on 250 ml of 5% aqueous citric acid. Under ice cooling and stirring, it is adjusted to a pH of 6.5 with concentrated hydrochloric acid. The organic phase is separated and dried on sodium sulfate. After evaporation of the solvent in a vacuum, the oily residue is chromatographed on silica gel (mobile solvent: dichloromethane/methanol, 0-10% methanol).
Yield: 7.7 g (56.0%), colorless oil

| Elementary analysis: | | | | | |
|---|---|---|---|---|---|
| Cld | C 67.60 | H 6.96 | N 5.09 | O 8.72 | S 11.64 |
| Fnd | C 67.48 | H 7.03 | N 4.92 | | S 11.43 |

### d) N-Diisopropyl-O-cyanoethyl-0'-[7,10-diaza-8,11-dioxo-13-thia-15-(triphenylmethylmercapto)-pentadec-1-yl]-phosphorous acid amide

5.51 g (10 mmol) of N-[5-(triphenylmethylmercapto)-3-thia-1-oxo-pent-1-yl]-glycine-N'-(6-hydroxyhexyl)-amide (example 22c) is dissolved in 50 ml of absolute dichloromethane and 50 ml of absolute pyridine. 4.73 g (20 mmol) of diisopropylamino-O-cyanoethyl-phosphorous acid chloride, dissolved in 50 ml of absolute dichloromethane, is instilled in the batch at room temperature. After 4 hours, it is poured on 250 ml of saturated, aqueous sodium bicarbonate solution, stirred and the organic phase is dried on magnesium sulfate. After evaporation of the solvent, the oily residue is chromatographed on silica gel (dichloromethane/methanol/triethylamine 98:1:1).
Yield: 4.32 g (57.5%), colorless foamy oil

| Elementary analysis: | | | | | | |
|---|---|---|---|---|---|---|
| Cld: | C 63.97 | H 7.38 | N 7.46 | O 8.52 | P 4.12 | S 8.54 |
| Fnd: | C 63.81 | H 7.41 | N 7.22 | | P 3.97 | S 8.31 |

### e) 5'-[N-(3'-Aza-8'-mercapto-1',4'-dioxo-6'-thia-oct-1'-yl)-6-aminohexyl-phosphoric acid ester] of 5'-CUCAUGGAGCGCAAGACGAAUAGCUACAUAT*T*T*T*T*-3'

A 30mer-oligonucleotide 5'-CUCAUGGAGCGCAAGACGAAUAGCUACAUA-3', identified according to the SELEX process, with the modification of a sequence T*T*T*T*T*-3' placed upstream, is produced with the help of an automatic synthesizer of the Pharmacia company (see Oligonucleotides and Analogues, A Practical Approach, Ed. F. Eckstein, Oxford University Press, Oxford, New York, Tokyo, 1931), and the oligonucleotide in protected form is still present on the column of the solid vehicle. The load on the column is about 15 mg of 35mer-oligonucleotide. After cleavage of the 5'-DMT-protective group, it is coupled according to the standard methods with the phosphoramidite described under example 22d. After oxidation with iodine in tetrahydrofuran, the conjugate is cleaved off from the vehicle. For this purpose, the material is mixed with 10 ml of 30% ammonia solution and shaken overnight at 55°C. It is cooled to 0°C, centrifuged, the vehicle is washed with 10 ml of water and the combined aqueous phases are subjected to a freeze-drying. For purification, the material is taken up in 5 ml of water, 4 ml of 0.5 mol ammonium acetate is added and mixed with 20 ml of ethanol. For completion of the precipitation, it is cooled overnight (-20°C), centrifuged, the residue is washed with 1 ml of ethanol (-20°C) and dried in a vacuum. 9.5 mg of white powder is obtained. For cleavage of the S-trityl protective group, the material is taken up in 5 ml of 50 mmol triethylammonium acetate solution (pH=7) and incubated with 500 µl of 0.1 M silver nitrate solution for 30 minutes. Then, 500 µl of 0.14 M dithiothreitol solution is added and incubated for another 30 minutes. After centrifuging, the clear supernatant is desalted on Sephadex G 10. The fractions containing the product are freeze-dried. 3.5 mg of white powder is obtained.

### f) Tc-99m Complex of 5'-[N-(3'-aza-8'-mercapto-1',4'-dioxo-6'-thia-oct-1'-yl)-6-aminohexyl-phosphoric acid ester] of 5'-CUCAUGGAGCGCAAGACGAAUAG CUACAUAT*T*T*T*T*-3'

1 mg of the conjugate described under example 22e) is dissolved in 1 ml of 0.1 M disodium hydrogen phosphate buffer (pH = 8.5). After adding 10 mg of disodium tartrate, it is mixed with sodium pertechnetate solution (1 mCi) and then with 10 µl of tin(II) chloride solution (5 mg/l ml of 0.01 M HCl). The tracer yield (about 95%) is determined by HPLC.

### Example 23

### a) 5'-[N-(6'-Aza-7'-hydroxycarboxy-9'-mercapto-1',5'-dioxo-3'thia-non-1'-yl)-6-aminohexylphosphoric acid ester] of 5'-CUCAUGGAGCGCAAGACGAAUAGCUACAUAT*T*T*T*T*-3'

First, a solution of the N-hydroxysuccinimide ester of N-(2-oxo-tetrahydrothiophen-3-yl)-thiodiglycolic acid monoamide (DE 43 11 023) in 500 µl of absolute DMF is produced. For this purpose, 24.9 mg (0.1 mmol) of N-(2-oxo-tetra-hydrothyophen-3-yl)-thiodiglycolic acid monoamide and 11.5 mg (0.1 mmol) of N-hydroxysuccinimide are dissolved in 500 µl of absolute DMF. At 0°C, 19.2 mg (0.1 mmol) of EDC is added to the batch. It is stirred for 30 minutes at 0°C.

10 mg of the 5'-(6-amino-hexyl-phosphoric acid ester) of 5'-CUCAUGGAGCGCAAGACGAAUAGCUACAUAT*T*T*T*T*-3' produced under example 1 is dissolved in 1 ml of a sodium bicarbonate/sodium carbonate buffer (pH = 8.0). The DMF solution of the NHS ester prepared in advance is added and incubated for 16 hours at room temperature under argon atmosphere. Then, it is centrifuged, concentrated to a volume of 500 µl and chromatographed on Sephadex G-25. After freeze-drying, 2 mg of the conjugate is obtained as white powder.

### b) Tc-99m Complex of 5'-[N-(6'-aza-7'-hydroxy-9'-mercapto-1',5'-dioxo-3'-thia-non-1'-yl)-6-aminohexyl-phosphoric acid ester] of 5'-CUCAUGGAGCGCAAGACGAAUAGCUACAUAT*T*T*T*T*-3'

1 mg of the conjugate described under example 23a) is dissolved in 1 ml of 0.1 M disodium hydrogen phosphate buffer. After adding 10 mg of disodium tartrate, it is mixed with sodium pertechnetate solution (1 mCi) and then with 10 µl of tin(II) chloride solution (5 mg/l ml of 0.01 M HCl). The tracer yield is determined by HPLC (about 92%).

### Example 24

### a) 5'-[N-(Mereaptoacetyl)-6-aminohexylphosphoric acid ester] of 5'-CUCAUGGAGCGCAAGACGAAUAGCUACAUAT*T*T*T*T*-3'

10 mg of the 5'-(6-amino-hexyl-phosphoric acid ester) of 5'-CUCAUGGAGCGCAAGACGAAUAGCUACAUAT*T*T*T*T-3' produced under example 1 is dissolved in 1 ml of a sodium bicarbonate/sodium carbonate buffer (pH = 8.0). Then, 23.1 mg (0.1 mmol) of S-acetylmercaptoacetic acid-NHS ester, dissolved in 500 µl of absolute DMF, is added to the batch and incubated for 17 hours at room temperature under argon atmosphere. Then, it is centrifuged, concentrated to a volume of 500 µl and chromatographed on Sephadex G-25. After freeze-drying, 3 mg of the conjugate is obtained as white powder.

### b) Tc-99m Complex of 5'-[N-(mercaptoacetyl)-6-aminohexyl-phosphoric acid ester] of 5'-CUCAUGGAGCGCAAGACGAAUAGCUACAUAT*T*T*T*T*-3'

1 mg of the conjugate described under example 24a) is dissolved in 1 ml of 0.1 M disodium hydrogen phosphate buffer (pH = 8.5). After adding 10 mg of disodium tartrate, it is mixed with sodium pertechnetate solution (1 mCi) and then with 10 µl of tin(II) chloride solution (5 mg/l ml of 0.01 M HCl). The tracer yield (about 97%) is determined by HPLC.

### Example 25

### a) N-[2-(Triphenylmethylmercapto)-eth-1-yl]-thiodiglycolic acid monoamide

31.9 g (0.1 mol) of 2-(triphenylmethylmercapto)-ethylamine and 10.1 g (0.1 mol) of triethylamine are introduced in 500 ml of absolute dichloromethane. At 0°C, a solution of 13.2 g (0.1 mol) of thiodiglycolic acid anhydride is instilled, stirred for 1 hour at 0°C and for 16 hours at room temperature. Then, it is poured on 250 ml of 5% aqueous citric acid, well-stirred, the organic phase is separated and dried on sodium sulfate. After evaporation of the solvent in a vacuum, the residue is chromatographed on silica gel (mobile solvent: dichloromethane/methanol, 0-20% methanol).
Yield: 20.32 g (45.0%), colorless oil

| Elementary analysis: | | | | | |
|---|---|---|---|---|---|
| Cld | C 66.49 | H 5.58 | N 3.10 | O 10.63 | S 14.20 |
| Fnd | C 66.21 | H 5.73 | N 2.98 | | S 14.02 |

### b) 5'-[N-(1',5'-Dioxo-6'-aza-3'-thia-8'-mercapto-oct-1-yl)-aminohexylphosphoric acid ester] of 5'-CUCAUGGAGCGCAAGACGAAUAGCUACAUAT*T*T*T*T*-3'

First, the NHS-ester of N-[2-(triphenylmethylmercapto)-eth-1-yl]-thiodiglycolic acid monoamide (example 25a) is produced. For this purpose, 45.2 mg (0.1 mmol) of the previously mentioned acid is dissolved in 500 µl of absolute DMF (0.1 mmol) and mixed with 11.5 mg (0.1 mmol) of NHS. After cooling to 0°C, 19.2 mg (0.1 mmol) of EDC is added and incubated for 30 minutes at 0°C.

A solution of 10 mg of the 5-(6-aminohexyl-phosphoric acid ester) of 5'-CUCAUGGAGCGCAAGACGAAUAGCUACAUAT*T*T*T*T*-3' in 1 ml of a sodium bicarbonate/sodium carbonate buffer (pH = 8.0), described under example 1, is mixed with 500 µl of the NHS-ester solution produced in advance. It is incubated for 16 hours at room temperature. Then, it is concentrated by evaporation to 500 µl and chromatographed on Sephadex G-25. 6 mg of the S-trityl-protected compound is obtained. The cleavage of the S-trityl protective group, the isolation of the oligonucleotide carrying SH groups and the purification take place as described under example 22e. 4 mg of white lyophilizate is obtained.

### c) Tc-99m Complex of 5'-[N-(1',5'-dioxo-6'-aza-3'-thia-8'-mercapto-oct-1-yl)-aminohexylphosphoric acid ester] of 5'-CUCAUGGAGCGCAAGACGAAUAGCUACAUAT*T*T*T*T*-3'

1 mg of the conjugate produced under example 25b is dissolved in 1 ml of 0.1 M disodium hydrogen phosphate buffer (pH = 8.5). After adding 10 mg of disodium tartrate, it is mixed with sodium pertechnetate solution (1 mCi) and then with 10 µl of tin(II) chloride solution (5 mg/l ml of 0.01 M HCl). The tracer yield is determined by HPLC (91%).

### Example 26

### a) N,N'-Bis-[2-(triphenylmethylmercapto)-1-oxo-eth-1-yl]-3,4-diaminobenzoic acid methyl ester

3.32 g (20 mmol) of 3,4-diaminobenzoic acid methyl ester and 13.38 g (40 mmol) of S-triphenylmethyl-mercaptoacetic acid are dissolved in 200 ml of absolute dichloromethane. At 0°C, 8.25 g (40 mmol) of dicyclohexylcarbodiimide, dissolved in 100 ml of absolute dichloromethane, is instilled in the batch. It is stirred for 1 more hour at 0°C and finally for 16 more hours at room temperature. It is filtered, shaken against 1% aqueous citric acid, the organic phase is dried on sodium sulfate and the solvent is evaporated in a vacuum. The residue is chromatographed on silica gel (mobile solvent: dichloromethane/methanol, 0-10% methanol).
Yield: 10.2 g (63.8%), colorless oil

| Elementary analysis: | | | | | |
|---|---|---|---|---|---|
| Cld | C75.16 | H 5.30 | N 3.51 | 08.01 | S8.02 |
| Fnd | C 75.01 | H 5.58 | N 3.30 | | S 7.89 |

### b) N,N'-Bis-[2-(triphenylmethylmercapto)-1-oxo-eth-1-yl]-3,4-diaminobenzoic acid

7.99 g (10 mmol) of N,N'-bis-[2-triphenylmethylmercapto)-1-oxo-eth-1-yl]-3,4-diaminobenzoic acid methyl ester (example 26a) is mixed in 200 ml of dioxane, 20 ml of water and 20 ml of methanol with 4 g (100 mmol) of sodium hydroxide. It is stirred for 5 hours at room temperature and the batch is poured on 300 ml of 5% aqueous citric acid. It is extracted exhaustively with dichloromethane, the organic phase is dried on sodium sulfate. After evaporation of the solvent, the residue is chromatographed on silica gel (mobile solvent: dichloromethane/methanol, methanol 0-40%).
Yield: 3.56 g (45.4%), colorless oil

| Elementary analysis: | | | | | |
|---|---|---|---|---|---|
| Cld | C 74.97 | H 5.14 | N 3.57 | O 8.15 | S 8.17 |
| Fnd | C 74.71 | H 5.32 | N 3.31 | | S 7.88 |

### c) 5'-{N-[3',4'-Bis-(2"-mercaptoacetylamino)-benzoyl]-6-aminohexylphosphoric acid ester} of 5'-CUCAUGGAGCGCAAGACGAAUAGCUACAUAT*T*T*T*T*-3'

First, the NHS-ester of N,N'-bis-[2-(triphenylmethylmercapto)-1-oxo-eth-1-yl]-3,4-diaminobenzoic acid (example 26b) is produced. For this purpose, 78.5 mg (0.1 mmol) of the acid is dissolved in 500 µl of absolute DMF and mixed with 11.5 mg (0.1 mmol) of NHS. After cooling to 0°C, 19.2 mg (0.1 mmol) of EDC is added and incubated for 30 minutes at 0°C. A solution of 10 mg of the 5'-(6-aminohexyl-phosphoric acid ester) of 5'-CUCAUGGAGCGCAAGACGAAUAGCUACAUAT*T*T*T*T*-3' in 1 ml of a sodium bicarbonate/sodium carbonate buffer (pH = 8.0), described under example 1, is mixed with 500 µl of the NHS-ester solution produced in advance. It is incubated for 17 hours at room temperature. Then, it is concentrated by evaporation to 500 µl and chromatographed on Sephadex G-25. 7 mg of the S-trityl-protected compound is obtained.

The cleavage of the S-trityl protective group, the isolation of the oligonucleotide carrying SH groups and the purification take place as described under example 22e. 3 mg of white lyophilizate is obtained.

### d) Tc-99m Complex of 5'-{N-[3',4'-bis-(2"-mercaptoacetylamino)-benzoyl]-6-aminohexylphosphorous acid ester} of 5'-CUCAUGGAGCGCAAGACGAAUAGCUACAUAT*T*T*T*T*-3'

1 mg of the conjugate described under example 26c) is dissolved in I ml of 0.1 M disodium hydrogen phosphate buffer (pH = 9.5). After adding 10 mg of disodium tartrate, it is mixed with sodium pertechnetate solution (1 mCi) and then with 10 µl of tin(II) chloride solution (5 mg/l ml of 0.01 M HCl). The tracer yield (about 91%) was determined by HPLC.

### Example 27

### a) N-[O-Acetyl-hydroxyacetyl]-glycyl-glycyl-glycine-tert-butyl ester

24.5 g (0.1 mol) of glycyl-glycyl-glycine-tert-butyl ester and 11.8 g (0.1 mol) of O-acetyl-glycolic acid are added together at 0°C in 500 ml of absolute dimethylformamide. A solution of 20.6 (0.1 mol) of dicylcohexylcarbodiimide in 500 ml of absolute dimethylformamide is instilled in the batch, stirred for 1 hour at 0°C and finally overnight at room temperature. It is filtered and the filtrate is concentrated by evaporation on the oil pump. It is crystallized repeatedly from ethyl acetate/n-pentane.
Yield: 12.5 g (36.2%), white powder

| Elementary analysis: | | | | |
|---|---|---|---|---|
| Cld | C 48.69 | H 6.71 | N 12.17 | O 32.43 |
| Fnd | C 48.43 | H 7.01 | N 11.93 | |

### b) N-[O-Acetyl-hydroxyacetyl]-glycyl-glycyl-glycine

3.45 g (10 mmol) of N-[O-acetyl-hydroxyacetyl]-glycyl-glycyl-glycine-tert-butyl ester is stirred in 50 ml of trifluoroacetic acid for 15 minutes. Then, it is poured on 500 ml of absolute diethyl ester and the product is filtered off. It is recrystallized repeatedly from ethyl acetate/n-pentane.
Yield: 1.23 g (42.5%), white powder

| Elementary analysis: | | | | |
|---|---|---|---|---|
| Cld | C 41.53 | H 5.23 | N 14.53 | O 38.72 |
| Fnd | C 41.31 | H 5.51 | N 14.32 | |

### c) 5-{N-[N'-(Hydroxyacetyl)-glycyl-glycyl-glycyl]-6-aminohexylphosphoric acid ester} of 5'-CUCAUGGAGCCAAGACGAAUAGCUACAUAT*T*T*T*T*-3'

First, the NHS-ester of N-(O-acetylhydroxyacetyl)-glycyl-glycyl-glycine (example 27b) is produced. For this purpose, 28.9 mg (0.1 mmol) of the acid is dissolved in 500 µl of absolute DMF and mixed with 11.5 mg (0.1 mmol) of NHS. After cooling to 0°C, 19.2 mg (0.1 mmol) of EDC is added and incubated for 30 minutes at 0°C. A solution of 10 mg of the (6-aminohexyl-phosphoric acid ester) of 5'-CUCAUGGAGCGCAAGACGAAUAGCUACAUAT*T*T*T*T*-3' in 1 ml of 1 M sodium carbonate solution, described under example 1, is mixed with 500 µl of the NHS-ester solution produced in advance. It is incubated for 18 hours at room temperature. Then, it is concentrated by evaporation to 500 µl and chromatographed on Sephadex G-25. After freeze-drying, 3 mg of the title compound is obtained.

### d) Tc-99m Complex of 5-{N-[N'-(hydroxyacetyl)-glycyl-glycyl-glycyl]-6-aminohexylphosphoric acid ester} of 5'-CUCAUGGAGCCAAGACGAAUAGCUACAUAT*T*T*T*T*-3'

1 mg of the conjugate described under example 27c is dissolved in 1 ml of 0.1 M disodium hydrogen phosphate buffer (pH = 10.5). After adding 10 mg of disodium tartrate, it is mixed with sodium pertechnetate solution (1 mCi) and then with 10 µl of tin(II) chloride solution (5 mg/l ml of 0.01 M HCl). The tracer yield is determined by HPLC (95%).

The preceding examples can be repeated with similar success by substituting the generically or specifically described reactants and/or operating conditions of this invention for those used in the preceding examples.

## Claims

1. Oligonucleotide conjugates consisting of an oligonucleotide radical N and n substituents (B-K), in which the compound exhibits general formula I)
N-(B-K)ₙ (I)
in which N is an oligonucleotide with 15 to 100 nucleotides, which bonds specifically with high bonding affinity to target structures and exhibits modifications that prevents or at least significantly reduce the degradation by naturally occurring nucleases,
B is a chemical bond or a connecting component, which produces the connection between N and K, and K means a complexing agent or complex of radioactive metal isotopes, or stable isotopes, which
-- are converted by radiation from outside to radioactive isotopes,
-- convert radiation from outside to radiation of different quality, different energy content and/or different wavelength,
of elements of atomic numbers 5, 21-29, 31, 39, 42-44, 49, 57-83 or 85,
n is a number between 1 and 10
wherein
**a)** the 2'-position of the sugar unit, independently of one another, is occupied by the following groups:
a group -OR, in which
R is an alkyl radical with 1 to 20 carbon atoms, which optionally contains up to 2 hydroxyl groups and which optionally is interrupted by 1-5 oxygen atoms,
a hydrogen atom,
a hydroxyl group,
a fluorine atom,
an amine radical,
an amino group,
and hydroxyl groups present in terminal positions 3' and 5', independently of one another, optionally are etherified with radical R and/or
**b)** the phosphodiesters, optionally being used as intemucleotide bond, independently of one another, are replaced by phosphorothioates, phosphorodithioates or alkylphosphonates, preferably methyl phosphonate, and/or
**c)** the terminal radicals in 3'- and 5'-positions are linked in an intramolecular manner with one another by an intemucleotide bond as described in b) and/or
**d)** it contains an internucleotide bond as described in b), which links 3'-3'- or 5'-5'-positions, and/or
**e)** it contains a phosphodiester bond as described in b), which connects, esterlike, two thymidines respectively by a C₂-C₁₀ hydroxyalkyl radical in 3-position or connects an analogously substituted thymidine radical, esterlike, with a hydroxyl group of another sugar in 2'- or 3'- or 5'-position and/or
**f)** the terminal radicals in 3'- and 5'-positions contain internucleotide bonds optionally modified as described in b).

2. Compound according to claim 1, wherein N is an oligonucleotide, which bonds specifically with high bonding affinity to other target structures and which can be obtained in that a mixture of oligonucleotides containing random sequences is brought together with the target structure, and certain oligonucleotides exhibit an increased affinity to the target structure relative to the mixture of the oligonucleotides, the latter are separated from the remainder of the oligonucleotide mixture, then the oligonucleotides with increased affinity to the target structure are amplified to obtain a mixture of oligonucleotides that exhibits an increased portion of oligonucleotides that bond on the target structures.

3. Compound according to one of the claims 1 to 2, wherein N is an oligonucleotide, which specifically bonds with high bonding affinity to other target structures, and which can be obtained in that
**a)** first, a DNA strand is produced by chemical synthesis, so that this DNA strand exhibits a defined sequence on the 3'-end, which is complementary to a promoter for an RNA-polymerase and at the same time complementary to a primer of the polymerase chain reaction (PCR), and so that this DNA strand exhibits a defined DNA sequence on the 5'-end, which is complementary to a primer sequence for the polymerase chain reaction, and the sequence between the defined sequences contains a random sequence, and in that
**b)** this DNA strand is transcribed in a complementary RNA strand with the help of an RNA-polymerase, and nucleotides are offered to the polymerase, which are modified in the 2'-position of the ribose unit, and in that
**c)** the RNA oligonucleotides, produced in this way, are brought together with the target structure on which the oligonucleotide specifically is to bond, and in that
**d)** those oligonucleotides that have bound on the target structure are separated first together with the target structure from the nonbinding oligonucleotides and then the bound oligonucleotides are separated again from the target structure, and in that
**e)** these target-structure-specific RNA oligonucleotides are transcribed with the help of reverse transcriptase in a complementary DNA strand, and in that
**f)** these DNA strands are amplified using the defined primer sequences with the polymerase chain reaction, and in that
**g)** the DNA oligonucleotides amplified in this manner are then transcribed again with the help of the RNA-polymerase and with modified nucleotides in RNA-oligonucleotides, and in that
**h)** above-mentioned selection steps c) to g) optionally are repeated often until the oligonucleotides, which are **characterized by** a high bonding affinity to the target structure, are sufficiently selected, and then the sequences of the thus obtained oligonucleotides optionally can be determined.

4. Compound according to claim 3, wherein the target structure is selected from among macromolecules, tissue structures of higher organisms, such as animals or humans, organs or parts of organs of an animal or human, cells, tumor cells or tumors.

5. Compound according to one of claims 1 to 4, wherein connecting component(s) B is (are) bound
**a)** to the 4'-end of oligonucleotide radical N reduced in 4'-position by the CH₂-OH group and/or
**b)** to the 3'-end of oligonucleotide radical N reduced in 3'-position by a hydrogen atom and/or
**c)** to the phosphodiester bridge(s), reduced by the OH group(s), between two nucleotides each and/or
**d)** to 1 to 10 nucleobase(s), which is (are) reduced by a hydrogen atom respectively in 5-, 8-position(s) and/or the amino group(s) in 2-, 4- and 6-position(s).

6. Compound according to claim 5, paragraph a) or b), wherein B has general formula X-Y-Z¹, which is connected on the X side with the complexing agent or complex and on the Z side with the oligonucleotide, in which
X is a direct bond, an -NH or -S group,
Y is a straight-chain, branched-chain, saturated or unsaturated C₁-C₂₀ alkylene chain, which optionally contains 1-2 cyclohexylene, 1-5 imino, 1-3 phenylene, 1-3 phenylenimino, 1-3 phenylenoxy, 1-3 hydroxyphenylene, 1-5 amido, 1-2 hydrazido, 1-5 carbonyl, 1-5 ethylenoxy, a ureido, a thioureido, 1-2 carboxyalkylimino, 1-2 ester groups, 1-3 groups of Ar, in which Ar stands for a saturated or unsaturated 5- or 6-ring, which optionally contains 1-2 heteroatoms selected from nitrogen, oxygen and sulfur and/or 1-2 carbonyl groups; 1-10 oxygen, 1-5 nitrogen and/or 1-5 sulfur atoms, and/or optionally is substituted by 1-5 hydroxy, 1-2 mercapto, 1-5 oxo, 1-5 thioxo, 1-3 carboxy, 1-5 carboxy-C₁-C₄-alkyl, 1-5 ester, 1-3 amino, 1-3 hydroxy-C₁-C₄ alkyl, 1-3 C₁-C₇-alkoxy groups, and
Z¹ is -CONH-CH₂-4', -NH-CO-4', -O-P(O)R¹-NH-CH₂-4', -O-P(O)R¹-O-CH₂-4', -O-P(S)R¹-O-3' or -O-P(O)R¹-O-3', in which 4' or 3' indicates the linkage to the terminal sugar unit(s) and R¹ is O⁻, S⁻, a C₁-C₄ alkyl or NR²R³ group, with R² and R³ meaning hydrogen or C₁-C₄ alkyl radicals.

7. Compound according to claim 5, paragraph c), wherein B has general formula X-Y-Z², which is connected on the X side with the complexing agent or complex and on the Z side with the oligonucleotide, in which
Z², in the bridge linking two adjacent sugar units, 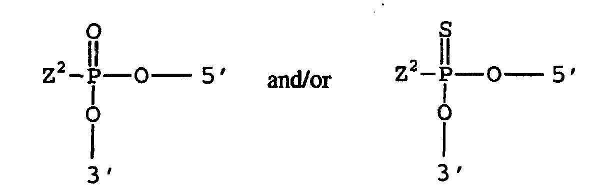 is the group -NR²-, -O- or -S-,
X is a direct bond, an -NH or -S group,
Y is a straight-chain, branched-chain, saturated or unsaturated C₁-C₂₀ alkylene chain, which optionally contains 1-2 cyclohexylene, 1-5 imino, 1-3 phenylene, 1-3 phenylenimino, 1-3 phenylenoxy, 1-3 hydroxyphenylene, 1-5 amido, 1-2 hydrazido, 1-5 carbonyl, 1-5 ethylenoxy, a ureido, a thioureido, 1-2 carboxyalkylimino, 1-2 ester groups, 1-3 groups of Ar, in which Ar stands for a saturated or unsaturated 5- or 6-ring, which optionally contains 1-2 heteroatoms selected from nitrogen, oxygen and sulfur and/or 1-2 carbonyl groups; 1-10 oxygen, 1-5 nitrogen and/or 1-5 sulfur atoms, and/or optionally is substituted by 1-5 hydroxy, 1-2 mercapto, 1-5 oxo, 1-5 thioxo, 1-3 carboxy, 1-5 carboxy-C₁-C₄-alkyl, 1-5 ester, 1-3 amino, 1-3 hydroxy-C₁-C₄ alkyl, 1-3 C₁-C₇-alkoxy groups, and
R² is hydrogen or C₁-C₄ alkyl radicals.

8. Compound according to claim 5d), wherein B has general formula X-Y-Z³, in which Z³ stands for an -NH group or a direct bond to the nucleobase,
X is a direct bond, an -NH or -S group, and
Y is a straight-chain, branched-chain, saturated or unsaturated C₁-C₂₀ alkylene chain, which optionally contains 1-2 cyclohexylene, 1-5 imino, 1-3 phenylene, 1-3 phenylenimino, 1-3 phenylenoxy, 1-3 hydroxyphenylene, 1-5 amido, 1-2 hydrazido, 1-5 carbonyl, 1-5 ethylenoxy, a ureido, a thioureido, 1-2 carboxyalkylimino, 1-2 ester groups, 1-3 groups of Ar, in which Ar stands for a saturated or unsaturated 5- or 6-ring, which optionally contains 1-2 heteroatoms selected from nitrogen, oxygen and sulfur and/or 1-2 carbonyl groups; 1-10 oxygen, 1-5 nitrogen and/or 1-5 sulfur atoms, and/or optionally is substituted by 1-5 hydroxy, 1-2 mercapto, 1-5 oxo, 1-5 thioxo, 1-3 carboxy, 1-5 carboxy-C₁-C₄-alkyl, 1-5 ester, 1-3 amino, 1-3 hydroxy-C₁-C₄ alkyl, 1-3 C₁-C₇-alkoxy groups.

9. Compounds according to one of the preceding claims, wherein the metal complex, as imaging element, contains a radioactive isotope, selected from the elements copper, bismuth, technetium, rhenium or indium.

10. Process for detecting a target structure, wherein one or more of the compounds according to one of the preceding claims are brought together with the sample to be studied in vivo or in vitro and based on the signal, it is detected whether the target structure, on which oligonucleotide N bonds specifically and with high bonding affinity, is present in the sample.

11. Compounds according to one of the claims 1-9 for noninvasive diagnosis of diseases.

12. Use of a compound according to one of claims 1 to 9 for the production of a medicament useful in radiodiagnosis and/or in radiotherapy.

13. Diagnosis kit for in vivo and/or in vitro detection of target structures, wherein the diagnosis kit contains at least one compound according to one of claims 1 to 9.

14. Compound according to claim 1, wherein N is a non-naturally occuring oligonucleotide ligand having a specific binding affinity for a target molecule, such target molecule being a three dimensional chemical structure other than a polynucleotide that binds to said oligonucleotide ligand through a mechanism which predominantly depends on Watson/Crick base pairing or triple helix binding, wherein said oligo-nucleotide ligand is not a nucleic acid having the known physiological function of being bound by the target molecule.

## Patentansprüche

1. Oligonucleotid-Konjugate, bestehend aus einem Oligonucleotid-Rest N und n Substituenten (B-K), wobei die Verbindung die allgemeine Formel I)
N-(B-K)ₙ (I)
aufweist, worin N ein Oligonucleotid mit 15 bis 100 Nucleotiden ist, das spezifisch mit hoher Bindungsaffinität an Zielstrukturen bindet und Modifikationen aufweist, die den Abbau durch natürlich vorkommende Nucleasen verhindert oder wenigstens wesentlich reduziert,
B eine chemische Bindung oder eine Verbindungskomponente ist, die die Verbindung zwischen N und K herstellt, und K ein Komplexbildner oder ein Komplex radioaktiver Metallisotopen oder stabiler Isotopen ist, die
- durch Strahlung von außen in radioaktive Isotope umgewandelt werden,
- Strahlung von außen in Strahlung anderer Qualität, anderen Energiegehalts und/oder anderer Wellenlänge umwandeln,
der Elemente der Ordnungszahlen 5, 21 - 29, 31, 39, 42 - 44, 49, 57 - 83 oder 85,
n eine Zahl zwischen 1 und 10 ist,
**dadurch gekennzeichnet, daß**
**a)** die 2'-Position der Zuckereinheit unabhängig voneinander mit folgenden Gruppen besetzt ist:
einer Gruppe -OR, worin
R ein Alkylrest mit 1 bis 20 Kohlenstoffatomen, der gegebenenfalls bis zwei Hydroxylgruppen enthält und der gegebenenfalls durch 1 - 5 Sauerstoffatome unterbrochen ist, bedeutet,
einem Wasserstoffatom,
einer Hydroxylgruppe,
einem Fluoratom,
einem Aminrest,
einer Aminogruppe,
und in den terminalen Positionen 3' und 5' vorhandene Hydroxylgruppen unabhängig voneinander gegebenenfalls mit dem Rest R verethert sind und/oder
**b)** gegebenenfalls als Internucleotidbindung dienende Phosphodiester, unabhängig voneinander durch Phosphorothioate, Phosphorodithioate oder Alkylphosphonate, bevorzugt Methylphosphonat, ersetzt sind, und/oder
**c)** die terminalen Reste in den Positionen 3' und 5' durch eine wie in b) beschriebene Internucleotidbindung intramolekular miteinander verknüpft sind und/oder
**d)** es eine wie in b) beschriebene Internucleotidbindung enthält, die die Positionen 3'-3' oder 5'-5' verknüpft und/oder
**e)** es eine wie in b) beschriebene Phosphodiesterbindung enthält, die zwei Thymidine über jeweils einen in der 3-Stellung befindlichen C₂-C₁₀-Hydroxyalkylrest esterartig verbindet oder einen analog substituierten Thymidinrest esterartig mit einer Hydroxylgruppe eines anderen Zuckers in den Positionen 2' oder 3' oder 5' verbindet und/oder
**f)** die terminalen Reste in den Positionen 3' und 5' gegebenenfalls wie in b) beschrieben modifizierte Internucleotidbindungen enthalten.

2. Verbindungen nach Anspruch 1, **dadurch gekennzeichnet, daß** N ein Oligonucleotid ist, das spezifisch mit hoher Bindungsaffinität an andere Zielstrukturen bindet und das dadurch erhältlich ist, daß eine Zufallssequenzen enthaltende Mischung von Oligonucleotiden mit der Zielstruktur zusammengebracht wird, wobei bestimmte Oligonucleotide eine erhöhte Affinität zu der Zielstruktur im Verhältnis zu der Mischung der Oligonucleotide aufweisen, diese von dem Rest der Oligonucleotidmischung abgetrennt werden, dann die Oligonucleotide mit erhöhter Affinität zu der Zielstruktur amplifiziert werden, wodurch man eine Mischung von Oligonucleotiden erhält, die einen erhöhten Anteil an Oligonucleotiden aufweist, die an die Zielstrukturen binden.

3. Verbindungen nach Anspruch 1 oder 2, **dadurch gekennzeichnet, daß** N ein Oligonucleotid ist, das spezifisch mit hoher Bindungsaffinität an andere Zielstrukturen bindet und das dadurch erhältlich ist, daß
**a)** zunächst ein DNA-Strang durch chemische Synthese derart hergestellt wird, daß dieser DNA-Strang am 3'-Ende eine definierte Sequenz aufweist, die komplementär zu einem Promoter für eine RNA-Polymerase ist und zugleich komplementär zu einem Primer der Polymerase-Kettenreaktion (PCR) ist, und daß dieser DNA-Strang am 5'-Ende eine definierte DNA-Sequenz aufweist, die komplementär zu einer Primersequenz für die Polymerase-Kettenreaktion ist, wobei die Sequenz zwischen den definierten Sequenzen eine Zufallssequenz enthält, und das
**b)** dieser DNA-Strang mit Hilfe einer RNA-Polymerase in einem komplementären RNA-Strang umgeschrieben wird, wobei der Polymerase Nucleotide angeboten werden, die an der 2'-Position der Riboseeinheit modifiziert sind, und daß
**c)** die auf diese Weise hergestellten RNA-Oligonucleotide mit der Zielstruktur, an die das Oligonucleotid spezifisch binden soll, zusammengebracht werden, und daß
**d)** diejenigen Oligonucleotide, die an die Zielstruktur gebunden haben, zuerst zusammen mit der Zielstruktur von den nichtbindenden Oligonucleotiden abgetrennt werden und dann die gebundenen Oligonucleotide von der Zielstruktur wieder abgetrennt werden, und daß
**e)** diese Zielstruktur-spezifischen RNA-Oligonucleotide mit Hilfe der reversen Transkriptase in einem komplementären DNA-Strang umgeschrieben werden, und daß
**f)** diese DNA-Stränge unter Verwendung der hier definierten Primersequenzen mit der Polymerase-Kettenreaktion amplifiziert werden, und daß
**g)** die auf diese Art und Weise amplifizierten DNA-Oligonucleotide dann wieder mit Hilfe der RNA-Polymerase und mit modifizierten Nucleotiden in RNA-Oligonucleotide umgeschrieben werden, und daß
**h)** die obengenannten Selektionsschritte c) bis g) gegebenenfalls so oft wiederholt werden, bis die Oligonucleotide, die durch eine hohe Bindungsaffinität zu der Zielstruktur charakterisiert sind, hinreichend selektioniert sind und anschließend die Sequenzen der so erhaltenen Oligonucleotide gegebenenfalls bestimmt werden können.

4. Verbindungen nach Anspruch 3, **dadurch gekennzeichnet, daß** die Zielstruktur ausgewählt ist unter Makromolekülen, Gewebestrukturen von höheren Organismen wie Tieren oder Menschen, Organen oder Teilen von Organen eines Tieres oder eines Menschen, Zellen, Tumorzellen oder Tumoren.

5. Verbindungen nach einem der Ansprüche 1 bis 4, **dadurch gekennzeichnet, daß** die Verbindungskomponente(n) B
**a)** an das 4'-Ende des in der 4'-Position um die CH₂-OH- Gruppe verminderten Oligonucleotid-Restes N und/oder
**b)** an das 3'-Ende des in der 3'-Position um ein Wasserstoffatom verminderten Oligonucleotid-Restes N und/oder
**c)** an die um die OH-Gruppe(n) verminderte(n) Phosphodiesterbrücke(n) zwischen jeweils zwei Nucleotiden und/oder
**d)** an 1 bis 10 Nucleobase(n), die jeweils in der/den Position(en) 5, 8 und/oder der/den Aminogruppe(n) der Position(en) 2, 4 und 6 um ein Wasserstoffatom vermindert ist (sind),
gebunden ist (sind).

6. Verbindungen nach Anspruch 5, Absatz a) oder b), **dadurch gekennzeichnet, daß** B die allgemeine Formel X-Y-Z¹ hat, die X-seitig mit dem Komplexbildner oder Komplex und Z-seitig mit dem Oligonucleotid verbunden ist, worin
X für eine direkte Bindung, eine -NH- oder -S-Gruppe steht,
Y für eine geradkettige, verzweigte, gesättigte oder ungesättigte C₁-C₂₀-Alkylenkette steht, die gegebenenfalls 1-2 Cyclohexylen-, 1-5 Imino-, 1-3 Phenylen-, 1-3 Phenylenimino-, 1-3 Phenylenoxy-, 1-3 Hydroxyphenylen-, 1-5 Amido-, 1-2 Hydrazido-, 1-5 Carbonyl-, 1-5 Ethylenoxy-, eine Ureido-, eine Thioureido-, 1-2 Carboxyalkylimino-, 1-2 Estergruppen, 1-3 Gruppen Ar, worin Ar für einen gesättigten oder ungesättigten 5- oder 6gliedrigen Ring steht, der gegebenenfalls 1-2 Heteroatome ausgewählt aus Stickstoff, Sauerstoff und Schwefel und/oder 1-2 Carbonylgruppen enthält, 1-10 Sauerstoff-, 1-5 Stickstoff- und/oder 1-5 Schwefelatome enthält und/oder gegebenenfalls durch 1-5 Hydroxy-, 1-2 Mercapto-, 1-5 Oxo-, 1-5 Thioxo-, 1-3 Carboxy-, 1-5 Carboxy-C₁-C₄-alkyl-, 1-5 Ester-, 1-3 Amino-, 1-3 Hydroxy-C₁-C₄-alkyl-, 1-3 C₁-C₇-Alkoxygruppen substituiert ist und Z¹ für -CONH-CH₂-4', -NH-CO-4', -O-P(O)R¹-NH-CH₂-4', -O-P(O)R¹-O-CH₂-4', -O-P(S)R¹-O-3' oder -O-P(O)R¹-O-3'steht, worin 4' bzw. 3' die Verknüpfung an die endständige(n) Zuckereinheit(en) angibt und R¹ für O⁻, S⁻, eine C₁-C₄-Alkyl- oder NR²R³-Gruppe steht, mit R² und R³ in der Bedeutung von Wasserstoff und C₁-C₄-Alkylresten.

7. Verbindungen nach Anspruch 5, Absatz c), **dadurch gekennzeichnet, daß** B die allgemeine Formel X-Y-Z² hat, die X-seitig mit dem Komplexbildner oder Komplex und Z-seitig mit dem Oligonucleotid verbunden ist, wobei Z², in der zwei benachbarte Zuckereinheiten verknüpfenden Brücke 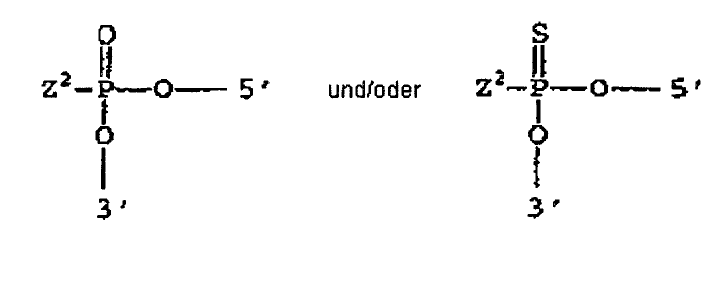 für die Gruppe -NR²-, -O- oder -S- steht,
X für eine direkte Bindung, eine -NH- oder -S-Gruppe steht,
Y für eine geradkettige, verzweigte, gesättigte oder ungesättigte C₁-C₂₀-Alkylenkette steht, die gegebenenfalls 1-2 Cyclohexylen-, 1-5 Imino-, 1-3 Phenylen-, 1-3 Phenylenimino-, 1-3 Phenylenoxy-, 1-3 Hydroxyphenylen-, 1-5 Amido-, 1-2 Hydrazido-, 1-5 Carbonyl-, 1-5 Ethylenoxy-, eine Ureido-, eine Thioureido-, 1-2 Carboxyalkylimino-, 1-2 Estergruppen, 1-3 Gruppen Ar, worin Ar für einen gesättigten oder ungesättigten 5- oder 6gliedrigen Ring steht, der gegebenenfalls 1-2 Heteroatome ausgewählt aus Stickstoff, Sauerstoff und Schwefel und/oder 1-2 Carbonylgruppen enthält, 1-10 Sauerstoff-, 1-5 Stickstoff- und/oder 1-5 Schwefelatome enthält und/oder gegebenenfalls durch 1-5 Hydroxy-, 1-2 Mercapto-, 1-5 Oxo-, 1-5 Thioxo-, 1-3 Carboxy-, 1-5 Carboxy-C₁-C₄-alkyl-, 1-5 Ester-, 1-3 Amino-, 1-3 Hydroxy-C₁-C₄-alkyl-, 1-3 C₁-C₇-Alkoxygruppen substituiert ist und
R² für Wasserstoff oder C₁-C₄-Alkylreste steht.

8. Verbindung nach Anspruch 5d), **dadurch gekennzeichnet, daß** B die allgemeine Formel X-Y-Z³ hat, wobei Z³ für eine -NH-Gruppe oder eine direkte Bindung zur Nucleobase steht,
X für eine direkte Bindung, eine -NH- oder -S-Gruppe steht und
Y für eine geradkettige, verzweigte, gesättigte oder ungesättigte C₁-C₂₀-Alkylenkette steht, die gegebenenfalls 1-2 Cyclohexylen-, 1-5 Imino-, 1-3 Phenylen-, 1-3 Phenylenimino-, 1-3 Phenylenoxy-, 1-3 Hydroxyphenylen-, 1-5 Amido-, 1-2 Hydrazido-, 1-5 Carbonyl-, 1-5 Ethylenoxy-, eine Ureido-, eine Thioureido-, 1-2 Carboxyalkylimino-, 1-2 Estergruppen, 1-3 Gruppen Ar, worin Ar für einen gesättigten oder ungesättigten 5- oder 6gliedrigen Ring steht, der gegebenenfalls 1-2 Heteroatome ausgewählt aus Stickstoff, Sauerstoff und Schwefel und/oder 1-2 Carbonylgruppen enthält, 1-10 Sauerstoff-, 1-5 Stickstoff- und/oder 1-5 Schwefelatome enthält und/oder gegebenenfalls durch 1-5 Hydroxy-, 1-2 Mercapto-, 1-5 Oxo-, 1-5 Thioxo-, 1-3 Carboxy-, 1-5 Carboxy-C₁-C₄-alkyl-, 1-5 Ester-, 1-3 Amino-, 1-3 Hydroxy-C₁-C₄-alkyl-, 1-3 C₁-C₇-Alkoxygruppen substituiert ist.

9. Verbindungen nach einem der vorhergehenden Ansprüche, **dadurch gekennzeichnet, daß** der Metallkomplex als bildgebendes Element ein radioaktives Isotop ausgewählt aus den Elementen Kupfer, Wismut, Technetium, Rhenium oder Indium enthält.

10. Verfahren zum Nachweis einer Zielstruktur, **dadurch gekennzeichnet, daß** man eine oder mehrere der Verbindungen nach einem der vorhergehenden Ansprüche mit der zu untersuchenden Probe in vivo oder in vitro zusammenbringt und anhand des Signales nachweist, ob die Zielstruktur, an die das Oligonucleotid N spezifisch und mit hoher Bindungsaffinität bindet, in der Probe vorhanden ist.

11. Verbindungen nach einem der Ansprüche 1 - 9 zur nicht invasiven Diagnose von Krankheiten.

12. Verwendung einer Verbindung nach einem der Ansprüche 1 bis 9 zur Herstellung eines für die Radiodiagnostik und/oder Radiotherapie geeigneten Medikaments.

13. Diagnosekit zum Nachweis von Zielstrukturen in vivo und/oder in vitro, **dadurch gekennzeichnet, daß** das Diagnosekit wenigstens eine Verbindung nach einem der Ansprüche 1 bis 9 enthält.

14. Verbindung nach Anspruch 1, wobei es sich bei N um einen nicht natürlich vorkommenden Oligonucleotidliganden mit spezifischer Bindungsaffinität für ein Zielmolekül handelt, wobei solch ein Zielmolekül eine dreidimensionale chemische Struktur ist, bei der es sich nicht um ein Polynucleotid handelt, das sich über einen Mechanismus, der hauptsächlich auf Watson/Crick-Basenpaarung oder Triplehelixbindung beruht, an den Oligonucleotidliganden bindet, wobei es sich bei dem Oligonucleotidliganden nicht um eine Nucleinsäure handelt, von der bekannt ist, daß eine ihrer physiologischen Funktionen die Bindung durch das Zielmolekül ist.

## Revendications

1. Conjugués oligonucléotidiques constitués d'un radical oligonucléotidique N et n substituants (B-K), dans lesquels le composé présente la formule générale I)
N-(B-K)ₙ (I)
dans laquelle N est un oligonucléotide de 15 à 100 nucléotides, qui se lie de manière spécifique avec une forte affinité de liaison à des structures cibles et présente des modifications qui empêchent ou au moins réduisent significativement la dégradation par les nucléases naturelles,
B est une liaison chimique ou un composant de connexion, qui produit la connexion entre N et K, et K signifie un agent complexant ou un complexe d'isotopes de métaux radioactifs, ou des isotopes stables, qui
- sont transformés par radiation de l'extérieur en isotopes radioactifs,
- transforment la radiation de l'extérieur en radiation de qualité différente, de teneur en énergie différente et/ou de longueurs d'onde différentes,
d'éléments de nombres atomiques 5, 21 - 29, 31, 39, 42 - 44, 49, 57 - 83 ou 85,
n est un nombre compris entre 1 et 10,
**caractérisés en ce que**
**a)** la position 2' du motif sucre, indépendamment l'un de l'autre, est occupée par les groupements suivants :
un groupement -OR, dans lequel
R est un radical alkyle de 1 à 20 atomes de carbone, qui contient éventuellement jusqu'à 2 groupements hydroxy et qui est éventuellement interrompu par 1 - 5 atomes d'oxygène,
un atome d'hydrogène,
un groupement hydroxy,
un atome de fluor,
un radical amine,
un groupement amino,
et des groupements hydroxy présents en positions terminales 3' et 5', indépendamment l'un de l'autre, sont éventuellement éthérifiés avec un radical R et/ou
**b)** les phosphodiesters, étant éventuellement utilisés comme liaison internucléotidique, indépendamment les uns des autres, sont remplacés par des phosphorothioates, des phosphorodithioates ou des alkylphosphonates, de préférence le méthylphosphonate, et/ou
**c)** les radicaux terminaux en positions 3' et 5' sont liés de manière intramoléculaire les uns aux autres par une liaison internucléotidique comme décrit en b) et/ou
**d)** il contient une liaison internucléotidique comme décrit en b), qui lie les positions 3'-3' ou 5'-5', et/ou
**e)** il contient une liaison phosphodiester comme décrit en b), qui connecte, comme un ester, deux thymidines respectivement par un radical hydroxyalkyle en C₂-C₁₀ en position 3 ou connecte un radical thymidine substitué de manière analogue, comme un ester, avec un groupement hydroxy d'un autre sucre en position 2' ou 3' ou 5', et/ou
**f)** les radicaux terminaux en positions 3' et 5' contiennent des liaisons internucléotidiques éventuellement modifiées comme décrit en b).

2. Composé selon la revendication 1, **caractérisé en ce que** N est un oligonucléotide qui se lie de manière spécifique avec une forte affinité de liaison à d'autres structures cibles et qui peut être obtenu par le fait qu'un mélange d'oligonucléotides contenant des séquences aléatoires est mis en contact avec la structure cible, et certains oligonucléotides présentent une affinité accrue vis-à-vis de la structure cible par rapport au mélange d'oligonucléotides, ces derniers sont séparés du reste du mélange oligonucléotidique, puis les oligonucléotides présentant une affinité accrue vis-à-vis de la structure cible sont amplifiés pour obtenir un mélange d'oligonucléotides présentant une portion accrue d'oligonucléotides se liant aux structures cibles.

3. Composé selon l'une des revendications 1 à 2, **caractérisé en ce que** N est un oligonucléotide qui se lie de manière spécifique avec une forte affinité de liaison à d'autres structures cibles, et qui peut être obtenu par le fait que
**a)** premièrement, un brin d'ADN est produit par synthèse chimique, de sorte que ce brin d'ADN présente une séquence définie à l'extrémité 3', qui est complémentaire d'un promoteur pour une ARN polymérase et en même temps complémentaire d'une amorce de la réaction en chaîne par la polymérase (PCR), et de sorte que ce brin d'ADN présente une séquence d'ADN définie à l'extrémité 5', qui est complémentaire d'une séquence amorce pour la réaction en chaîne par la polymérase, et la séquence entre les séquences définies contient une séquence aléatoire, et par le fait que
**b)** ce brin d'ADN est transcrit en un brin d'ARN complémentaire à l'aide d'une ARN polymérase, et des nucléotides sont présentés à la polymérase, qui sont modifiés en position 2' du motif ribose, et par le fait que
**c)** les oligonucléotides d'ARN, produits de cette manière, sont mis en contact avec la structure cible à laquelle l'oligonucléotide se lie de manière spécifique, et par le fait que
**d)** les oligonucléotides qui se sont liés à la structure cible sont séparés d'abord ensemble avec la structure cible des oligonucléotides qui ne se lient pas, puis les oligonucléotides liés sont séparés de nouveau de la structure cible, et par le fait que
**e)** ces oligonucléotides d'ARN spécifiques de la structure cible sont transcrits à l'aide d'une transcriptase inverse en un brin d'ADN complémentaire, et par le fait que
**f)** ces brins d'ADN sont amplifiés en utilisant les séquences amorces définies par la réaction en chaîne par la polymérase, et par le fait que
**g)** les oligonucléotides d'ADN amplifiés de cette manière sont alors transcrits de nouveau à l'aide de l'ARN polymérase et avec des nucléotides modifiés en oligonucléotides d'ARN, et par le fait que
**h)** les étapes de sélection c) à g) mentionnées ci-dessus sont éventuellement répétées souvent jusqu'à ce que les oligonucléotides, qui sont **caractérisés par** une forte affinité de liaison à la structure cible, soient suffisamment sélectionnés, puis les séquences des oligonucléotides ainsi obtenus peuvent être éventuellement déterminées.

4. Composé selon la revendication 3, **caractérisé en ce que** la structure cible est choisie parmi les macromolécules, les structures tissulaires d'organismes supérieurs, tels que les animaux ou l'homme, les organes ou les parties d'organes d'un animal ou de l'homme, les cellules, les cellules tumorales ou les tumeurs.

5. Composé selon l'une des revendications 1 à 4, **caractérisé en ce que** le(s) composant(s) de connexion B est (sont) lié(s)
**a)** à l'extrémité 4' du radical oligonucléotidique N réduit en positon 4' par le groupement CH₂-OH et/ou
**b)** à l'extrémité 3' du radical oligonucléotidique N réduit en position 3' par un atome d'hydrogène et/ou
**c)** au(x) pont(s) phosphodiester, réduit(s) par le(s) groupement(s) OH, entre deux nucléotides chacun et/ou
**d)** à 1 à 10 nucléobase(s), qui est (sont) réduite(s) par un atome d'hydrogène respectivement en position(s) 5, 8 et/ou le(s) groupement(s) amino en position(s) 2, 4 et 6.

6. Composé selon la revendication 5, paragraphe a) ou b), **caractérisé en ce que** B est de formule générale X-Y-Z¹, qui est connectée du côté X à l'agent complexant ou au complexe et au côté Z à l'oligonucléotide, dans laquelle
X est une liaison directe, ou un groupement -NH ou -S,
Y est une chaîne droite, une chaîne ramifiée, une chaîne alkylène en C₁-C₂₀ saturée ou insaturée, qui contient éventuellement 1 - 2 groupements cyclohexylène, 1 - 5 groupements imino, 1 - 3 groupements phénylène, 1 - 3 groupements phénylèneimino, 1 - 3 groupements phénylèneoxy, 1 - 3 groupements hydroxyphénylène, 1 - 5 groupements amido, 1 - 2 groupements hydrazido, 1 - 5 groupements carbonyle, 1 - 5 groupements éthylèneoxy, un groupement uréido, un groupement thiouréido, 1 - 2 groupements carboxyalkylimino, 1 - 2 groupements ester, 1 - 3 groupements Ar, où Ar représente un cycle saturé ou insaturé à 5 ou 6 chaînons, contenant éventuellement 1 - 2 hétéroatomes choisis parmi l'azote, l'oxygène et le soufre et/ou 1 - 2 groupements carbonyle ; 1 - 10 atomes d'oxygène, 1 - 5 atomes d'azote et/ou 1 - 5 atomes de soufre, et/ou est éventuellement substituée par 1 - 5 groupements hydroxy, 1 - 2 groupements mercapto, 1 - 5 groupements oxo, 1 - 5 groupements thioxo, 1 - 3 groupements carboxy, 1 - 5 groupements carboxyalkyle en C₁-C₄, 1 - 5 groupements ester, 1 - 3 groupements amino, 1 - 3 groupements hydroxyalkyle en C₁-C₄, 1 - 3 groupements alcoxy en C₁-C₇, et
Z¹ est -CONH-CH₂-4', -NH-CO-4', -O-P(O)R¹-NH-CH₂-4', -O-P(O)R¹-O-CH₂-4', -O-P(S)R¹-O-3' ou -O-P(O)R¹-O-3', dans lesquels 4' ou 3' indique la liaison au(x) motif(s) sucre terminal (terminaux) et R¹ est O-, S-, ou un groupement alkyle en C₁-C₄ ou NR²R³, R² et R³ signifiant l'hydrogène ou des radicaux alkyle en C₁-C₄.

7. Composé selon la revendication 5, paragraphe c), **caractérisé en ce que** B est de formule générale X-Y-Z², qui est connectée du côté X à l'agent complexant ou au complexe et du côté Z à l'oligonucléotide, dans laquelle
Z², dans le pont reliant deux motifs sucre adjacents, 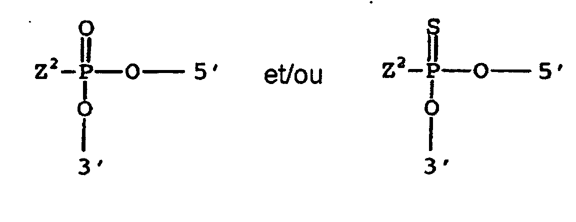 est le groupement -NR²-, -O- ou -S-,
X est une liaison directe, ou un groupement -NH ou -S,
Y est une chaîne droite, une chaîne ramifiée, une chaîne alkylène en C₁-C₂₀ saturée ou insaturée, qui contient éventuellement 1 - 2 groupements cyclohexylène, 1 - 5 groupements imino, 1 - 3 groupements phénylène, 1 - 3 groupements phénylèneimino, 1 - 3 groupements phénylèneoxy, 1 - 3 groupements hydroxyphénylène, 1 - 5 groupements amido, 1 - 2 groupements hydrazido, 1 - 5 groupements carbonyle, 1 - 5 groupements éthylèneoxy, un groupement uréido, un groupement thiouréido, 1 - 2 groupements carboxyalkylimino, 1 - 2 groupements ester, 1 - 3 groupements Ar, où Ar représente un cycle saturé ou insaturé à 5 ou 6 chaînons, contenant éventuellement 1 - 2 hétéroatomes choisis parmi l'azote, l'oxygène et le soufre et/ou 1 - 2 groupements carbonyle ; 1 - 10 atomes d'oxygène, 1 - 5 atomes d'azote et/ou 1 - 5 atomes de soufre, et/ou est éventuellement substituée par 1 - 5 groupements hydroxy, 1 - 2 groupements mercapto, 1 - 5 groupements oxo, 1 - 5 groupements thioxo, 1 - 3 groupements carboxy, 1 - 5 groupements carboxyalkyle en C₁-C₄, 1 - 5 groupements ester, 1 - 3 groupements amino, 1 - 3 groupements hydroxyalkyle en C₁-C₄, 1 - 3 groupements alcoxy en C₁-C₇, et
R² est hydrogène ou des radicaux alkyle en C₁-C₄.

8. Composé selon la revendication 5d), **caractérisé en ce que** B est de formule générale X-Y-Z³, dans laquelle Z³ représente un groupement -NH ou une liaison directe à la nucléobase,
X est une liaison directe, ou un groupement -NH ou -S, et Y est une chaîne droite, une chaîne ramifiée, une chaîne alkylène en C₁-C₂₀ saturée ou insaturée, qui contient éventuellement 1 - 2 groupements cyclohexylène, 1 - 5 groupements imino, 1 - 3 groupements phénylène, 1 - 3 groupements phénylèneimino, 1 - 3 groupements phénylèneoxy, 1 - 3 groupements hydroxyphénylène, 1 - 5 groupements amido, 1 - 2 groupements hydrazido, 1 - 5 groupements carbonyle, 1 - 5 groupements éthylèneoxy, un groupement uréido, un groupement thiouréido, 1 - 2 groupements carboxyalkylimino, 1 - 2 groupements ester, 1 - 3 groupements Ar, où Ar représente un cycle saturé ou insaturé à 5 ou 6 chaînons, contenant éventuellement 1 - 2 hétéroatomes choisis parmi l'azote, l'oxygène et le soufre et/ou 1 - 2 groupements carbonyle ; 1 - 10 atomes d'oxygène, 1 - 5 atomes d'azote et/ou 1 - 5 atomes de soufre et/ou est éventuellement substituée par 1 - 5 groupements hydroxy, 1 - 2 groupements mercapto, 1 - 5 groupements oxo, 1 - 5 groupements thioxo, 1 - 3 groupements carboxy, 1 - 5 groupements carboxyalkyle en C₁-C₄, 1 - 5 groupements ester, 1 - 3 groupements amino, 1 - 3 groupements hydroxyalkyle en C₁-C₄, 1 - 3 groupements alcoxy en C₁-C₇.

9. Composés selon l'une des revendications précédentes, **caractérisés en ce que** le complexe métallique, à titre d'élément d'imagerie, contient un isotope radioactif, choisi parmi les éléments cuivre, bismuth, technétium, rhénium ou indium.

10. Procédé de détection d'une structure cible, **caractérisé en ce que** un ou plusieurs des composés selon l'une des revendications précédentes sont mis en contact avec l'échantillon à étudier in vivo ou in vitro et, en fonction du signal, on détecte dans l'échantillon la présence de la structure cible à laquelle l'oligonucléotide N se lie de manière spécifique et avec une forte affinité de liaison.

11. Composés selon l'une des revendications 1 - 9, destinés au diagnostic non invasif de maladies.

12. Utilisation d'un composé selon l'une des revendications 1 à 9, pour la production de médicament utile en radiodiagnostic et/ou en radiothérapie.

13. Kit de diagnostic pour la détection in vivo et/ou in vitro de structures cibles, **caractérisé en ce que** le kit de diagnostic contient au moins un composé selon l'une des revendications 1 à 9.

14. Composé selon la revendication 1, **caractérisé en ce que** N est un ligand oligonucléotidique non naturel ayant une affinité de liaison spécifique pour une molécule cible, une telle molécule cible étant une structure chimique tridimensionnelle autre qu'un polynucléotide se liant audit ligand oligonucléotidique par un mécanisme qui dépend principalement de la liaison triple hélix ou de l'appariement de base Watson/Crick, et **en ce que** ledit ligand oligonucléotidique n'est pas un acide nucléique ayant la fonction physiologique connue d'être lié par la molécule cible.
